# EUROPEAN PATENT SPECIFICATION

(11) **EP 1 670 782 B1**
(45) Date of publication and mention of the grant of the patent: **14.02.2007**
(21) Application number: 04768652.2
(22) Date of filing: 15.09.2004
(51) Int. Cl.: C07D 401/12, A61K 31/517, A61P 35/00

(54) **QUINAZOLINE DERIVATIVES**
CHINAZOLINDERIVATE
DERIVES DE QUINAZOLINE

(30) Priority: 19.09.2003 EP 03292308; 14.05.2004 EP 04291249
(43) Date of publication of application: 21.06.2006
(73) Proprietor: AstraZeneca AB, 151 85 Södertälje (SE)
(72) Inventor: BRADBURY, Robert, Hugh, MacClesfield Cheshire SK10 4TG (GB); HENNEQUIN, Laurent, Francois, Andre, BP 1050 F-51689 Reims (FR); BARLAAM, Bernard, Christophe, BP 1050 F-51689 Reims Cedex 02 (FR)
(86) International application number: PCT/GB2004/004109
(87) International publication number: WO 2005/028470

(56) References cited:
- WO-A-03/082290
- WO-A-03/082831
- US-A1- 2002 173 646

## Description

The invention concerns certain novel quinazoline derivatives, or pharmaceutically acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors as well as paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen *et al*, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba *et al,* Biochimica et Biophysica Acta, 1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised in to 20 receptor tyrosine kinase and 10 non-receptor tyrosine kinase sub-families (Robinson *et al*, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands (such as Epidermal Growth Factor (EGF) for the EGF Receptor). Binding of ligand results in the activation of the receptor's kinase enzymatic activity that is encoded by the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112, 1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga *et al.,* Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors, it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish *et al.* Science, 1988, 242, 933, Kolibaba *et al,* Biochimica et Biophysica Acta, 1997, 133, F217-F248; Al-Obeidi *et al*, 2000, Oncogene, 19, 5690-5701; Mendelsohn *et al*, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn *et al*, 2000, Oncogene, 19, 6550-6565).

Amplification and/or activity of members of the erbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

European patent application EP 566 226 discloses certain 4-anilinoquinazolines that are receptor tyrosine kinase inhibitors.

International patent applications WO 96/33977, WO 96/33978, WO 96/33979, WO 96/33980, WO 96/33981, WO 97/30034, WO 97/38994 disclose that certain quinazoline derivatives which bear an anilino substituent at the 4-position and a substituent at the 6-and/or 7- position possess receptor tyrosine kinase inhibitory activity.

European patent application EP 837 063 discloses aryl substituted 4-aminoquinazoline derivatives carrying a moiety containing an aryl or heteroaryl group at the 6-or 7- position on the quinazoline ring. The compounds are stated to be useful for treating hyperproliferative disorders.

International patent applications WO 97/30035 and WO 98/13354 disclose certain 4-anilinoquinazolines substituted at the 7- position are vascular endothelial growth factor receptor tyrosine kinase inhibitors.

WO 00/55141 discloses 6,7-substituted 4-anilinoquinazoline compounds characterised in that the substituents at the 6-and/or 7-position carry an ester linked moiety (RO-CO).

WO 00/56720 discloses 6,7-dialkoxy-4-anilinoquinazoline compounds for the treatment of cancer or allergic reactions.

WO 02/41882 discloses 4-anilinoquinazoline compounds substituted at the 6- and/or 7- position by a substituted pyrrolidinyl-alkoxy or piperidinyl-alkoxy group.

WO 03/082290 discloses that certain 6,7-substituted 4-anilinoquinazoline compounds possess receptor tyrosine kinase inhibitory activity. A specific example of such a compound is 4-[(3-chloro-4-fluorophenyl)amino]-6-[1-(tert-butyloxycarbonyl)-piperidin-4-yl-oxy]-7-methoxy-quinazoline.

None of the above prior art discloses 4-(2,3-dihalogenoanilino)quinazoline or 4-(2,3,4-trihalogenoanilino)quinazoline compounds.

Copending International Patent Application No. PCT/GB03/01306 describes that certain 4-(2,3-dihalogenoanilino)quinazoline derivatives that possess potent anti-tumour activity, and in particular are selective against EGFR. A specific example of such a compound is 6-(1-acetylpiperidin-4-yloxy)-4-(3-chloro-2-fluoro anilino)-7-methoxyquinazoline.

The applicants have surprisingly found however that modification of a side chain and, optionally, adding a further substituent to the aniline group produces a select group of compounds with enhanced activity in that the compounds have a good erbB2 kinase inhibitory effect in addition to a EGF inhibitory effect, making them of particular clinical application in the treatment of tumours where both these kinases are implicated.

Without wishing to imply that the compounds disclosed in the present invention possess pharmacological activity only by virtue of an effect on a single biological process, it is believed that the compounds provide an anti-tumour effect by way of inhibition of two of the erbB family of receptor tyrosine kinases that are involved in the signal transduction steps which lead to the proliferation of tumour cells. In particular, it is believed that the compounds of the present invention provide an anti-tumour effect by way of inhibition of EGFR and/or erbB2 receptor tyrosine kinases.

According to a first aspect of the invention there is provided a quinazoline derivative of the Formula I: 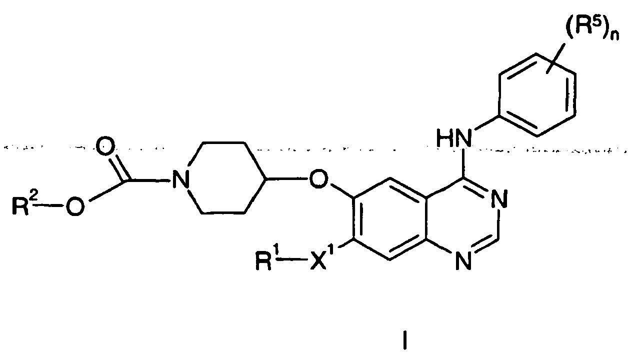
wherein n is 0, 1, 2 or 3,
each **R⁵** is independently selected from halogeno, cyano, nitro, hydroxy, amino, carboxy, sulfamoyl, trifluoromethyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylsulfamoyl, and N,N-di-[(1-6C)alkyl]sulfamoyl, C(O)NR⁶R⁷ where R⁶ and R⁷ are independently selected from hydrogen, optionally substituted (1-6C)alkyl, optionally substituted (3-8C)cycloalkyl or optionally substituted aryl, or R⁶ and R⁷ together with the nitrogen to which they are attached form an optionally substituted heterocyclic ring which may contain additional heteroatoms;
**X¹** is a direct bond or O;
**R¹** is selected from hydrogen and (1-6C)alkyl, wherein the (1-6C)alkyl group is optionally substituted by one or more substituents, which may be the same or different, selected from hydroxy and halogeno, and/or a substituent selected from amino, nitro, carboxy, cyano, halogeno, (1-6C)alkoxy, hydroxy(1-6C)alkoxy, (2-8C)alkenyl, (2-8C)alkynyl, (1-6C)alkylthio, (1-6C)alkylsulfinyl, (1-6C)alkylsulfonyl, (1-6C)alkylamino, di-[(1-6C)alkyl]amino, carbamoyl, N-(1-6C)alkylcarbamoyl, N,N di-[(1-6C)alkyllcarbamoyl, (2-6C)alkanoyl, (2-6C)alkanoyloxy, (2-6C)alkanoylamino,
N-(1-6C)alkyl-(2-6C)alkanoylamino, (1-6C)alkoxycarbonyl, sulfamoyl,
N-(1-6C)alkylsulfamoyl, N,N-di-[(1-6C)alkyllsulfamoyl, (1-6C)alkanesulfonylamino and N-(1-6C)alkyl-( 1-6C)alkanesulfonylamino;
**R²** is (1-6C)alkyl, (2-6C)alkenyl or (2-6C)alkynyl, any of which may be optionally substituted by fluoro, (1-6C)alkoxy, (1-6C)alkylthio, (1-6C)alkylsulfinyl, (1-6C)alkylsulfonyl, or a group of sub-formula (i) 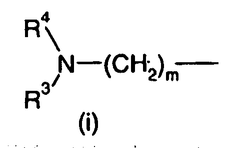
wherein m is 0, 1, 2 or 3;
**R³** and **R⁴** are independently selected from hydrogen or (1-6C)alkyl,
or R³ and R⁴ together with the nitrogen atom to which they are attached form a saturated 5 or 6 membered heterocyclic ring which optionally contains additional heteroatoms selected from oxygen, S, SO, SO₂ or NR⁸ where R⁸ is hydrogen, (1-6C)alkyl, (2-6C)alkenyl, (2-6C)alkynyl, (1-6C)alkylsulfonyl or (1-6C)alkylcarbonyl;
provided that the quinazoline derivative is not:
4-[(3-chloro-4-fluorophenyl)amino]-6-[1-(tert-butyloxycarbonyl)-piperidin-4-yl-oxy]-7-methoxy-quinazoline;
4-[(3-chloro-4-fluorophenyl)amino]-6-[1-(isopropyloxycarbonyl)-piperidin-4-yl-oxy]-7-methoxy-quinazoline;
4-[(3-ethynyl-phenyl)amino]-6-[1-(tert-butyloxycarbonyl)-piperidin-4-yl-oxy]-7-methoxyquinazoline; or
6-{[(1-tert-butoxycarbonyl)piperidin-4-yl]oxy}-4-(3-chloro-2-fluoroanilino)-7-methoxyquinazoline;
or a pharmaceutically acceptable salt thereof.

According to another aspect of the invention there is provided a quinazoline derivative of the Formula I: 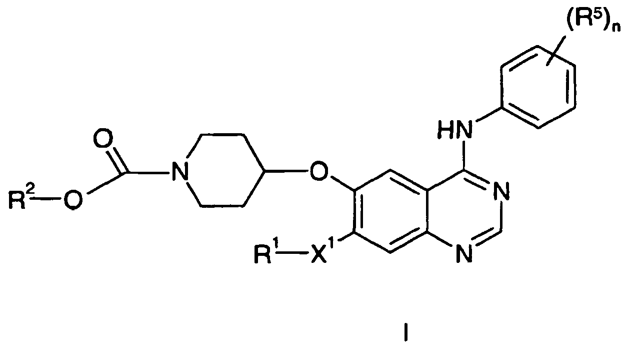 wherein n is 0, 1, 2 or 3,
each **R⁵** is independently selected from halogeno, cyano, nitro, hydroxy, amino, carboxy, sulfamoyl, trifluoromethyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylsulfamoyl, and N,N-di-[(1-6C)alkyl]sulfamoyl, C(O)NR⁶R⁷ where R⁶ and R⁷ are independently selected from hydrogen, optionally substituted (1-6C)alkyl, optionally substituted (3-8C)cycloalkyl or optionally substituted aryl, or R⁶ and R⁷ together with the nitrogen to which they are attached form an optionally substituted heterocyclic ring which may contain additional heteroatoms;
**X¹** is a direct bond or O;
**R¹** is selected from hydrogen and (1-6C)alkyl, wherein the (1-6C)alkyl group is optionally substituted by one or more substituents, which may be the same or different, selected from hydroxy and halogeno, and/or a substituent selected from amino, nitro, carboxy, cyano, halogeno, (1-6C)alkoxy, hydroxy(1-6C)alkoxy, (2-8C)alkenyl, (2-8C)alkynyl, (1-6C)alkylthio, (1-6C)alkylsulfinyl, (1-6C)alkylsulfonyl, (1-6C)alkylamino, di-[(1-6C)alkyl]amino, carbamoyl, N-(1-6C)alkylcarbamoyl, N,N di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (1-6C)alkoxycarbonyl, sulfamoyl, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino;
**R²** is (1-6C)alkyl, (2-6C)alkenyl or (2-6C)alkynyl, any of which may be optionally substituted by fluoro, (1-6C)alkoxy, (1-6C)alkylthio, (1-6C)alkylsulfinyl, (1-6C)alkylsulfonyl, or a group of sub-formula (i) 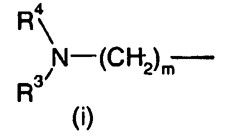
wherein m is 1, 2 or 3;
**R³** and **R⁴** are independently selected from hydrogen or (1-6C)alkyl,
or R³ and R⁴ together with the nitrogen atom to which they are attached form a saturated 5 or 6 membered heterocyclic ring which optionally contains additional heteroatoms selected from oxygen, S, SO, SO₂ or NR⁸ where R⁸ is hydrogen, (1-6C)alkyl, (2-6C)alkenyl, (2-6C)alkynyl, (1-6C)alkylsulfonyl or (1-6C)alkylcarbonyl;
provided that the quinazoline derivative is not:
4-[(3-chloro-4-fluorophenyl)amino]-6-[1-(tert-butyloxycarbonyl)-piperidin-4-yl-oxy]-7-methoxy-quinazoline;
4-[(3-chloro-4-fluorophenyl)amino]-6-[1-(isopropyloxycarbonyl)-piperidin-4-yl-oxy]-7-methoxy-quinazoline;
4-[(3-ethynyl-phenyl)amino]-6-[1-(tert-butyloxycarbonyl)-piperidin-4-yl-oxy]-7-methoxyquinazoline; or
6-{[(1-tert-butoxycarbonyl)piperidin-4-yl]oxy}-4-(3-chloro-2-fluoroanilino)-7-methoxyquinazoline;
or a pharmaceutically acceptable salt thereof.

In this specification the generic term "alkyl" includes both straight-chain and branched-chain alkyl groups such as propyl, isopropyl and tert-butyl, and (3-7C)cycloalkyl groups such as cyclopropyl, cyclobutyl, cyclopentyl, cyclohexyl and cycloheptyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclopentyl" are specific for that 5-membered ring only. An analogous convention applies to other generic terms, for example (1-6C)alkoxy includes methoxy, ethoxy, cyclopropyloxy and cyclopentyloxy, (1-6C)alkylamino includes methylamino, ethylamino, cyclobutylamino and cyclohexylamino, and di-[(1-6Calkyl]amino includes dimethylamino, diethylamino, N-cyclobutyl-N-methylamino and N-cyclohexyl-N-ethylamino.

The term "aryl" refers to aromatic hydrocarbon rings such as phenyl or naphthyl. The terms "heterocyclic" or "heterocyclyl" include ring structures that may be mono- or bicyclic and contain from 3 to 15 atoms, at least one of which, and suitably from 1 to 4 of which, is a heteroatom such as oxygen, sulfur or nitrogen. Rings may be aromatic, non-aromatic or partially aromatic in the sense that one ring of a fused ring system may be aromatic and the other non-aromatic. Particular examples of such ring systems include furyl, benzofuranyl, tetrahydrofuryl, chromanyl, thienyl, benzothienyl, pyridyl, piperidinyl, quinolyl, 1,2,3,4-tetrahydroquinolinyl, isoquinolyl, 1,2,3,4-tetrahydroisoquinolinyl, pyrazinyl, piperazinyl, pyrimidinyl, pyridazinyl, quinoxalinyl, quinazolinyl, cinnolinyl, pyrrolyl, pyrrolidinyl, indolyl, indolinyl, imidazolyl, benzimidazolyl, pyrazolyl, indazolyl, oxazolyl, benzoxazolyl, isoxazolyl, thiazolyl, benzothiazolyl, isothiazolyl, morpholinyl, 4H-1,4-benzoxazinyl, 4H-1,4-benzothiazinyl, 1,2,3-triazolyl, 1,2,4-triazolyl, oxadiazolyl, furazanyl, thiadiazolyl, tetrazolyl, dibenzofuranyl, dibenzothienyl oxiranyl, oxetanyl, azetidinyl, tetrahydropyranyl, oxepanyl, oxazepanyl, tetrahydro-1,4-thiazinyl, 1,1-dioxotetrahydro-1,4-thiazinyl, homopiperidinyl, homopiperazinyl, dihydropyridinyl, tetrahydropyridinyl, dihydropyrimidinyl, tetrahydropyrimidinyl, tetrahydrothienyl, tetrahydrothiopyranyl or thiomorpholinyl.

Particular examples of heterocyclic groups include tetrahydropyranyl, tetrahydrofuranyl or N-(1-6C)alkylpyrrolidine or N-(1-6C)alkylpiperidine.

Where rings include nitrogen atoms, these may carry a hydrogen atom or a substituent group such as an (1-6C)alkyl group if required to fulfil the bonding requirements of nitrogen, or they may be linked to the rest of the structure by way of the nitrogen atom. A nitrogen atom within a heterocyclyl group may be oxidized to give the corresponding N oxide.

Generally the compounds exhibit favourable physical properties such as a high solubility whilst retaining high antiproliferative activity. Furthermore, many of the compounds according to the present invention are inactive or only weakly active in a hERG assay.

It is to be understood that, insofar as certain of the compounds of Formula I defined above may exist in optically active or racemic forms by virtue of one or more asymmetrically substituted carbon and/or sulfur atoms, and accordingly may exist in, and be isolated as enantiomerically pure, a mixture of diastereoisomers or as a racemate. The present invention includes in its definition any racemic, optically-active, enantiomerically pure, mixture of diastereoisomers, stereoisomeric form of the compound of Formula (I), or mixtures thereof, which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

The invention relates to all tautomeric forms of the compounds of the Formula I that possess antiproliferative activity.

It is also to be understood that certain compounds of the Formula I may exist in solvated as well as unsolvated forms such as, for example, hydrated forms. It is to be understood that the invention encompasses all such solvated forms which possess antiproliferative activity.

It is also to be understood that certain compounds of the Formula I may exhibit polymorphism, and that the invention encompasses all such forms which possess antiproliferative activity.

Suitable values for the generic radicals referred to above include those set out below.

Suitable values for any of the R¹, R², R³, R⁴ or R⁵ groups as defined hereinbefore or hereafter in this specification include:-

| | |
|---|---|
| for halogeno | fluoro, chloro, bromo and iodo; |
| for (1-6C)alkyl: | methyl, ethyl, propyl, isopropyl, tert-butyl, pentyl |
| | and hexyl; |
| for (1-4C)alkyl: | methyl, ethyl, propyl, isopropyl and tert-butyl; |
| for (1-6C)alkoxy: | methoxy, ethoxy, propoxy, isopropoxy and butoxy; |
| for (2-8C)alkenyl: | vinyl, isopropenyl, allyl and but-2-enyl; |
| for (2-8C)alkynyl: | ethynyl, 2-propynyl and but-2-ynyl; |
| for (2-6C)alkenyloxy: | vinyloxy and allyloxy; |
| for (2-6C)alkynyloxy: | ethynyloxy and 2-propynyloxy; |
| for (1-6C)alkylthio: | methylthio, ethylthio and propylthio; |
| for (1-6C)alkylsulfinyl: | methylsulfinyl and ethylsulfinyl; |
| for (1-6C)alkylsulfonyl: | methylsulfonyl and ethylsulfonyl; |
| for (1-6C)alkylamino: | methylamino, ethylamino, propylamino, |
| | isopropylamino and butylamino; |
| for di-[(1-6C)alkyl]amino: | dimethylamino, diethylamino, N-ethyl- |
| | N-methylamino and diisopropylamino; |
| for (1-6C)alkoxycarbonyl: | methoxycarbonyl, ethoxycarbonyl, propoxycarbonyl |
| | and tert-butoxycarbonyl; |
| for N-(1-6C)alkylcarbamoyl: | N-methylcarbamoyl, N-ethylcarbamoyl, |
| | N-propylcarbamoyl and N-isopropylcarbamoyl; |
| for N,N-di-[(1-6C)alkyl]carbamoyl: | N,N-dimethylcarbamoyl, N-ethyl- |
| | N-methylcarbamoyl and N,N-diethylcarbamoyl; |
| for (2-6C)alkanoyl: | acetyl, propionyl and isobutyryl; |
| for (2-6C)alkanoyloxy: | acetoxy and propionyloxy; |
| for (2-6C)alkanoylamino: | acetamido and propionamido; |
| for N-(1-6C)alkyl-(2-6C)alkanoylamino: | N-methylacetamido and N-methylpropionamido; |
| for N-(1-6C)alkylsulfamoyl: | N-methylsulfamoyl, N-ethylsulfamoyl and |
| | N-isopropylsulfamoyl; |
| for N,N-di-[(1-6C)alkyl]sulfamoyl: | N,N-dimethylsulfamoyl and |
| | N-methyl-N-ethylsulfamoyl; |
| for (1-6C)alkanesulfonylamino: | methanesulfonylamino and ethanesulfonylamino; |
| for N-(1-6C)alkyl-(1-6C)alkanesulfonylamino: | N-methylmethanesulfonylamino and |
| | N-methylethanesulfonylamino; |
| for hydroxy-(1-6C)alkoxy: | hydroxymethoxy, 2-hydroxyethoxy, |
| | 1-hydroxyethoxy and 3-hydroxypropoxy. |

It is to be understood that when, R¹ is a group (1-6C)alkyl substituted by, for example amino to give for example a 2-aminoethyl group, it is the (1-6C)alkyl group that is attached to the group X¹ (or the quinazoline ring when X¹ is a direct bond).

When in this specification reference is made to a (1-4C)alkyl group it is to be understood that such groups refer to alkyl groups containing up to 4 carbon atoms. A skilled person will realise that representative examples of such groups are those listed above under (1-6C)alkyl that contain up to 4 carbon atoms, such as methyl, ethyl, propyl, isopropyl, butyl and tert-butyl. Similarly, reference to a (1-3C)alkyl group refers to alkyl groups containing up to 3 carbon atoms such as methyl, ethyl, propyl and isopropyl. A similar convention is adopted for the other groups listed above such as (1-4C)alkoxy, (2-4C)alkenyl, (2-4C)alkynyl and (2-4C)alkanoyl.

In the compound of Formula I hydrogen atoms are present at the 2, 5 and 8 positions on the quinazoline ring.

A suitable pharmaceutically-acceptable salt of a compound of the Formula I is, for example, an acid-addition salt of a compound of the Formula I, for example an acid-addition salt with an inorganic or organic acid such as hydrochloric, hydrobromic, sulfuric, trifluoroacetic, citric or maleic acid; or, for example, a salt of a compound of the Formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular examples of n are 1, 2 or 3, suitably 2 or 3.

Suitably each R⁵ is independently selected from halogeno, trifluoromethyl, (1-6C)alkyl, (2-8C)alkenyl, (2-8C)alkynyl or a group C(O)NR⁶R⁷ where R⁶ and R⁷ are as defined above.

In particular, each group R⁵ is independently selected from halogeno, such as chloro or fluoro.

Particular substituents for groups R⁶ and R⁷ where these are other than hydrogen, include halogeno, nitro, cyano, hydroxy, amino, carboxy, carbamoyl, sulfamoyl, trifluoromethyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkyl carbamoyl, N,N-di-[(1-6C)alkyl] carbamoyl, N-(1-6C)alkylsulfamoyl, N,N-di-[(1-6C)alkyl]sulfamoyl, (3-8C)cycloalkyl, aryl or heterocyclic groups.

Particular examples of aryl substituents for R⁶ or R⁷ include phenyl or naphthyl, particularly phenyl.

Particular examples of heterocyclic substituents for R⁶ or R⁷ include 5 or 6 membered heterocyclic rings such as furyl, tetrahydrofuryl, thienyl, pyridyl, piperidinyl, pyrazinyl, piperazinyl, pyrimidinyl, pyridazinyl, pyrrolyl, pyrrolidinyl, imidazolyl, pyrazolyl, oxazolyl, isoxazolyl, thiazolyl, isothiazolyl, morpholinyl, 1,2,3-triazolyl, 1,2,4-triazolyl, oxadiazolyl, furazanyl, thiadiazolyl or tetrazolyl.

When R⁶ and R⁷ together with the nitrogen to which they are attached form an optionally substituted heterocyclic ring, it is for example a 5 or 6 membered ring, which is saturated or unsaturated. Particular examples include piperidinyl, pyrrolidinyl, morpholinyl or thiomorpholino. Alternatively, R⁶ and R⁷ together form a (3-6C)alkenyl group.

Heterocyclic rings formed by R⁶ and R⁷ together with the nitrogen atom to which they are attached may be substituted by any of the groups mentioned above in relation to R⁶ and R⁷. In addition, these rings may be substituted by one or more (1-6C) alkyl groups, which may themselves be optionally substituted by one or more groups selected from halogeno, nitro, cyano, hydroxy, amino, carboxy, carbamoyl, sulfamoyl, trifluoromethyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl] carbamoyl, N-(1-6C)alkylsulfamoyl, or N,N-di-[(1-6C)alkyl]sulfamoyl.

An exemplary group of substituents for R⁶ or R⁷ where they are other than hydrogen are cyano, hydroxy, (2-8C)alkenyl, (2-8C)alkynyl, (1-6C)alkoxy, (1-6C)alkylthio, (1-6C)alkylamino, aryl such as phenyl or heterocyclic groups such as furyl, and additionally, where R⁶ and R⁷ together with the nitrogen atom to which they are attached form a ring, (1-6C) alkyl groups such as methyl.

Where n is 1, 2 or 3, one group R⁵ is suitably at an ortho- position on the benzene ring.

Where n is 1, 2 or 3, one group R⁵ is suitably at a meta- position on the benzene ring.

Thus, when n is 1, the group R⁵ is suitably at an ortho- or a meta-position on the benzene ring,

In one aspect of the invention, when n is 2, the first R⁵ group is suitably at a meta-position and the second R⁵ group is suitably at an ortho- or para- position on the benzene ring, and thus the ring has substituents at 2- and 3- or 3- and 4- positions on the benzene ring.

In another aspect of the invention, when n is 2 or 3, the first R⁵ group is suitably at an ortho- position, the second R⁵ group is suitably at a meta- position and, optionally (when n is 3), the third R⁵ group is suitably at a para- position on the benzene ring. Thus, when n is 2, the ring suitably has substituents at 2- and 3- positions on the benzene ring and when n is 3, the ring suitably has substituents at 2-, 3- and 4- positions on the benzene ring.

The applicants have surprisingly found that quinazoline derivatives having substituents (for example halogeno substituents) at 2- and 3- positions or at 2-, 3- and 4-positions on the benzene ring compared to quinazoline derivatives having substituents at 3-and 4- positions on the benzene ring produces a select group of compounds with enhanced activity in that the compounds have an increased potency against erbB2 and/or EGFR (particularly erbB2) receptor tyrosine kinases in cellular assays. It is believed that quinazoline derivatives having substituents (for example halogeno substituents) at 2- and 3-positions or at 2-, 3- and 4- positions on the benzene ring will also have an increased potency against both erbB2 and/or EGFR (particularly erbB2) receptor tyrosine kinases *in vivo.*

Suitably when n is 2 or 3, each R⁵ group is the same or different halogeno atom, such as chloro or fluoro. Suitably, at least one R⁵ group is fluoro, which at least one fluoro is suitably positioned at an ortho- (2-) position on the benzene ring.

Suitably when n is 2, each R⁵ group is the same or different halogeno atom. In particular, one R⁵ group is chloro, and this is preferably at a meta- (3-) position on the benzene ring to which it is attached, and the other R⁵ group is fluoro which is preferably at an ortho- (2-) or a para- (4-) (preferably an ortho- (2-)) position on the benzene ring.

Suitably when n is 3, each R⁵ group is the same or different halogeno atom. In particular, one R⁵ group is chloro, and this is preferably at a meta- (3-) position on the benzene ring to which it is attached, and the other two R⁵ groups are each fluoro, which are preferably at an ortho- (2-) and a para- (4-) position respectively on the benzene ring.

Thus particular examples of the group of sub-formula (ii): 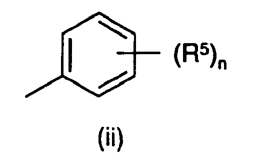 in Formula (I) are groups of sub-formula (iii): 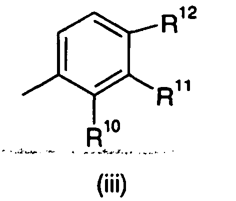 wherein (a) one of R¹⁰ or R¹² is hydrogen and the other is halogeno, such as chloro or fluoro, and particularly fluoro, and R¹¹ is halogeno such as chloro or fluoro, and particularly chloro, or (b) R¹⁰ is halogeno, such as chloro or fluoro, and particularly fluoro, R¹¹ is halogeno such as chloro or fluoro, and particularly chloro, and R¹² is hydrogen or halogeno, such as chloro or fluoro, and particularly fluoro, or (c) R¹⁰ is fluoro, R¹¹ is chloro, and R¹² is hydrogen or fluoro. In particular, R¹⁰, R¹¹ and R¹² are as defined in (b) and/or (c).

In one embodiment, when n is 2, each R⁵ group is the same or different halogeno atom (such as fluoro and/or chloro) and the first R⁵ group is at an ortho- position and the second R⁵ group is at a meta- position on the benzene ring, then R² is not (optionally substituted) (1-6C)alkyl. In particular, R² is not (1-6C)alkyl optionally substituted by fluoro, (1-6C)alkoxy or a group of the sub-formula (i) 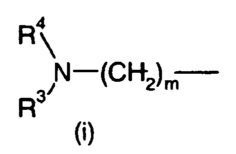 wherein m is 0 and R³ and R⁴ are independently selected from hydrogen or (1-4C)alkyl.

Suitably X¹ is oxygen.

In particular R¹ is selected from hydrogen, (1-6C)alkyl and (1-6C)alkoxy(1-6C)alkyl, wherein any (1-6C)alkyl group in R¹ optionally bears one or more (suitably 1 or 2) hydroxy or halogeno substituents. More particularly, R¹ is selected from (1-6C)alkyl, preferably from (1-4C)alkyl and even more preferably from (1-2C)alkyl. For example, R¹ may be methyl.

For instance, R¹-X¹- is selected from methoxy, ethoxy, isopropyloxy, cyclopropylmethoxy, 2-hydroxyethoxy, 2-fluoroethoxy, 2-methoxyethoxy, 2,2-difluoroethoxy, 2,2,2-trifluoroethoxy or 3-hydroxy-3-methylbutoxy.

In particular R'-X- is selected from hydrogen, (1-4C)alkoxy and (1-4C)alkoxy(1-4C)alkoxy. For instance, R'-X- is selected from hydrogen, methoxy, ethoxy and 2-methoxyethoxy. A particular example of a group R¹-X¹- is methoxy.

Suitably R² is (1-6C)alkyl (particularly (1-3C)alkyl, more particularly (1-2C)alkyl) which is optionally substituted by a fluoro, (1-6C)alkoxy, (1-6C)alkylthio, (1-6)alkylsulfinyl (1-6C)alkylsulfonyl, or a group of sub-formula (i) as defined above. A particular example of a substituent for R² is a group of sub-formula (i) as defined above.

In particular R² is a (1-3C)alkyl group such as methyl or ethyl, which is optionally substituted by a group of sub-formula (i) as defined above. When R² contains a substituent of sub-formula (i), m is suitably 0, 1 or 2.

When R² contains a substituent of sub-formula (i), m is suitably 1 or 2, and preferably 2. In another aspect, m is particularly 0 or 1.

When R³ and R⁴ together with the nitrogen atom to which they are attached form a saturated 5 or 6 membered heterocyclic ring which optionally contains additional heteroatoms, this suitably contains additional heteroatoms selected from O and NR⁸, where R⁸ is as defined in relation to Formula I.

When R³ and R⁴ together with the nitrogen atom to which they are attached form a saturated 5 or 6 membered heterocyclic ring which optionally contains additional heteroatoms, this suitably comprises a pyrrolidine ring, a morpholine ring, a piperidine ring, or a piperazine ring which is optionally substituted on the available nitrogen atom by a group R⁸ as defined above. Particular examples of R⁸ groups include (1-3C) alkyl such as methyl; (1-3C)alkylsulfonyl such as methyl sulfonyl; (1-3C)alkylcarbonyl, such as acetyl; or (2-4C)alkenyl such allyl; or (2-4C)alkynyl such as propargyl. In particular R⁸ is a (1-3C)alkyl group such as methyl.

Alternatively, the groups R³ and R⁴ may suitably be independently selected from (1-6C)alkyl, particularly from (1-3C)alkyl, such as methyl and ethyl. For example, each of the groups R³ and R⁴ may suitably be (1-3C)alkyl, such as, in one aspect, each of the groups R³ and R⁴ may be ethyl.

Particular examples of groups R² include methyl, 2-(pyrrolidin-1-yl)ethyl, 2-(dimethylamino)ethyl, 2-(diethylamino)ethyl, 2-(piperidinyl)ethyl, 2-(morpholin-4-yl)ethyl or 2-(4-methylpiperazin-1-yl)ethyl. More particularly, examples of groups R² include methyl, 2-(pyrrolidin-1-yl)ethyl, 2-(diethylamino)ethyl, 2-(piperidin-1-yl)ethyl, 2-(morpholin-4-yl)ethyl or 2-(4-methylpiperazin-1-yl)ethyl.

In a particular embodiment, R² is methyl. In an alternative embodiment, R² is selected from 2-(piperidin-1-yl)ethyl, 2-(4-methylpiperazin-1-yl)ethyl and 2-(pyrrolidin-1-yl)ethyl, particularly R² is 2-(pyrrolidin-1-yl)ethyl. In another alternative embodiment, R² is selected from 2-(dimethylamino)ethyl and 2-(diethylamino)ethyl. In another alternative embodiment, R² is 2-(morpholin-4-yl)ethyl.

Particular examples of the compounds of Formula I are compounds of Formula IA: 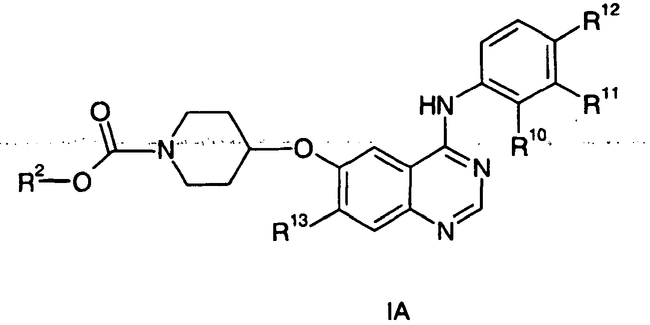 where R² is as defined above in relation to Formula I, R¹⁰, R¹¹ and R¹² are as defined above in relation to sub-formula (iii), and R¹³ is selected from hydrogen, methoxy, ethoxy and 2-methoxyethoxy, and especially methoxy;

For the avoidance of any doubt, when the compounds of Formula I are defined as compounds of Formula IA, the quinazoline derinvative is not:
4-[(3-chloro-4-fluorophenyl)amino]-6-[1-(tert-butyloxycarbonyl)-piperidin-4-yl-oxy]-7-methoxy-quinazoline;
4-[(3-chloro-4-fluorophenyl)amino]-6-[1-(isopropyloxycarbonyl)-piperidin-4-yl-oxy]-7-methoxy-quinazoline; or
6-{[(1-tert-butoxycarbonyl)piperidin-4-yl]oxy}-4-(3-chloro-2-fluoroanilino)-7-methoxyquinazoline;
or a pharmaceutically acceptable salt thereof.

Other particular examples of the compounds of Formula I are compounds of the Formulae IB and/or IC: 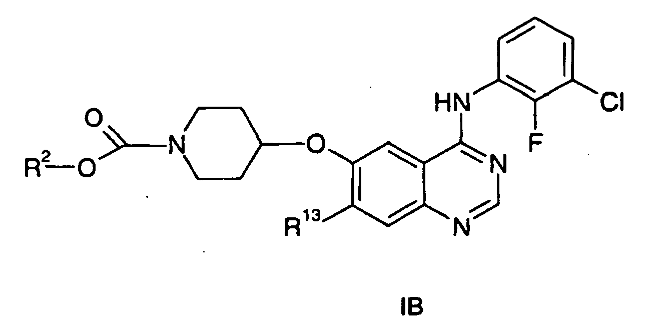 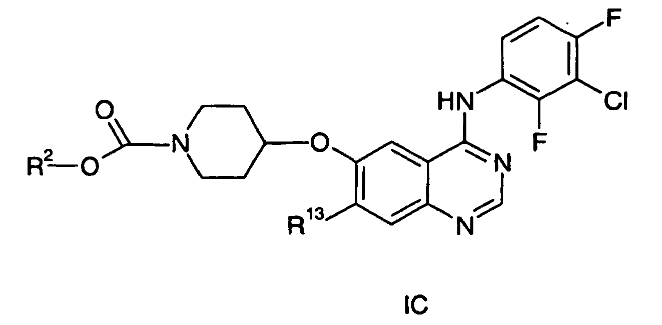 where R² is as defined above in relation to Formula I and R¹³ is selected from hydrogen, methoxy, ethoxy and 2-methoxyethoxy, and especially methoxy.

For the avoidance of any doubt, when the compounds of Formula I are defined as compounds of Formula IB, the quinazoline derivative is not 6-{[(1-tert-butoxycarbonyl) piperidin-4-yl]oxy}-4-(3-chloro-2-fluoroanilino)-7-methoxyquinazoline, or a pharmaceutically acceptable salt thereof.

Other particular examples of the compounds of Formula I are compounds of the Formula ID: 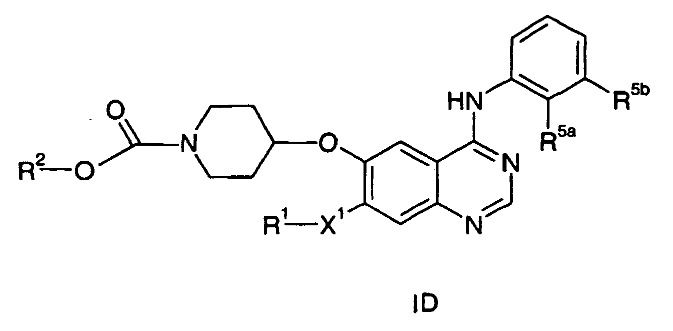 wherein:
**R^{5a}** and **R^{5b}** are independently selected from halogeno (for example fluoro and/or chloro);
**X¹** is a direct bond or O;
**R¹** is selected from hydrogen and (1-6C)alkyl, wherein the (1-6C)alkyl group is optionally substituted by one or more substituents, which may be the same or different, selected from hydroxy and halogeno, and/or a substituent selected from amino, nitro, carboxy, cyano, halogeno, (1-6C)alkoxy, hydroxy(1-6C)alkoxy, (2-8C)alkenyl, (2-8C)alkynyl, (1-6C)alkylthio, (1-6C)alkylsulfinyl, (1-6C)alkylsulfonyl, (1-6C)alkylamino, di-[(1-6C)alkyl]amino, carbamoyl, N-(1-6C)alkylcarbamoyl, N,N di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (1-6C)alkoxycarbonyl, sulfamoyl, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino;
**R²** is (1-6C)alkyl, wherein the (1-6C)alkyl group is optionally substituted by fluoro, (1-6C)alkoxy, or a group of sub-formula (iv) 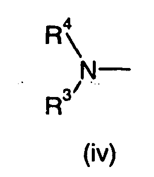
wherein **R³** and **R⁴** are independently selected from hydrogen or (1-4C)alkyl,
or R³ and R⁴ together with the nitrogen atom to which they are attached form a saturated 5 or 6 membered heterocyclic ring which optionally contains additional heteroatoms selected from oxygen, S, SO, SO₂ or NR⁸ where R⁸ is hydrogen, (1-4C)alkyl or (1-4C)alkylsulfonyl;
or a pharmaceutically acceptable salt thereof.

For the avoidance of any doubt, when the compounds of Formula I are defined as compounds of Formula ID, the quinazoline derivative is not 6-{[(1-tert-butoxycarbonyl) piperidin-4-yl]oxy}-4-(3-chloro-2-fluoroanilino)-7-methoxyquinazoline, or a pharmaceutically acceptable salt thereof.

In the compounds of the Formula ID, the group R² is suitably (1-6C)alkyl, particularly unsubstituted (1-6C)alkyl. For example, the group R² may be methyl or ethyl, particularly methyl.

In the compounds of the Formula ID, X¹ is suitably oxygen. R¹ is suitably selected from hydrogen and (1-6C)alkyl, wherein any (1-6C)alkyl group in R¹ optionally bears one or more (suitably 1 or 2) hydroxy or halogeno substituents. More particularly, R¹ is selected from (1-6C)alkyl, preferably from (1-4C)alkyl and even more preferably from (1-2C)alkyl. For example, R¹ may be methyl. A particular example of a group R¹-X¹ in the compounds of Formula ID is methoxy.

It would be clear to a person skilled in the art that particular novel compounds of the invention include those compounds of the Formula I (including IA, IB, IC and ID) in which, unless otherwise stated, each of R¹, R², R³, R⁴, R⁵, X¹, m and n has any of the meanings as hereinbefore defined.

Examples of compounds of Formula I include, for example, one or more of:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl} piperidin-4-yl]oxy}quinazoline;
4-(3-Chloro-2-fluoroanilino)-7-methoxy-6-{[1-2-(N,N-dimethylamino)ethoxycarbonyl) piperidin-4-yl]oxy}quinazoline;
4-(3-Chloro-4-fluoroanilino)-7-methoxy-6-{[1-{2-(pyrrolidin-1-yl)ethoxycarbonyl} piperidin-4-yl]oxy}quinazoline;
4-(3-Chloroanilino)-7-methoxy-6-{[1-{2-(pyrrolidin-1-yl)ethoxycarbonyl}piperidin-4-yl]oxy}quinazoline;
4-(3-Chloro-2,4-difluoroanilino)-7-methoxy-6-{[1-(methoxycarbonyl)piperidin-4-yl]oxy}quinazoline;
4-(3-Chloro-2,4-difluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-{2-(piperidin-1-yl)ethoxycarbonyl} piperidin-4-yl]oxy}quinazoline;
4-(3-Chloro-2-fluoroanilino)-7-methoxy-6-{[1-{2-(piperidin-1-yl)ethoxycarbonyl} piperidin-4-yl]oxy}quinazoline;
4-(3-Chloro-2-fluoroanilino)- 6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(morpholin-4-yl)ethoxycarbonyl} piperidin-4-yl]oxy}quinazoline; and
4-(3-Chloro-2-fluoroanilino)-7-methoxy-6-{[1-{2-(4-methylpiperidin-1-yl) ethoxycarbonyl}piperidin-4-yl]oxy}quinazoline;
or a pharmaceutically acceptable salt thereof.

Preferred examples of compounds of Formula I include, for example, one or more of:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-(methoxycarbonyl)piperidin-4-yl] oxy}quinazoline;
4-(3-Chloro-2,4-difluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-{2-(piperidin-1-yl)ethoxycarbonyl} piperidin-4-yl]oxy}quinazoline;
4-(3-Chloro-2-fluoroanilino)-7-methoxy-6-{[1-{2-(piperidin-1-yl)ethoxycarbonyl} piperidin-4-yl]oxy}quinazoline;
4-(3-Chloro-2-fluoroanilino)- 6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(morpholin-4-yl)ethoxycarbonyl} piperidin-4-yl]oxy}quinazoline; and
4-(3-Chloro-2-fluoroanilino)-7-methoxy-6-{[1-{2-(4-methylpiperidin-1-yl) ethoxycarbonyl }piperidin-4-yl]oxy}quinazoline;
or a pharmaceutically acceptable salt thereof.

A particular group of examples of quinazoline derivatives of the Formula IA includes one or more of:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl}piperidin-4-yl]oxy}quinazoline;
4-(3-Chloro-2-fluoroanilino)-7-methoxy-6-{[1-2-(N,N-dimethylamino)ethoxycarbonyl) piperidin-4-yl]oxy}quinazoline;
4-(3-Chloro-4-fluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-(methoxycarbonyl)piperidin-4-yl]oxy}quinazoline;
4-(3-Chloro-2,4-difluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-{2-(piperidin-1-yl)ethoxycarbonyl} piperidin-4-yl]oxy}quinazoline;
4-(3-Chloro-2-fluoroanilino)-7-methoxy-6-{[1-{2-(piperidin-1-yl)ethoxycarbonyl} piperidin-4-yl]oxy}quinazoline;
4-(3-Chloro-2-fluoroani lino)- 6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(morpholin-4-yl)ethoxycarbonyl} piperidin-4-yl]oxy}quinazoline; and
4-(3-Chloro-2-fluoroanilino)-7-methoxy-6-{[1-{2-(4-methylpiperidin-1-yl) ethoxycarbonyl}piperidin-4-yl]oxy}quinazoline;
or a pharmaceutically acceptable salt thereof.

A particular group of examples of quinazoline derivatives of the Formula IB includes one or more of:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl} piperidin-4-yl]oxy}quinazoline;
4-(3-Chloro-2-fluoroanilino)-7-methoxy-6-{[1-2-(N, N-dimethylamino)ethoxycarbonyl) piperidin-4-yl]oxy}quinazoline;
4-(3-Chloro-2-fluoroanilino)-7-methoxy-6-{[1-{2-(piperidin-1-yl)ethoxycarbonyl} piperidin-4-yl]oxy}quinazoline;
4-(3-Chloro-2-fluoroanilino)- 6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(mocpholin-4-yl)ethoxycarbonyl} piperidin-4-ylloxylquinazoline; and
4-(3-Chloro-2-fluoroanilino)-7-methoxy-6-{[1-{2-(4-methylpiperidin-1-yl) ethoxycarbonyl}piperidin-4-yl]oxy}quinazoline;
or a pharmaceutically acceptable salt thereof.

A particular group of examples of quinazoline derivatives of the Formula IC includes one or more of:
4-(3-Chloro-2,4-difluoroanilino)-7-methoxy-6-{[1-(methoxycarbonyl)piperidin-4-yl]oxy}quinazoline;
4-(3-Chloro-2,4-difluoroanilino)-7-methoxy-6-{[1-{2-(pyrrolidin-1-yl)ethoxycarbonyl} piperidin-4-yl]oxy}quinazoline; and
4-(3-Chloro-2,4-difluoroanilino)-7-methoxy-6-{[1-{2-(piperidin-1-yl)ethoxycarbonyl} piperidin-4-yl]oxy}quinazoline;
or a pharmaceutically acceptable salt thereof.

A particular example of a quinazoline derivative of the Formula ID is:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl} piperidin-4-yl]oxy}quinazoline;
4-(3-Chloro-2-fluoroanilino)-7-methoxy-6-{[1-2-(N,N-dimethylamino)ethoxycarbonyl) piperidin-4-yl]oxy}quinazoline;
4-(3-Chloro-2-fluoroanilino)-7-methoxy-6-{[1-{2-(piperidin-1-yl)ethoxycarbonyl} piperidin-4-yl]oxy}quinazoline;
4-(3-Chloro-2-fluoroanilino)- 6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(morpholin-4-yl)ethoxycarbonyl} piperidin-4-yl]oxy}quinazoline; and
4-(3-Chloro-2-fluoroanilino)-7-methoxy-6-{[1-{2-(4-methylpiperidin-1-yl) ethoxycarbonyl }piperidin-4-yl]oxy}quinazoline;
or a pharmaceutically acceptable salt thereof.

Preferred compounds of Formula I are, for example, one or more of:
4-(3-Chloro-2-fluoroanilino)-7-methoxy-6-{[1-(methoxycarbonyl)piperidin-4-yl]oxy} quinazoline; and
4-(3-Chloro-2-fluoroanilino)-7-methoxy-6-{[1-{2-(pyrrolidin-1-yl)ethoxycarbonyl} piperidin-4-yl]oxy}quinazoline,
or a pharmaceutically acceptable salt thereof.

### Synthesis of Quinazoline Derivatives of the Formula I

A further aspect the present invention provides a process for preparing a quinazoline derivative of Formula I or a pharmaceutically-acceptable salt thereof. It will be appreciated that during certain of the following processes certain substituents may require protection to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons). Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Thus, if reactants include, for example, groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluoroacetic acid, or for example a benzyl group which may be removed, for example, by hydrogenation over a catalyst such as palladium-on-carbon.
Resins may also be used as a protecting group.

The protecting groups may be removed at any convenient stage in the synthesis using conventional techniques well known in the chemical art.

A quinazoline derivative of the Formula I, or a pharmaceutically acceptable salt thereof, may be prepared by any process known to be applicable to the preparation of chemically-related compounds. Such processes, when used to prepare a quinazoline derivative of the Formula I, or a pharmaceutically acceptable salt thereof, are provided as a further feature of the invention and are illustrated by the following representative examples. Necessary starting materials may be obtained by standard procedures of organic chemistry (see, for example, Advanced Organic Chemistry (Wiley-Interscience), Jerry March). The preparation of such starting materials is described within the accompanying non-limiting Examples. Alternatively, necessary starting materials are obtainable by analogous procedures to those illustrated which are within the ordinary skill of an organic chemist. Information on the preparation of necessary starting materials or related compounds (which may be adapted to form necessary starting materials) may also be found in the following Patent and Application Publications, the contents of the relevant process sections of which are hereby incorporated herein by reference: WO94/27965, WO 95/03283, WO 96/33977, WO 96/33978, WO 96/33979, WO 96/33980, WO 96/33981, WO 97/30034, WO 97/38994, WO01/66099, US 5,252,586, EP 520 722, EP 566 226, EP 602 851 and EP 635 507.

The present invention also provides that quinazoline derivatives of the Formula I, or pharmaceutically acceptable salts thereof, can be prepared by a process (a) to (k) as follows (wherein the variables are as defined above unless otherwise stated):
**Process (a)** By reacting a compound of the Formula II: 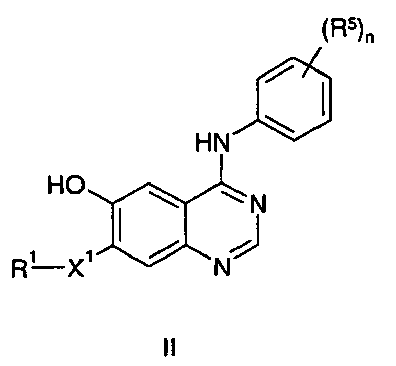 wherein R¹, X¹, R⁵ and n have any of the meanings defined hereinbefore except that any functional group is protected if necessary,
   with a compound of the Formula III: 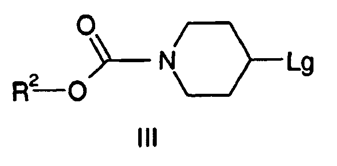 wherein R² has any of the meanings defined hereinbefore except that any functional group is protected if necessary and Lg is a displaceable group, wherein the reaction is conveniently performed in the presence of a suitable base,
   and whereafter any protecting group that is present is removed by conventional means.
   A convenient displaceable group Lg is, for example, a halogeno, alkanesulfonyloxy or arylsulfonyloxy group, for example a chloro, bromo, methanesulfonyloxy, 4-nitrobenzenesulfonyloxy or toluene-4-sulfonyloxy group (suitably a methanesulfonyloxy, 4-nitrobenzenesulfonyloxy or toluene-4-sulfonyloxy group).
   The reaction is advantageously carried out in the presence of base. A suitable base is, for example, an organic amine base such as, for example, di-isopropylethylamine, pyridine, 2,6-lutidine, collidine, 4-dimethylaminopyridine, triethylamine, N-methylmoipholine or diazabicyclo[5.4.0]undec-7-ene, or for example, an alkali metal or alkaline earth metal carbonate or hydroxide, for example sodium carbonate, potassium carbonate, cesium carbonate, calcium carbonate, sodium hydroxide or potassium hydroxide. Alternatively such a base is, for example, an alkali metal hydride, for example sodium hydride, an alkali metal or alkaline earth metal amide, for example sodium amide or sodium bis(trimethylsilyl)amide, or a sufficiently basic alkali metal halide, for example cesium fluoride or sodium iodide. The reaction is suitably effected in the presence of an inert solvent or diluent, for example an alkanol or ester such as methanol, ethanol, 2-propanol or ethyl acetate, a halogenated solvent such as methylene chloride, trichloromethane or carbon tetrachloride, an ether such as tetrahydrofuran or 1,4-dioxan, an aromatic hydrocarbon solvent such as toluene, or (suitably) a dipolar aprotic solvent such as N,N-dimethylformamide, N,N-dimethylacetamide, N-methylpyrrolidin-2-one or dimethylsulfoxide. The reaction is conveniently effected at a temperature in the range, for example, 10 to 150°C (or the boiling point of the solvent), suitably in the range 20 to 90°C.
   A particularly suitable base is cesium fluoride. This reaction is suitably performed in an inert dipolar aprotic solvent such as N,N-dimethylacetamide or N,N-dimethylformamide. The reaction is suitably carried out at a temperature of from 25 to 85°C.
**Process (b)** By modifying a substituent in or introducing a substituent into another quinazoline derivative of Formula I or a pharmaceutically acceptable salt thereof, as hereinbefore defined except that any functional group is protected if necessary, and whereafter any protecting group that is present is removed by conventional means.
   Methods for converting substituents into other substituents are known in the art. For example an alkylthio group may be oxidised to an alkylsulfinyl or alkylsulfonyl group, a cyano group reduced to an amino group, a nitro group reduced to an amino group, a hydroxy group alkylated to a methoxy group, a bromo group converted to an alkylthio group, an amino group may be acylated to give an alkanoylamino group (for example by reaction with a suitable acid chloride or acid anhydride) or an alkanoyloxy group may be hydrolysed to a hydroxy group (for example an acetyloxyacetyl group may be converted to a hydroxyacetyl group). Conveniently, one R¹ group may be converted into another R¹ group as a final step in the preparation of a compound of the Formula I.
**Process (c)** By reacting a compound of Formula IV: 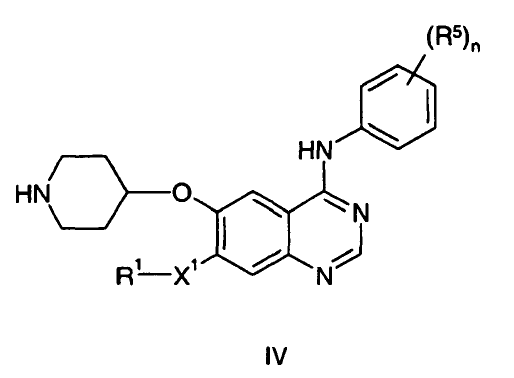 where R¹, X¹, R⁵ and n are as defined in relation to Formula I, with a compound of Formula V: 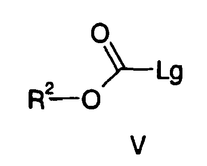 wherein R² is as defined above, and Lg is a displaceable group (for example halogeno such as chloro or bromo, or 1-imidazolyl). The reactions described above are conveniently performed in the presence of a suitable base (such as those described above in process (a), for example potassium carbonate or di-isopropylethylamine) and conveniently in the presence of an inert solvent or diluent (for example the inert solvents and diluents described in process (a) such as acetonitrile, N,N-dimethylacetamide, methanol, ethanol or methylene chloride).
**Process (d)** By removal of a protecting group from a quinazoline derivative of Formula I, or a pharmaceutically acceptable salt thereof.
   Suitable methods for removal of protecting groups are well known and are discussed herein. For example for the production of those compounds of the Formula I wherein R¹ contains a primary or secondary amino group, the cleavage of the corresponding compound of Formula I wherein R¹ contains a protected primary or secondary amino group.
   Suitable protecting groups for an amino group are, for example, any of the protecting groups disclosed hereinbefore for an amino group. Suitable methods for the cleavage of such amino protecting groups are also disclosed hereinbefore. In particular, a suitable protecting group is a lower alkoxycarbonyl group such as a tert-butoxycarbonyl group which may be cleaved under conventional reaction conditions such as under acid-catalysed hydrolysis, for example in the presence of trifluoroacetic acid.
**Process (e)** By reacting a compound of the Formula II as hereinbefore defined with a compound of the Formula III as defined hereinbefore except Lg is OH under Mitsunobu conditions, and whereafter any protecting group that is present is removed by conventional means.
   Suitable Mitsunobu conditions include, for example, reaction in the presence of a suitable tertiary phosphine and a di-alkylazodicarboxylate in an organic solvent such as THF, or suitably dichloromethane and in the temperature range 0°C - 60°C, but suitably at ambient temperature. A suitable tertiary phosphine includes for example tri-n-butylphosphine or suitably tri-phenylphosphine. A suitable di-alkylazodicarboxylate includes for example diethyl azodicarboxylate (DEAD) or suitably di-tert-butyl azodicarboxylate. Details of Mitsunobu reactions are contained in Tet. Letts., 31, 699, (1990); The Mitsunobu Reaction, D.L.Hughes, Organic Reactions, 1992, Vo1.42, 335-656 and Progress in the Mitsunobu Reaction, D.L.Hughes, Organic Preparations and Procedures International, 1996, Vol.28, 127-164.
**Process (f)** For the preparation of those compounds of the Formula I wherein R¹-X¹ is a hydroxy group by the cleavage of a quinazoline derivative of the Formula I wherein R¹-X¹ is a (1-6C)alkoxy group.
   The cleavage reaction may conveniently be carried out by any of the many procedures known for such a transformation. The cleavage reaction of a compound of the Formula I wherein R¹ is a (1-6C)alkoxy group may be carried out, for example, by treatment of the quinazoline derivative with an alkali metal (1-6C)alkylsulfide such as sodium ethanethiolate or, for example, by treatment with an alkali metal diarylphosphide such as lithium diphenylphosphide. Alternatively the cleavage reaction may conveniently be carried out, for example, by treatment of the quinazoline derivative with a boron or aluminium trihalide such as boron tribromide, or by reaction with an organic or inorganic acid, for example trifluoroacetic acid. Such reactions are suitably carried out in the presence of a suitable inert solvent or diluent as defined hereinbefore. A preferred cleavage reaction is the treatment of a quinazoline derivative of the Formula I with pyridine hydrochloride. The cleavage reactions are suitably carried out at a temperature in the range, for example, from 10 to 200°C. In some cases this may be achieved at temperatures, for example from 25 to 80°C, but in other cases, for instance using a pyridine hydrochloride deprotection, melting typically at 160-200°C is suitable.
**Process (g)** For the preparation of those compounds of the Formula I wherein X¹ is O, by the reaction of a compound of the Formula VI: 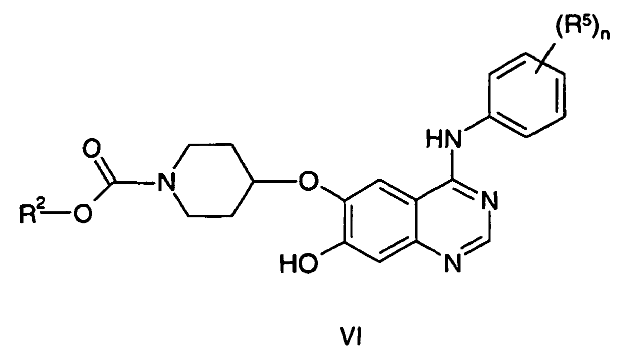 wherein R², R⁵ and n have any of the meanings defined hereinbefore except that any functional group is protected if necessary, with a compound of the formula R¹-Lg, wherein R¹ has any of the meanings defined hereinbefore, except that any functional group is protected if necessary and Lg is a displaceable group, wherein the reaction is conveniently performed in the presence of a suitable base;
   and whereafter any protecting group that is present is removed by conventional means.
   Suitable displaceable groups, Lg, are as hereinbefore defined for process (a), for example chloro or bromo. The reaction is suitably performed in the presence of a suitable base. Suitable solvents, diluents and bases include, for example those hereinbefore described in relation to process (a) above. Alternatively, Lg may be OH whereupon the reaction is carried out under Mitsunobu conditions, as described in process (e) above.
**Process (h)** For the preparation of those compounds of the Formula I wherein R¹ contains a (1-6C)alkoxy or substituted (1-6C)alkoxy group or a (1-6C)alkylamino or substituted (1-6C)alkylamino group, the alkylation, conveniently in the presence of a suitable base as defined hereinbefore for process (a), of a quinazoline derivative of the Formula I wherein or R¹ contains a hydroxy group or a primary or secondary amino group as appropriate.
   A suitable alkylating agent is, for example, any agent known in the art for the alkylation of hydroxy to alkoxy or substituted alkoxy, or for the alkylation of amino to alkylamino or substituted alkylamino, for example an alkyl or substituted alkyl halide, for example a (1-6C)alkyl chloride, bromide or iodide or a substituted (1-6C)alkyl chloride, bromide or iodide, conveniently in the presence of a suitable base as defined hereinbefore, in a suitable inert solvent or diluent as defined hereinbefore and at a temperature in the range, for example, 10 to 140°C, conveniently at or near ambient temperature. An analogous procedure may be used to introduce optionally substituted (2-6C)alkanoyloxy, (2-6C)alkanoylamino and (1-6C)alkanesulfonylamino groups into R¹.
   Conveniently for the production of those compounds of the Formula I wherein R¹ contains a (1-6C)alkylamino or substituted (1-6C)alkylamino group, a reductive amination reaction may be employed using formaldehyde or a (2-6C)alkanolaldehyde (for example acetaldehyde or propionaldehyde). For example, for the production of those compounds of the Formula I wherein R¹ contains an N-methyl group, the corresponding compound containing a N-H group may be reacted with formaldehyde in the presence of a suitable reducing agent. A suitable reducing agent is, for example, a hydride reducing agent, for example formic acid, an alkali metal aluminium hydride such as lithium aluminium hydride, or, suitably, an alkali metal borohydride such as sodium borohydride, sodium cyanoborohydride, sodium triethylborohydride, sodium trimethoxyborohydride and sodium triacetoxyborohydride. The reaction is conveniently performed in a suitable inert solvent or diluent, for example tetrahydrofuran and diethyl ether for the more powerful reducing agents such as lithium aluminium hydride, and, for example, methylene chloride or a protic solvent such as methanol and ethanol for the less powerful reducing agents such as sodium triacetoxyborohydride and sodium cyanoborohydride. When the reducing agent is formic acid the reaction is conveniently carried out using an aqueous solution of the formic acid. The reaction is performed at a temperature in the range, for example, 10 to 100°C, such as 70 to 90°C or, conveniently, at or near ambient temperature. Conveniently, when the reducing agent is formic acid, protecting groups such as tert-butoxycarbonyl on the NH group to be alkylated (for example present from the synthesis of the starting material) may be removed in-situ during the reaction.
**Process (i)** For the preparation of those compounds of the Formula I wherein R¹ is substituted by a group T, wherein T is selected from (1-6C)alkylamino, di-[(1-6C)alkyllamino, (2-6C)alkanoylamino, (1-6C)alkylthio, (1-6C)alkylsulfinyl and (1-6C)alkylsulfonyl, the reaction of a compound of the Formula VII: 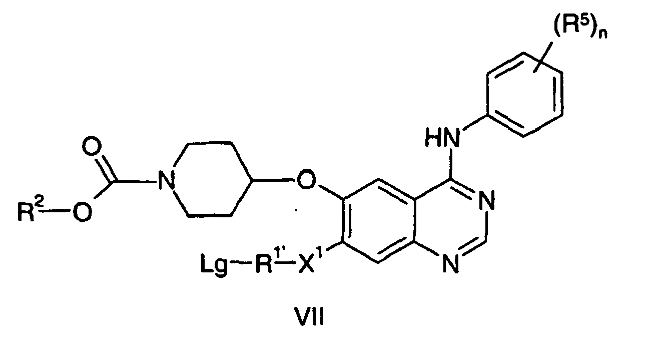 wherein R², R⁵, X¹, n and m have any of the meanings defined hereinbefore except that any functional group is protected if necessary, R^{1'} is a group R¹ as defined herein except that any T groups are replaced with Lg, and Lg is a displaceable group (for example chloro or bromo or aryl/(1-6C)alkyl sulfonates such as mesylate) with a compound of the formula TH, wherein T is as defined above except that any functional group is protected if necessary;
   and whereafter any protecting group that is present is removed by conventional means.
   The reaction is conveniently carried out in the presence of a suitable base. The reaction may conveniently be performed in a suitable inert solvent or diluent. Suitable bases, solvents and diluents are for example those described under process (a). The reaction is suitably performed at a temperature of for example, from 10 to 150°C, for example 30 to 60°C.
   It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group.
**Process (j)** By reacting a compound of the Formula VIII: 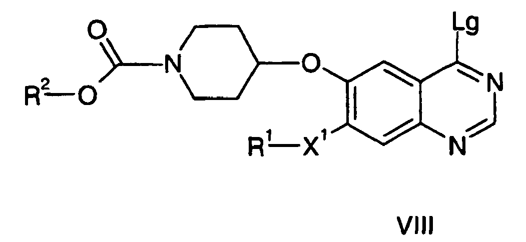 wherein R¹, R², X¹, and m have any of the meanings defined hereinbefore except that any functional group is protected if necessary and Lg is a displaceable group as hereinbefore defined, with an aniline of the Formula IX: 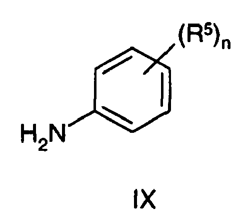 wherein R⁵ and n have any of the meanings defined hereinbefore except that any functional group is protected if necessary, and wherein the reaction is conveniently performed in the presence of a suitable acid,
   and whereafter any protecting group that is present is removed by conventional means.
   Suitable displaceable groups represented by Lg are as hereinbefore defined, in particular halogeno such as chloro.
   The reaction is conveniently carried out in the presence of a suitable inert solvent or diluent, for example an alcohol or ester such as methanol, ethanol, isopropanol or ethyl acetate, a halogenated solvent such as methylene chloride, chloroform or carbon tetrachloride, an ether such as tetrahydrofuran or 1,4-dioxane, an aromatic solvent such as toluene, or a dipolar aprotic solvent such as N,N-dimethylformamide, N,N-dimethylacetamide, N-methylpyrrolidin-2-one acetonitrile or dimethylsulfoxide. The reaction is conveniently carried out at a temperature in the range, for example, 10 to 250°C, conveniently in the range 40 to 120°C or where a solvent or diluent is used at the reflux temperature. Conveniently, the compound of Formula VIII may be reacted with a compound of the Formula IX in the presence of a protic solvent such as isopropanol, conveniently in the presence of an acid, for example a catalytic amount of an acid, under the conditions described above. Suitable acids include hydrogen chloride gas in diethyl ether or dioxane, and hydrochloric acid, for example a 4M solution of hydrogen chloride in dioxane. Alternatively, this reaction may be conveniently carried out in an aprotic solvent, such as dioxane or a dipolar aprotic solvent such as N,N-dimethylacetamide or acetonitrile in the presence of an acid, for example hydrogen chloride gas in diethyl ether or dioxane, or hydrochloric acid.
   The compound of the Formula VIII, wherein Lg is halogeno, may be reacted with a compound of the Formula IX in the absence of an acid. In this reaction displacement of the halogeno leaving group Lg results in the formation of the acid HLg in-situ and auto-catalysis of the reaction. Conveniently the reaction is carried out in a suitable inert organic solvent, for example isopropanol, dioxane or N,N-dimethylacetamide. Suitable conditions for this reaction are as described above.
   Alternatively, the compound of Formula VIII may be reacted with a compound of the Formula IX in the presence of a suitable base. Suitable bases for this reaction are as hereinbefore defined under process (a). For example, suitable bases are alkaline earth metal amides, such as sodium bis(trimethylsilyl)amide. This reaction is conveniently performed in an inert solvent or diluent, for example those mentioned above in relation to this process (j); **Process (k)** By reacting a compound of Formula X: 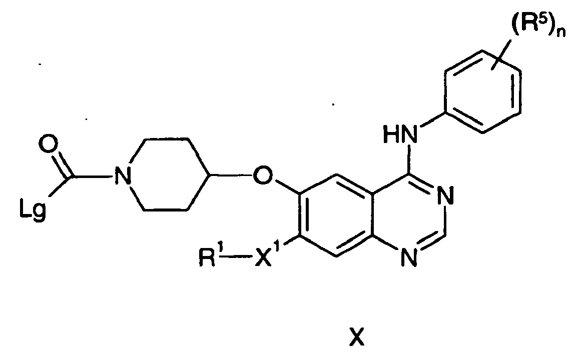 where R⁵, X¹, R¹ and n are as defined above, and where Lg is a leaving group, such as halogeno, especially chloro, or 1-imidazolyl, with an alcohol of formula R²-OH, where R² is as defined above. The reaction is suitably affected in aprotic solvent such as DCM in the presence of a base such as tertiary amine/pyridine. Suitable temperatures will be apparent to a skilled chemist.
**Process (l)** for compounds where R² includes a group of sub-formula (i), reacting a compound of the Formula XI: 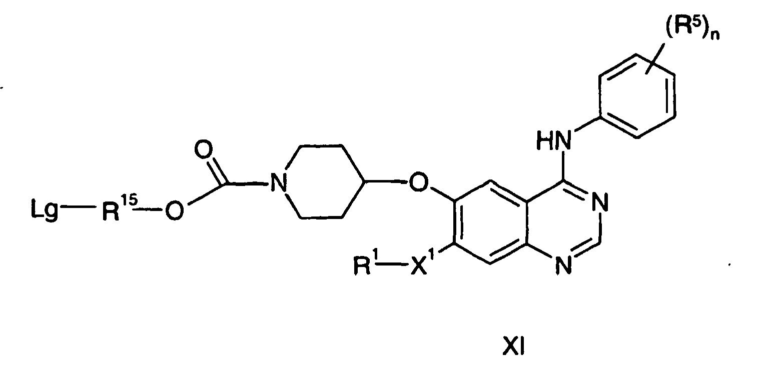 where R¹, X¹, R⁵, and n are as defined hereinbefore, R¹⁵ is a (1-6C)alkylene group, and Lg is a leaving group, with a compound of formula R³R⁴NH where R³ and R⁴ are as defined in relation to sub-formula (i) above. Suitable leaving groups Lg in this case include halogeno such as chloro, or an alkyl/aryl sulfonate such as mesylate). The reaction is suitably effected in the presence of an iodide source such as potassium iodide or tetrabutyl ammonium iodide in an organic solvent such a dimethylacetamide, N-methyl pyrrolidone or dimethylformamide. Suitably, an excess of the amine R³R⁴NH is used. This may be useful, for example when Lg is chloro, to quench the hydrogen chloride that is formed during the course of the reaction. Elevated temperatures, for example of from 50-120°C, for example at about 80°C, are suitably employed.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

When a pharmaceutically-acceptable salt of a quinazoline derivative of the Formula I is required, for example an acid-addition salt, it may be obtained by, for example, reaction of said quinazoline derivative with a suitable acid using a conventional procedure. To facilitate isolation of the compound during preparation, the compound may be prepared in the form of a salt that is not a pharmaceutically acceptable salt. The resulting salt can then be modified by conventional techniques to give a pharmaceutically acceptable salt of the compound. Such techniques include, for example ion exchange techniques or re-precipitation of the compound in the presence of a pharmaceutically acceptable counter ion. For example re-precipitation in the presence of a suitable acid such as HCl to give a hydrochloride acid addition salt.

In the section above the expression "inert solvent" refers to a solvent which does not react with the starting materials, reagents, intermediates or products in a manner which adversely affects the yield of the desired product.

### Preparation of Starting Materials

Compounds of Formula II are commercially available or may be prepared using conventional techniques or analogous processes to those described in the prior art. In particular those patents and applications listed hereinbefore, such as WO96/15118, WO 01/66099 and EP 566 226. For example, the compounds of Formula II may be prepared in accordance with Reaction Scheme 1: 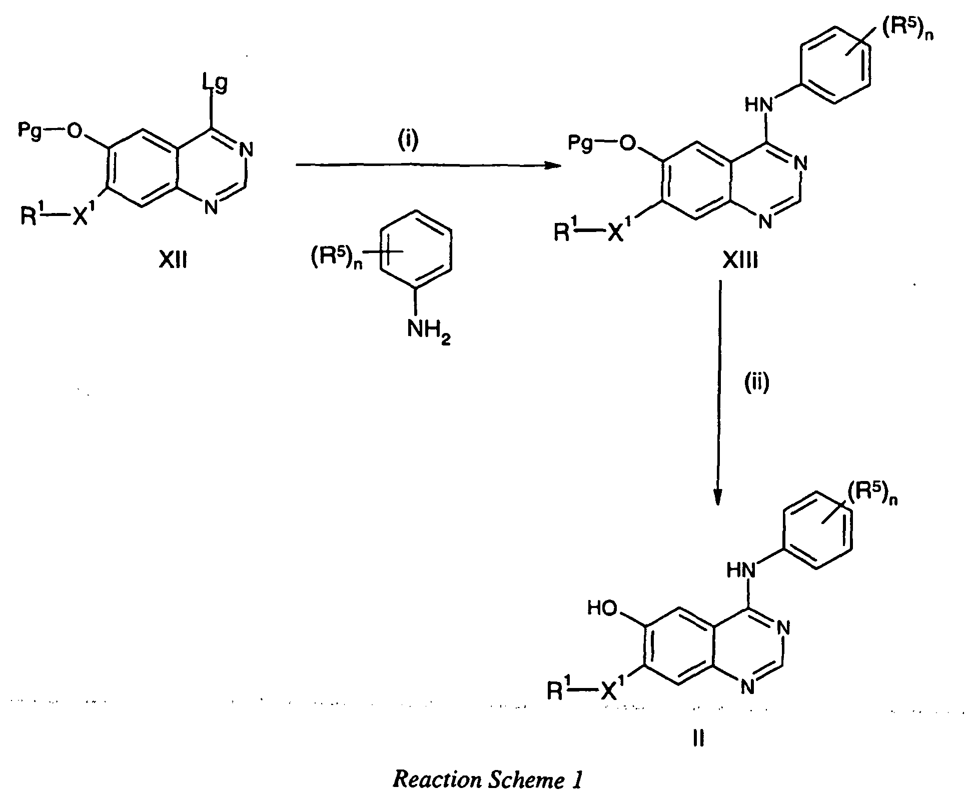 wherein R¹, X¹, R⁵, Lg and n are as hereinbefore defined and Pg is a hydroxy protecting group.
(i) Reaction suitably in an inert protic solvent (such as an alkanol for example iso-propanol), an aprotic solvent (such as dioxane) or a dipolar aprotic solvent (such as N,N-dimethylacetamide) in the presence of an acid, for example hydrogen chloride gas in diethyl ether or dioxane, or hydrochloric acid, under analogous conditions to those described above under process (i).
   Alternatively the reaction may be carried out in one of the above inert solvents conveniently in the presence of a base, for example potassium carbonate. The above reactions are conveniently carried out at a temperature in the range, for example, 0 to 150°C, suitably at or near the reflux temperature of the reaction solvent.
(ii) Cleavage of Pg may be performed under standard conditions for such reactions. For example when Pg is an alkanoyl group such as acetyl, it may be cleaved by heating in the presence of a methanolic ammonia solution.

Compounds of Formula XII are known or can be prepared using known processes for the preparation of analogous compounds. If not commercially available, compounds of the Formula XII may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl. By way of example the compound of the Formula XII in which R¹-X¹- is methoxy, Lg is chloro and Pg is acetyl may be prepared using the process illustrated in Reaction Scheme 2:

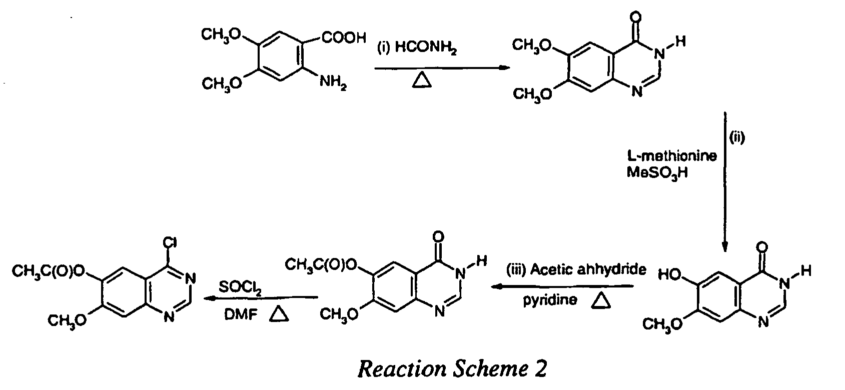

Reaction Scheme 2 may be generalised by the skilled man to apply to compounds within the present specification which are not specifically illustrated (for example to introduce a substituent other than methoxy at the 7-position in the quinazoline ring).

Compounds of the Formula III are commercially available or may be prepared using standard techniques, for example as illustrated in US 5,252,586 and WO 94/27965.

Compounds of the Formula IV may be prepared by reaction of a compound of Formula II with a compound of XVa or XVb: 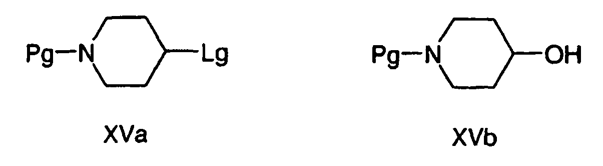 wherein Lg is a displaceable group as hereinbefore defined and Pg is a suitable protecting group.

The reaction of the compound of Formula II with the compound of Formula XVa may be carried out using analogous conditions to those described in process (a) above, followed by removal of the protecting group under standard conditions.

The reaction of the compound of Formula II with the compound of Formula XVb may be carried out under Mitsunobu conditions as described in process (e) above, followed by removal of the protecting group under standard conditions.

Compounds of the Formula IV may also be prepared by reaction of a compound of the Formula IX with a compound of the Formula XVc: 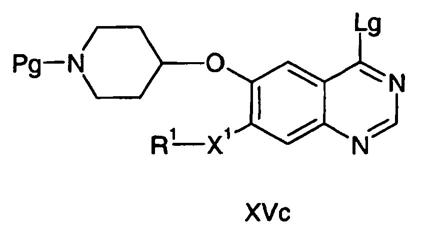 wherein Lg, X¹ and R¹ are as hereinbefore defined and Pg is a suitable protecting group.

The reaction of the compound of Formula IX with the compound of Formula XVc may be carried out using analogous conditions to those described in process (j) above, followed by removal of the protecting group under standard conditions.

Compounds of the Formula V and IX are commercially available or may be prepared using standard techniques.

Compounds of the Formula VI may be prepared using process (e) above, starting with a compound prepared, for example using process (a).

Compounds of the Formula VII may be prepared using, for example process (a) or process (d) or process (e) in which the group represented by R¹ is appropriately functionalised with a suitable displaceable group Lg such as chloro or bromo.

Compounds of the Formula VIII may be prepared using conventional methods well known in the art. For example the hydroxy protecting group, Pg, in a compound of the Formula XII as hereinbefore described in Reaction Scheme 1 is removed to give the compound of the Formula XIV: 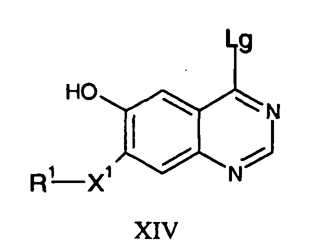 The protecting group Pg may be removed from the compound of Formula XII using conventional techniques.

The compound of the Formula XIV may then be coupled with a compound of the Formula III as hereinbefore defined using analogous conditions to those described in process (a) or process (e).

Compounds of the Formula X may be prepared using conventional methods well known in the art. For example, by reaction of a compound of the Formula IV with a compound of the formula Lg-CO-Lg, where Lg is a displaceable group, such as halogeno (for example, chloro or bromo) or imidazolyl. The reaction of the compound of Formula IV with the compound of the formula Lg-CO-Lg may be carried out using similar conditions to those described in process (c) above, in the presence of a weak base, such as pyridine or lutidine, and in the presence of an inert solvent (for example acetonitrile or methylene chloride). Examples of suitable compounds of the formula Lg-CO-Lg are phosgene and 1,1'-carbonyldiimidazole.

Certain novel intermediates utilised in the above processes are provided as a further feature of the present invention together with the process for their preparation.

According to a further feature of the present invention there is provided the compounds of the formulae VI, VII, X and XI or a salt thereof, (including pharmaceutically acceptable salts thereof), as hereinbefore defined.

### Biological Assays

The inhibitory activities of compounds were assessed in non-cell based protein tyrosine kinase assays as well as in cell based proliferation assays before their *in vivo* activity was assessed in Xenograft studies.

### a) Protein Tyrosine Kinase phosphorylation Assays

This test measures the ability of a test compound to inhibit the phosphorylation of a tyrosine containing polypeptide substrate by EGFR and erbB2 tyrosine kinase enzyme.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM N-2-hydroxyethylpiperizine-N'-2-ethanesulfonic acid (HEPES) pH7.5, 150mM NaCl, 10% glycerol, 1% Triton X-100, 1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of the recombinant protein was determined by its ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 100µl phosphate buffered saline (PBS) solution and incubated at 4°C overnight). Plates were washed in PBS-T (phosphate buffered saline with 0.5% Tween 20) then in 50mM HEPES pH 7.4 at room temperature to remove any excess unbound synthetic peptide. EGFR, ErbB2 or ErbB4 tyrosine kinase activity was assessed by incubation in peptide coated plates for 20 minutes at 22°C in 100mM HEPES pH 7.4, adenosine trisphosphate (ATP) at Km concentration for the respective enzyme, 10mM MnCl₂, 0.1mM Na₃VO₄, 0.2mM DL-dithiothreitol (DTT), 0.1% Triton X-100 with test compound in DMSO (final concentration of 2.5%). Reactions were terminated by the removal of the liquid components of the assay followed by washing of the plates with PBS-T.

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulfonate (6)] diammonium salt crystals (ABTS^{™} from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) EGFR driven KB cell proliferation assay

This assay measures the ability of a test compound to inhibit the proliferation of KB cells (human naso-pharangeal carcinoma obtained from the American Type Culture Collection (ATCC).

KB cells (human naso-pharangeal carcinoma obtained from the ATCC were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulfoxide (DMSO) (0.1% final) before incubation for 4 days. Following the incubation period, cell numbers were determined by addition of 50µl of 3-(4,5-Dimethylthiazol-2-yl)-2,5-diphenyltetrazolium bromide (MTT) (stock 5mg/ml) for 2 hours. MTT solution was then tipped off, the plate gently tapped dry and the cells dissolved upon the addition of 100µl of DMSO.

Absorbance of the solubilised cells was read at 540nm using a Molecular Devices ThermoMax microplate reader. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) In vivo Xenograft assays

### (i) LOVO

This assay measures the ability of a test compound to inhibit the growth of a LoVo tumour (colorectal adenocarcinoma obtained from the ATCC) in Female Swiss athymic mice (Alderley Park, *nu*/*nu* genotype).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. LoVo tumour cell (colorectal adenocarcinoma obtained from the ATCC) xenografts were established in the hind flank of donor mice by sub cutaneous injections of 1x10⁷ freshly cultured cells in 100µl of serum free media per animal. On day 5 post-implant, mice were randomised into groups of 7 prior to the treatment with compound or vehicle control that was administered once daily at 0.1ml/10g body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of study was calculated by comparison of the mean changes in tumour volume for the control and treated groups, and statistical significance between the two groups was evaluated using a Students t test.

### (ii) In vivo BT-474 Xenograft assay

This assay measures the ability of a test compound to inhibit the growth of a BT-474 tumour cell xenograft (human mammary carcinoma obtained from Dr Baselga, Laboratorio Recerca Oncologica, Paseo Vall D'Hebron 119-129, Barcelona 08035, Spain) in Female Swiss athymic mice (Alderley Park, *nu*/*nu* genotype) (Baselga, J. *et al*. (1998) *Cancer Research,* **58**, 2825-2831).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. BT-474 tumour cell xenografts were established in the hind flank of donor mice by sub-cutaneous injections of 1x10⁷ freshly cultured cells in 100µl of serum free media with 50% Matrigel per animal. On day 14 post-implant, mice were randomised into groups of 10 prior to the treatment with compound or vehicle control that was administered once daily at 0.1ml/kg body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students t test.

### d) hERG-encoded Potassium Channel Inhibition Assay

This assay determines the ability of a test compound to inhibit the tail current flowing through the human ether-a-go-go-related-gene (hERG)-encoded potassium channel.

Human embryonic kidney (HEK) cells expressing the hERG-encoded channel were grown in Minimum Essential Medium Eagle (EMEM; Sigma-Aldrich catalogue number M2279), supplemented with 10% Foetal Calf Serum (Labtech International; product number 4-101-500), 10% M1 serum-free supplement (Egg Technologies; product number 70916) and 0.4 mg/ml Geneticin G418 (Sigma-Aldrich; catalogue number G7034). One or two days before each experiment, the cells were detached from the tissue culture flasks with Accutase (TCS Biologicals) using standard tissue culture methods. They were then put onto glass coverslips resting in wells of a 12 well plate and covered with 2 ml of the growing media.

For each cell recorded, a glass coverslip containing the cells was placed at the bottom of a Perspex chamber containing bath solution (see below) at room temperature (-20 °C). This chamber was fixed to the stage of an inverted, phase-contrast microscope. Immediately after placing the coverslip in the chamber, bath solution was perfused into the chamber from a gravity-fed reservoir for 2 minutes at a rate of - 2 ml/min. After this time, perfusion was stopped.

A patch pipette made from borosilicate glass tubing (GC120F, Harvard Apparatus) using a P-97 micropipette puller (Sutter Instrument Co.) was filled with pipette solution (see hereinafter). The pipette was connected to the headstage of the patch clamp amplifier (Axopatch 200B, Axon Instruments) via a silver/silver chloride wire. The headstage ground was connected to the earth electrode. This consisted of a silver/silver chloride wire embedded in 3% agar made up with 0.85% sodium chloride.

The cell was recorded in the whole cell configuration of the patch clamp technique. Following "break-in", which was done at a holding potential of -80 mV (set by the amplifier), and appropriate adjustment of series resistance and capacitance controls, electrophysiology software (*Clampex,* Axon Instruments) was used to set a holding potential (-80 mV) and to deliver a voltage protocol. This protocol was applied every 15 seconds and consisted of a 1 s step to +40 mV followed by a 1 s step to -50 mV. The current response to each imposed voltage protocol was low pass filtered by the amplifier at 1 kHz. The filtered signal was then acquired, on line, by digitising this analogue signal from the amplifier with an analogue to digital converter. The digitised signal was then captured on a computer running *Clampex* software (Axon Instruments). During the holding potential and the step to + 40 mV the current was sampled at 1 kHz. The sampling rate was then set to 5 kHz for the remainder of the voltage protocol.

The compositions, pH and osmolarity of the bath and pipette solution are tabulated below.

| **Salt** | **Pipette (mM)** | **Bath (mM)** |
|---|---|---|
| NaCl | - | 137 |
| KCl | 130 | 4 |
| MgCl₂ | 1 | 1 |
| CaCl₂ | - | 1.8 |
| HEPES | 10 | 10 |
| glucose | - | 10 |
| Na₂ATP | 5 | - |
| EGTA | 5 | - |

| **Parameter** | **Pipette** | **Bath** |
|---|---|---|
| pH | 7.18-7.22 | 7.40 |
| pH adjustment with | 1M KOH | 1M NaOH |
| Osmolarity (mOsm) | 275-285 | 285-295 |

The amplitude of the hERG-encoded potassium channel tail current following the step from +40 mV to -50 mV was recorded on-line by *Clampex* software (Axon Instruments). Following stabilisation of the tail current amplitude, bath solution containing the vehicle for the test substance was applied to the cell. Providing the vehicle application had no significant effect on tail current amplitude, a cumulative concentration effect curve to the compound was then constructed.

The effect of each concentration of test compound was quantified by expressing the tail current amplitude in the presence of a given concentration of test compound as a percentage of that in the presence of vehicle.

Test compound potency (IC₅₀) was determined by fitting the percentage inhibition values making up the concentration-effect to a four parameter Hill equation using a standard data-fitting package. If the level of inhibition seen at the highest test concentration did not exceed 50%, no potency value was produced and a percentage inhibition value at that concentration was quoted.

### e) Clone 24 phospho-erbB2 cell assay

This immunofluorescence end point assay measures the ability of a test compound to inhibit the phosphorylation of erbB2 in a MCF7 (breast carcinoma) derived cell line which was generated by transfecting MCF7 cells with the full length erbB2 gene using standard methods to give a cell line that overexpresses full length wild type erbB2 protein (hereinafter 'Clone 24' cells).

Clone 24 cells were cultured in Growth Medium (phenol red free Dulbecco's modified Eagle's medium (DMEM) containing 10% foetal bovine serum, 2 mM glutamine and 1.2mg/ml G418) in a 7.5% CO₂ air incubator at 37°C. Cells were harvested from T75 stock flasks by washing once in PBS (phosphate buffered saline, pH7.4, Gibco No. 10010-015) and harvested using 2mls of Trypsin (1.25mg/ml) / ethylaminediaminetetraacetic acid (EDTA) (0.8mg/ml) solution. The cells were resuspended in Growth Medium. Cell density was measured using a haemocytometer and viability was calculated using Trypan Blue solution before being further diluted in Growth Medium and seeded at a density of 1x10⁴ cells per well (in 100ul) into clear bottomed 96 well plates (Packard, No. 6005182).

3 days later, Growth Medium was removed from the wells and replaced with 100ul Assay Medium (phenol red free DMEM, 2mM glutamine, 1.2mg/ml G418) either with or without erbB inhibitor compound. Plates were returned to the incubator for 4hrs and then 20µl of 20% formaldehyde solution in PBS was added to each well and the plate was left at room temperature for 30 minutes. This fixative solution was removed with a multichannel pipette, 100µl of PBS was added to each well and then removed with a multichannel pipette and then 50µl PBS was added to each well. Plates were then sealed and stored for up to 2 weeks at 4°C.

Immunostaining was performed at room temperature. Wells were washed once with 200µl PBS / Tween 20 (made by adding 1 sachet of PBS / Tween dry powder (Sigma, No. P3563) to 1L of double distilled H₂O) using a plate washer then 200µl Blocking Solution (5% Marvel dried skimmed milk (Nestle) in PBS /Tween 20) was added and incubated for 10 minutes. Blocking Solution was removed using a plate washer and 200µl of 0.5% Triton X-100 / PBS was added to permeabalise the cells. After 10 minutes, the plate was washed with 200µl PBS / Tween 20 and then 200µl Blocking Solution was added once again and incubated for 15 minutes. Following removal of the Blocking Solution with a plate washer, 30µl of rabbit polyclonal anti-phospho ErbB2 IgG antibody (epitope phospho-Tyr 1248, SantaCruz, No. SC-12352-R), diluted 1:250 in Blocking Solution, was added to each well and incubated for 2 hours. Then this primary antibody solution was removed from the wells using a plate washer followed by two 200µl PBS / Tween 20 washes using a plate washer. Then 30µl of Alexa-Fluor 488 goat anti-rabbit IgG secondary antibody (Molecular Probes, No. A-11008), diluted 1:750 in Blocking Solution, was added to each well. From now onwards, wherever possible, plates were protected from light exposure, at this stage by sealing with black backing tape. The plates were incubated for 45 minutes and then the secondary antibody solution was removed from the wells followed by two 200ul PBS / Tween 20 washes using a plate washer. Then 100µl PBS was added to each plate, incubated for 10 minutes and then removed using a plate washer. Then a further 100µl PBS was added to each plate and then, without prolonged incubation, removed using a plate washer. Then 50µl of PBS was added to each well and plates were resealed with black backing tape and stored for up to 2 days at 4°C before analysis.

The Fluorescence signal is each well was measured using an Acumen Explorer Instrument (Acumen Bioscience Ltd.), a plate reader that can be used to rapidly quantitate features of images generated by laser-scanning. The instrument was set to measure the number of fluorescent objects above a pre-set threshold value and this provided a measure of the phosphorylation status of erbB2 protein. Fluorescence dose response data obtained with each compound was exported into a suitable software package (such as Origin) to perform curve fitting analysis. Inhibition of erbB2 phosphorylation was expressed as an IC₅₀ value. This was determined by calculation of the concentration of compound that was required to give 50% inhibition of erbB2 phosphorylation signal.

Although the pharmacological properties of the compounds of the Formula I vary with structural change as expected, in general activity possessed by compounds of the Formula I, may be demonstrated at the following concentrations or doses in one or more of the above tests:-

| | |
|---|---|
| Test (a):- | IC₅₀ in the range, for example, 0.001 - 10 µM; |
| Test (b):- | IC₅₀ in the range, for example, 0.001 - 10 µM; |
| Test (e):- | IC₅₀ in the range, for example, 0.001 - 10 µM; |
| Test (c):- | activity in the range, for example, 1-200 mg/kg/day; |

By way of example, Table A illustrates the activity of representative compounds according to the invention. Column 2 of Table A shows IC₅₀ data from Test (a) for the inhibition of EGFR tyrosine kinase protein phosphorylation; column 3 shows IC₅₀ data from Test (a) for the inhibition of erbB2 tyrosine kinase protein phosphorylation; column 4 shows IC₅₀ data for inhibition of proliferation of KB cells in Test (b) described above; and column 5 shows IC₅₀ data for inhibition of phosphorylation of erbB2 in a MCF7 derived cell line in Test (e) described above:

**Table A**

| **Example Number** | **IC₅₀ (µM) Test (a): Inhibition of EGFR tyrosine kinase protein phosphorylation** | **IC₅₀ (µM) Test (a): Inhibition of erbB2 tyrosine kinase protein phosphorylation** | **IC₅₀ (µM) Test (b): EGFR driven KB cell proliferation assay** | **IC₅₀ (µM) Test (e): Inhibition of erbB2 tyrosine kinase protein phosphorylation** |
|---|---|---|---|---|
| 1 | 0.001 | 0.038 | 0.035 | 0.009 |
| 2 | 0.004 | 0.010 | 0.009 | 0.036 |
| 4 | 0.002 | 0.002 | 0.015 | 0.029 |
| 6 | 0.002 | 0.002 | 0.010 | 0.041 |

According to a further aspect of the invention there is provided a pharmaceutical composition which comprises a quinazoline derivative of the Formula I, or a pharmaceutically-acceptable thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

We have found that the compounds of the present invention possess anti-proliferative properties such as anti-cancer properties that are believed to arise from their erbB family receptor tyrosine kinase inhibitory activity, and particularly a mixed erbB2/ EGF profile.

Accordingly, the compounds of the present invention are expected to be useful in the treatment of diseases or medical conditions mediated alone or in part by erbB receptor tyrosine kinases, i.e. the compounds may be used to produce an erbB receptor tyrosine kinase inhibitory effect in a warm-blooded animal in need of such treatment. Thus the compounds of the present invention provide a method for the treatment of malignant cells characterised by inhibition of one or more of the erbB family of receptor tyrosine kinases. Particularly the compounds of the invention may be used to produce an anti-proliferative and/or pro-apoptotic and/or anti-invasive effect mediated alone or in part by the inhibition of erbB receptor tyrosine kinases. Particularly, the compounds of the present invention are expected to be useful in the prevention or treatment of those tumours that are sensitive to inhibition of one or more of the erbB receptor tyrosine kinases, that are involved in the signal transduction steps which drive proliferation and survival of these tumour cells. Accordingly the compounds of the present invention are expected to be useful in the treatment of psoriasis, benign prostatic hyperplasia (BPH), atherosclerosis and restenosis and/or cancer by providing an anti-proliferative effect, particularly in the treatment of erbB receptor tyrosine kinase sensitive cancers. Such benign or malignant tumours may affect any tissue and include non-solid tumours such as leukaemia, multiple myeloma or lymphoma, and also solid tumours, for example bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancers.

According to this aspect of the invention there is provided a compound of the Formula I, or a pharmaceutically acceptable salt thereof, for use as a medicament.

According to a further aspect of the invention there is provided a compound of the Formula I, or a pharmaceutically acceptable salt thereof, for use in the production of an anti-proliferative effect in a warm-blooded animal such as man.

Thus according to this aspect of the invention there is provided the use of a quinazoline derivative of the Formula I, or a pharmaceutically-acceptable salt thereof, as defined hereinbefore in the manufacture of a medicament for use in the production of an anti-proliferative effect in a warm-blooded animal such as man.

According to a further feature of this aspect of the invention there is provided a method for producing an anti-proliferative effect in a warm-blooded animal, such as man, in need of such treatment which comprises administering to said animal an effective amount of a quinazoline derivative of the Formula I, or a pharmaceutically acceptable salt thereof, as hereinbefore defined.

According to a further aspect of the invention there is provided the use of a quinazoline derivative of the Formula I, or a pharmaceutically-acceptable salt thereof, as defined hereinbefore in the manufacture of a medicament for use in the prevention or treatment of those tumours which are sensitive to inhibition of erbB receptor tyrosine kinases, such as a combination of EGFR and erbB2, that are involved in the signal transduction steps which lead to the proliferation of tumour cells.

According to a further feature of this aspect of the invention there is provided a method for the prevention or treatment of those tumours which are sensitive to inhibition of one or more of the erbB family of receptor tyrosine kinases, such as a combination of EGFR and erbB2, that are involved in the signal transduction steps which lead to the proliferation and/or survival of tumour cells which comprises administering to said animal an effective amount of a quinazoline derivative of the Formula I, or a pharmaceutically-acceptable salt thereof, as defined hereinbefore.

According to a further feature of this aspect of the invention there is provided a compound of the Formula I, or a pharmaceutically acceptable salt thereof, for use in the prevention or treatment of those tumours which are sensitive to inhibition of erbB receptor tyrosine kinases, such as a combination of EGFR and erbB2, that are involved in the signal transduction steps which lead to the proliferation of tumour cells.

According to a further aspect of the invention there is provided the use of a quinazoline derivative of the Formula I, or a pharmaceutically-acceptable salt thereof, as defined hereinbefore in the manufacture of a medicament for use in providing a combined EGFR and erbB2 tyrosine kinase inhibitory effect.

According to a further feature of this aspect of the invention there is provided a method for providing a combined EGFR and erbB2 tyrosine kinase inhibitory effect which comprises administering to said animal an effective amount of a quinazoline derivative of the Formula I, or a pharmaceutically-acceptable salt thereof, as defined hereinbefore.

According to a further feature of this aspect of the invention there is provided a compound of the Formula I, or a pharmaceutically acceptable salt thereof, for use in providing a combined EGFR and erbB2 tyrosine kinase inhibitory effect.

According to a further aspect of the present invention there is provided the use of a quinazoline derivative of the Formula I, or a pharmaceutically-acceptable salt thereof, as defined hereinbefore in the manufacture of a medicament for use in the treatment of a cancer (for example a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer).

According to a further feature of this aspect of the invention there is provided a method for treating a cancer (for example a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer) in a warm-blooded animal, such as man, in need of such treatment, which comprises administering to said animal an effective amount of a quinazoline derivative of the Formula I, or a pharmaceutically-acceptable salt thereof, as defined hereinbefore.

According to a further aspect of the invention there is provided a compound of the Formula I, or a pharmaceutically acceptable salt thereof, for use in the treatment of a cancer (for example selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer).

As mentioned above the size of the dose required for the therapeutic or prophlyactic treatment of a particular disease will necessarily be varied depending upon, amongst other things, the host treated, the route of administration and the severity of the illness being treated.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, tyrosine kinase inhibitors and serine/threonine kinase inhibitors, for example other inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a quinazoline derivative of the Formula I as defined hereinbefore and an additional anti-tumour agent as defined hereinbefore for the conjoint treatment of cancer.

Although the compounds of the Formula I are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests and in the search for new pharmacological agents.

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulf ate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LCMS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 400 MHz using perdeuterio dimethyl sulfoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra (MS) were run with an electron energy of 70 electron volts in the chemical ionization (CI) mode using a direct exposure probe and ionization was effected by electrospray; values for m/z are given; generally, only ions which indicate the parent mass are reported; and unless otherwise stated, the mass ion quoted is (MH)⁺;
(xi) unless stated otherwise compounds containing an asymmetrically substituted carbon and/or sulfur atom have not been resolved;
(xii) where a synthesis is described as being analogous to that described in a previous example the amounts used are the millimolar ratio equivalents to those used in the previous example;
(xiii) the following abbreviations have been used:
   - DCM: dichloromethane;
   - DMF: *N,N*-dimethylformamide;
   - DMA: *N,N*-dimethylacetamide;
   - THF: Tetrahydrofuran;
(xiv) where a synthesis is described as leading to an acid addition salt (e.g. HCl salt), the specific stoichiometry of the salt was not confirmed.
(xv) In Examples 1 -2 and the Reference Examples unless otherwise stated, all NMR data is reported on free-base material, with isolated salts converted to the free-base form prior to characterisation.

### Example 1

### 4-(3-Chloro-2-fluoroanilino)-7-methoxy-6-{[1-(methoxycarbonyl)piperidin-4-yl]oxy} quinazoline

Methylchloroformate (23 µl, 0.3 mmol) was added dropwise to a ice-cooled mixture of 4-(3-chloro-2-fluoroanilino)-7-methoxy-6-[(piperidin-4-yl)oxy]quinazoline (120 mg, 0.3 mmol) and diisopropylethylamine (63 µl, 0.36 mmol) in dichloromethane (5 ml). The mixture was stirred at 0°C for one hour. The organic solution was washed with water and brine, dried over magnesium sulfate. After evaporation of the solvents under vacuum, the residue was purified by chromatography on silica gel (eluant: 2% 7N methanolic ammonia in dichloromethane) to give the title compound as a white solid (80 mg, 58%). ¹H NMR Spectrum: (CDCl₃) 1.90 (m, 2H), 2.00 (m, 2H), 3.44 (m, 2H), 3.72 (s, 3H), 3.84 (m, 2H), 4.01 (s, 3H), 4.64 (m, 1H), 7.17 (m, 3H), 7.30 (m, 2H), 8.48 (m, 1H), 8.71 (s, 1H); Mass spectrum: MH⁺ 461.

4-(3-Chloro-2-fluoroanilino)-7-methoxy-6-[(piperidin-4-yl)oxy]quinazoline used as the starting material was prepared as follows:

### Step 1

### 6-Acetoxy-4-(3-chloro-2-fluoroanilino)-7-methoxyquinazoline hydrochloride

6-Acetoxy-4-chloro-7-methoxyquinazoline (prepared as described in Example 25-5 of in WO01/66099, 6.00 g, 23.8 mmol) and 3-chloro-2-fluoroaniline (3.46 g, 23.8 mmol) were suspended in iso-propanol (200 ml). The mixture was heated to 80°C under reflux for 3 hours. The solvent was evaporated; the residue was crystallised from acetonitrile, giving the product hydrochloride as a pale pink crystalline solid (8.16 g, 92%). ¹H NMR: 2.37 (s, 3H), 4.00 (s, 3H), 7.34 (ddd, 1H), 7.48 (s, 1H), 7.52 (ddd, 1H), 7.61 (ddd, 1H), 8.62 (s, 1H), 8.86 (s, 1H); Mass Spectrum: 362.4, 364.4.

### Step 2

### 4-(3-Chloro-2-fluoroanilino)-6-hydroxy-7-methoxyquinazoline

6-Acetoxy-4-(3-chloro-2-fluoroanilino)-7-methoxyquinazoline hydrochloride from step 1 (8.72 g, 21.9 mmol) was dissolved in methanol (200 ml). Concentrated aqueous ammonia (15 ml) was added, and the solution heated to 50°C with stirring for 2 hours, causing precipitation of a cream coloured solid. The solid was collected by filtration, washed with diethyl ether (3x 200 ml), and dried *in vacuo* at 60°C over diphosphorous pentoxide, giving the product as an off white solid (5.40 g, 77%). ¹H NMR: 3.95 (s, 3H), 7.19 (s, 1H), 7.23 (dd, 1H), 7.42 (dd, 1H), 7.50 (dd, 1H), 7.64 (s, 1H), 8.32 (s, 1H), 9.43 (s, 1H), 9.67 (br.s, 1H); Mass Spectrum: 320.4, 322.4.

### Step 3

### 6-{[(1-tert-Butoxycarbonyl)piperidin-4-yl]oxy}-4-(3-chloro-2-fluoroanilino)-7-methoxyquinazoline

4-(3-Chloro-2-fluoroanilino)-6-hydroxy-7-methoxyquinazoline from Step 2 (1870 mg, 5.85 mmol) was dissolved in DMA (50 ml). *tert*-Butyl (4-methanesulfonyloxy)piperidine-1-carboxylate (prepared as in Chemical & Pharmaceutical Bulletin 2001, **49**(7), 822-829; 490 mg, 1.76 mmol) and cesium fluoride (890 mg, 5.85 mmol) were added, and the mixture was heated to 85°C with stirring. At intervals of 2 hours, 4 hours and 6 hours, *tert*-butyl 4-methanesulfonyloxypiperidine-1-carboxylate and cesium fluoride were added in the above quantities to the reaction mixture. Heating was continued at 85°C for a further 6 hours after the final addition. The solvent was evaporated, and the residue was partitioned between DCM (150 ml) and H₂O (150 ml). The aqueous layer was extracted with DCM (4x 100 ml), and the extractions combined with the DCM layer. The combined DCM fractions were dried over MgSO₄ and evaporated. The residue was purified by chromatography, eluting with 0 to 2.5% (7:1 MeOH / concentrated aqueous NH₄OH) in DCM. The appropriate fractions were combined and evaporated, giving the product as a light brown foam (2.40 g, 58%, allowing for 2.3 equivalents of residual DMA). ¹H NMR: 1.40 (s, 9H), 1.60-1.65 (m, 2H), 1.95-2.00 (m, 2H), 3.20-3.25 (m, 2H), 3.65-3.70 (m, 2H), 3.92 (s, 3H), 4.68 (m, 1H). 7.21 (s, 1H), 7.27 (dd, 1H), 7.47 (ddd, 1H), 7.51 (dd, 1H), 7.85 (s, 1H), 8.36 (s, 1H), 9.53 (s, 1H); Mass Spectrum: 503.5, 505.5.

### Step 4

### 4-(3-chloro-2-fluoroanilino)-7-methoxy-6-[(piperidin-4-yl)oxy]quinazoline

6-{[(1-*tert*-Butoxycarbonyl)piperidin-4-yl]oxy}-4-(3-chloro-2-fluoroanilino)-7-methoxyquinazoline from step 3 (350 mg, 0.70 mmol) was dissolved in trifluoroacetic acid (5 ml), and the solution stood for 2 hours. The excess trifluoroacetic acid was evaporated, and the residue was azeotroped twice with DCM. The residue was purified by chromatography, eluting with 0 to 4% (7:1 MeOH / concentrated aqueous NH₄OH) in DCM. Evaporation of the appropriate fractions gave the product as an off-white solid (270 mg, 96%). ¹H NMR: 1.53-1.64 (m, 2H), 2.00-2.05 (m, 2H), 2.64-2.72 (m, 2H), 3.00-3.07 (m, 2H), 3.92 (s, 3H), 4.60 (m, 1H), 7.20 (s, 1H), 7.26 (ddd, 1H), 7.47 (dd, 1H), 7.50 (dd, 1H), 7.82 (s, 1H), 8.34 (s, 1H), 9.56 (s, 1H); Mass Spectrum: 403.2,405.2.

### Example 2

### 4-(3-Chloro-2-fluoroanilino)-7-methoxy-6-{[1-{2-(pyrrolidin-1-yl)ethoxycarbonyl} piperidin-4-yl]oxy}quinazoline

A mixture of 6-{[1-(2-chloroethoxycarbonyl)piperidin-4-yl]oxy}-4-(3-chloro-2-fluoroanilino)-7-methoxyquinazoline (510 mg, 1 mmol), pyrrolidine (0.33 ml, 4 mmol) and potassium iodide (330 mg, 2 mmol) in dimethylacetamide was heated at 80°C for 4 hours. After cooling, the solvents were evaporated under high vacuum. The residue was partitioned between water and dichloromethane, and extracted with dichloromethane.

The organic layer was washed with water and brine and dried over magnesium sulfate. After evaporation of the solvents, the residue was purified by chromatography on silica gel (eluant: 2% to 3% 7N methanolic ammonia in dichloromethane) and further purified on an HPLC column (C18, 5 microns, 19 mm diameter, 100 mm length) of a preparative HPLC-MS system eluting with a mixture of water (containing 5% methanol and 1% acetic acid) and acetonitrile (gradient). After evaporation of the solvents, the residue was dissolved in dichloromethane and aqueous potassium carbonate. The organic layer was dried over magnesium sulfate. Evaporation of the solvents and trituration of the residue in pentane afforded the title compound as a white solid (230 mg, 42%). ¹H NMR Spectrum: (CDCl₃) 1.80 (m, 4H), 1.91 (m, 2H), 2.00 (m, 2H), 2.60 (m, 4H), 2.77 (t, 2H), 3.45 (m, 2H), 3.83 (m, 2H), 4.01 (s, 3H), 4.26 (t, 2H), 4.64 (m, 1H), 7.16 (m, 2H), 7.20 (s, 1H), 7.30 (m, 2H), 8.48 (m, 1H), 8.71 (s, 1H); Mass spectrum: MH⁺ 544.

The 6-{[1-(2-chloroethoxycarbonyl)piperidin-4-yl]oxy}-4-(3-chloro-2-fluoroanilino)-7-methoxyquinazoline used as starting material was made as follows:

2-Chloroethylchloroformate (0.52 ml, 5 mmol) was added dropwise to a ice-cooled mixture of 4-(3-chloro-2-fluoroanilino)-7-methoxy-6-[(piperidin-4-yl)oxy]quinazoline (2 g, 5 mmol) and diisopropylethylamine (1.05 ml, 6 mmol) in dichloromethane (100 ml). The mixture was stirred at 0°C for one hour. The organic solution was washed with water and brine, dried over magnesium sulfate. After evaporation of the solvents under vacuum, the residue was purified by chromatography on silica gel (eluant: 2% 7N methanolic ammonia in dichloromethane) to give the title compound as a white solid (1.6 g, 65%). ¹H NMR Spectrum: (CDCl₃) 1.90 (m, 2H), 2.00 (m, 2H), 3.47 (m, 2H), 3.71 (t, 2H), 3.83 (m, 2H), 4.01 (s, 3H), 4.36 (t, 2H), 4.65 (m, 1H), 7.15 (m, 2H), 7.21 (s, 1H), 7.31 (s, 1H), 7.34 (s br, 1H), 8.46 (m, 1H), 8.71 (s, 1H); Mass spectrum: MH⁺ 509, 511.

### Example 3

### 4-(3-Chloro-2,4-difluoroanilino)-7-methoxy-6-{[1-(methoxycarbonyl)piperidin-4-yl] oxy}quinazoline

Methylchloroformate (40 µl, 0.48 mmol) was added dropwise to a ice-cooled mixture of 4-(3-chloro-2,4-difluoroanilino)-7-methoxy-6-[(piperidin-4-yl)oxy]quinazoline (200 mg, 0.48 mmol) and diisopropylethylamine (170 µl, 0.95 mmol) in dichloromethane (2 ml). The mixture was stirred at 0°C for 90 minutes. After evaporation of the solvents, the residue was dissolved in DMSO and purified on an HPLC column (C18, 5 microns, 19 mm diameter, 100 mm length) of a preparative HPLC-MS system eluting with a mixture of water and acetonitrile containing 2g/l of ammonium formate (gradient). After evaporation of the solvents under vacuum, the residue was repurified by chromatography on silica gel (eluant: 0-3% 7N methanolic ammonia in dichloromethane). After evaporation of the solvents, the residue was triturated in acetonitrile to give the title compound as a white solid (35 mg, 15%). ¹H NMR Spectrum: (DMSOd₆) 1.67 (m, 2H), 2.02 (m, 2H), 3.35 (m, 2H), 3.61 (s, 3H), 3.73 (m, 2H), 3.94 (s, 3H), 4.72 (m, 1H), 7.23 (s, 1h), 7.42 (m, 1H), 7.58 (m, 1H), 7.86 (s, 1H), 8.37 (s, 1H), 9.61 (s br, 1H); Mass Spectrum: MH⁺ 479.

The 4-(3-chloro-2,4-difluoroanilino)-7-methoxy-6-[(piperidin-4-yl)oxy]quinazoline used as starting material was made as follows:
3-Chloro-2,4-difluoroaniline (1.7 g, 10.1 mmol) and 5N hydrogen chloride in isopropanol (2 ml) were added to a suspension of *tert*-butyl 4-[(4-chloro-7-methoxyquinazolin-6-yl)oxy]piperidine-1-carboxylate (4 g, 10.1 mmol, PCT Int. Appl. WO2003082831, AstraZeneca) in isopropanol (50 ml). The mixture was stirred at 80°C for 3 hours. After evaporation of the solvents, the residue was purified by chromatography on silica gel (eluant: 5-10% 7N methanolic ammonia in dichloromethane) to give 4-(3-chloro-2,4-difluoroanilino)-7-methoxy-6-[(piperidin-4-yl)oxy]quinazoline (3.63 g, 85%) as a white solid. ¹H NMR Spectrum: (CDCl₃ + CD3CO2D): 2.15 (m, 2H), 2.30 (m, 2H), 3.34 (m, 2H), 3.47 (m, 2H), 4.01 (s, 3H), 4.91 (m, 1H), 7.03 (m, 1H), 7.58 (m, 2H), 7.90 (s, 1H), 8.55 (s, 1H); Mass spectrum: MH⁺ 421.

### Examples 4 and 5

A mixture of 4-(3-chloro-2,4-difluoroanilino)-6-{[1-(2-chloroethoxycarbonyl)piperidin-4-yl]oxy}-7-methoxyquinazoline (350 mg, 0.66 mmol), the appropriate amine (2.6 mmol) and potassium iodide (220 mg, 1.33 mmol) in dimethylacetamide (5 ml) was heated at 95°C for 2 hours. After cooling, the solvents were evaporated under high vacuum. The residue was diluted in dichloromethane and the solids were filtered off. After evaporation of the filtrate, the residue was purified by chromatography on silica gel (eluant: 2% 7N methanolic ammonia in dichloromethane). Evaporation of the solvents afforded the title compound.

The 4-(3-chloro-2,4-difluoroanilino)-6-{[1-(2-chloroethoxycarbonyl)piperidin-4-yl]oxy}-7-methoxyquinazoline used as starting material was made from 2-chloroethylchloroformate and 4-(3-chloro-2,4-difluoroanilino)-7-methoxy-6-[(piperidin-4-yl)oxy]quinazoline using the same procedure as the one described in Example 2. Yield: 464 mg, 74%. ¹H NMR Spectrum: (DMSOd₆) 1.70 (m, 2H), 2.03 (m, 2H), 3.30 (m, 2H), 3.74 (m, 2H), 3.83 (t, 2H), 3.94 (s, 3H), 4.28 (t, 2H), 4.73 (m, 1H), 7.23 (s, 1H), 7.40 (m, 1H), 7.58 (m, 1H), 7.86 (s, 1H), 8.37 (s, 1H), 9.59 (s, 1H).

### Example 4

### 4-(3-Chloro-2,4-difluoroanilino)-7-methoxy-6-{[1-{2-(pyrrolidin-1-yl) ethoxycarbonyl}piperidin-4-yl]oxy}quinazoline

The amine used was pyrrolidine (0.22 ml, 2.6 mmol).

Yield: 190 mg, 51%. ¹H NMR Spectrum: (CDCl₃) 1.80 (m, 4H), 1.90 (m, 2H), 2.00 (m, 2H), 2.57 (m, 4H), 2.76 (t, 2H), 3.43 (m, 2H), 3.83 (m, 2H), 4.01 (s, 3H), 4.25 (t, 2H), 4.64 (m, 1H), 7.07 (m, 1H), 7.21 (s, 2H), 7.30 (s, 1H), 8.32 (m, 1H), 8.66 (s, 1H); Mass spectrum: MH⁺ 562.

### Example 5

### 4-(3-Chloro-2,4-difluoroanilino)-7-methoxy-6-{[1-{2-(piperidin-1-yl) ethoxycarbonyl}piperidin-4-yl]oxy}quinazoline

The amine used was piperidine.

Yield: 63 mg, 52%. ¹H NMR Spectrum: (CDCl₃) 1.45 (m, 2H), 1.60 (m, 4H), 1.90 (m, 2H), 2.00 (m, 2H), 2.46 (m, 4H), 2.63 (t, 2H), 3.43 (m, 2H), 3.83 (m, 2H), 4.01 (s, 3H), 4.24 (t, 2H), 4.64 (m, 1H), 7.07 (m, 1H), 7.18 (s br, 1H), 7.21 (s, 1H), 7.30 (s, 1H), 8.33 (m, 1H), 8.67 (s, 1H); Mass spectrum: MH⁺ 574.

### Examples 6 to 9

A mixture of 6-{[1-(2-chloroethoxycarbonyl)piperidin-4-yl]oxy}-4-(3-chloro-2-fluoroanilino)-7-methoxyquinazoline (204 mg, 0.4 mmol), potassium iodide (134 mg, 0.8 mmol) and the appropriate amine (1.6 mmol) in dimethylacetamide (4 ml) was heated at 80°C for 4 hours. After cooling, the solvents were evaporated under high vacuum. The residue was partitioned between water and dichloromethane, and extracted with dichloromethane. The organic layer was washed with water and brine and dried over magnesium sulfate. After evaporation of the solvents, the residue was purified by chromatography on silica gel (eluant: 2% to 3% 7N methanolic ammonia in dichloromethane) and triturated in pentane to give the title compound.

### Example 6

### 4-(3-Chloro-2-fluoroanilino)-7-methoxy-6-{[1-{2-(piperidin-1-yl) ethoxycarbonyl}piperidin-4-yl]oxy}quinazoline

The amine used was piperidine.

Yield: 150 mg, 54% (reaction run on 0.5 mmol scale). ¹H NMR Spectrum: (CDCl₃) 1.44 (m, 2H), 1.60 (m, 4H), 1.88 (m, 2H), 2.00 (m, 2H), 2.46 (m, 4H), 2.63 (t, 2H), 3.43 (m, 2H), 3.82 (m, 2H), 4.01 (s, 3H), 4.24 (t, 2H), 4.64 (m, 1H), 7.16 (m, 2H), 7.20 (s, 1H), 7.30 (m, 2H), 8.48 (m, 1H), 8.71 (s, 1H); Mass spectrum: MH⁺ 556.

### Example 7

### 4-(3-Chloro-2-fluoroanilino)- 6-{[1-{2-(diethylamino)ethoxycarbonyl}piperidin-4-yl] oxy}-7-methoxyquinazoline

The amine used was diethylamine.

Yield: 100 mg, 46% (the reaction was run in a sealed tube with a large excess of diethylamine). ¹H NMR Spectrum: (CDCl₃) 1.04 (m, 6H), 1.90 (m, 2H), 1.99 (m, 2H), 2.59 (m, 4H), 2.73 (t, 2H), 3.72 (m, 2H), 3.82 (m, 2H), 4.01 (s, 3H), 4.18 (t, 2H), 4.64 (m, 1H), 7.16 (m, 2H), 7.20 (s, 1H), 7.31 (m, 2H), 8.48 (m, 1H), 8.71 (s, 1H); Mass spectrum: MH⁺ 546.

### Example 8

### 4-(3-Chloro-2-fluoroanilino)-7-methoxy-6-{[1-{2-(morpholin-4-yl)ethoxycarbonyl} piperidin-4-yl]oxy}quinazoline

The amine used was morpholine.

Yield: 140 mg, 62%. ¹H NMR Spectrum: (CDCl₃) 1.91 (m, 2H), 2.00 (m, 2H), 2.53 (m, 4H), 2.65 (t, 2H), 3.44 (m, 2H), 3.72 (m, 4H), 3.83 (m, 2H), 4.01 (s, 3H), 4.25 (t, 2H), 4.64 (m, 1H), 7.16 (m, 2H), 7.20 (s, 1H), 7.31 (m, 2H), 8.48 (m, 1H), 8.71 (s, 1H); Mass spectrum: MH⁺ 560.

### Example 9

### 4-(3-Chloro-2-fluoroanilino)-7-methoxy-6-{[1-{2-(4-methylpiperidin-1-yl) ethoxycarbonyl}piperidin-4-yl]oxy}quinazoline

The amine used was 4-methylpiperidine.

Yield: 110 mg, 50%. ¹H NMR Spectrum: (CDCl₃) 1.89 (m, 2H), 2.00 (m, 2H), 2.28 (s, 3H), 2.6-2.3 (m, 8H), 2.68 (t, 2H), 3.44 (m, 2H), 3.82 (m, 2H), 4.01 (s, 3H), 4.25 (t, 2H), 4.64 (m, 1H), 7.16 (m, 2H), 7.20 (s, 1H), 7.31 (m, 2H), 8.48 (m, 1H), 8.71 (s, 1H); Mass spectrum: MH⁺ 573.

### Example 10

### Pharmaceutical compositions

The following illustrates a representative pharmaceutical dosage forms of the invention as defined herein (the active ingredient being termed "Compound X"), for therapeutic or prophylactic use in humans:

| | | |
|---|---|---|
| (a) | Tablet I | mg/tablet |
| | Compound X | 100 |
| | Lactose Ph.Eur | 182.75 |
| | Croscarmellose sodium | 12.0 |
| | Maize starch paste (5% w/v paste) | 2.25 |
| | Magnesium stearate | 3.0 |
| (b) | Injection I | (50 mg/ml) |
| | Compound X | 5.0% w/v |
| | 1M Sodium hydroxide solution | 15.0% v/v |
| | 0.1M Hydrochloric acid (to adjust pH to 7.6) | |
| | Polyethylene glycol 400 | 4.5% w/v |
| | Water for injection to 100%. | |

The above formulations may be obtained by conventional procedures well known in the pharmaceutical art. For example the tablet may be prepared by blending the components together and compressing the mixture into a tablet.

## Claims

1. A quinazoline derivative of the Formula I: 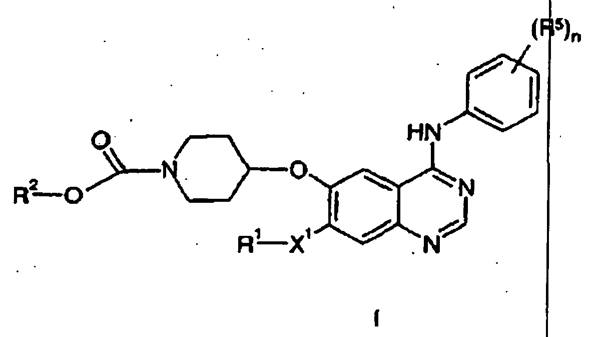 wherein n is 0, 1, 2 or 3,
each **R⁵** is independently selected from halogeno, cyano, nitro, hydroxy, amino, carboxy, sulfamoyl, trifluoromethyl, (1-6C)alkyl, (2-8C)alkenyl, (2-8C)alkynyl, (1-6C)alkoxy, (2-6C)alkenyloxy, (2-6c)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylsulfamoyl, and N,N-di-[(1-6C)alkyl]sulfamoyl, C(O)NR⁶R⁷ where R⁶ and R⁷ are independently selected from hydrogen, optionally substituted (1-6C)alkyl, optionally substituted (3-8C)cycloalkyl or optionally substituted aryl, or R⁶ and R⁷ together with the nitrogen to which they are attached form an optionally substituted heterocyclic ring which may contain additional heteroatoms;
**X¹** is a direct bond or O;
**R¹** is selected from hydrogen and (1-6C)alkyl, wherein the (1-6C)alkyl group is optionally substituted by one or more substituents, which may be the same or different, selected from hydroxy and halogeno, and/or a substituent selected from amino, nitro, carboxy, cyano, halogeno, (1-6C)alkoxy, hydroxy(1-6C)alkoxy, (2-8C)alkenyl, (2-8C)alkynyl, (1-6C)alkylthio, (1-6C)alkylsulfinyl, (1-6C)alkylsulfonyl, (1-6C)alkylamino, di-[(1-6C)alkyl]amino, carbamoyl, N-(1-6C)alkylcarbamoyl, N,N di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (1-6C)alkoxycarbonyl, sulfamoyl, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino;
**R²** is (1-6C)alkyl, (2-6C)alkenyl or (2-6C)alkynyl, any of which may be optionally substituted by fluoro, (1-6C)alkoxy, (1-6C)alkylthio, (1-6)alkylsulfinyl, (1-6C)alkylsulfonyl, or a group of sub-formula (i) 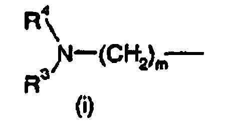
wherein m is 0, 1, 2 or 3;
**R³** and **R⁴** are independently selected from hydrogen or (1-6C)alkyl, or R³ and R⁴ together with the nitrogen atom to which they are attached form a saturated 5 or 6 membered heterocyclic ring which optionally contains additional heteroatoms selected from oxygen, S, SO, SO₂ or NR⁸ where R⁸ is hydrogen, (1-6C)alkyl, (2-6C)alkenyl, (2-6C)alkynyl, (1-6C)alkylsulfonyl or (1-6C)alkylcarbonyl;
provided that the quinazoline derinvative is not:
4-[(3-chloro-4-fluorophenyl)amino]-6-[1-(tert-butyloxycarbonyl)-piperidin-4-yl-oxy]-7-methoxy-quinazoline;
4-[(3-chloro-4-fluorophenyl)amino]-6-[1-(isopropyloxycarbonyl)-piperidin-4-yl-oxy]-7-methoxy-quinazoline;
4-[(3-ethynyl-phenyl)amino]-6-[1-(tert-butyloxycarbonyl)-piperidin-4-yl-oxy]-7-methoxyquinazoline; or
6-{[(1-tert-butoxycarbonyl)piperidin-4-yl]oxy}-4-(3-chloro-2-fluoroanilino)-7-methoxyquinazoline;
or a pharmaceutically acceptable salt thereof.

2. A quinazoline derivative according to claim 1, wherein R² is (1-6C)alkyl, (2-6C)alkenyl or (2-6C)alkynyl, any of which may be optionally substituted by fluoro, (1-6C)alkoxy, (1-6C)alkylthio, (1-6)alkylsulfinyl (1-6C)alkylsulfonyl, or a group of sub-formula (i) 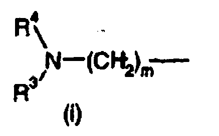 wherein m is 1, 2 or 3; and
R³ and R⁴ are independently selected from hydrogen or (1-6C)alkyl.
or R³ and R⁴ together with the nitrogen atom to which they are attached form a saturated 5 or 6 membered heterocyclic ring which optionally contains additional heteroatoms selected from oxygen, S, SO, SO₂ or NR⁸ where R⁸ is hydrogen, (1-6C)alkyl, (2-6C)alkenyl, (2-6C)alkynyl, (1-6C)alkylsulfonyl or (1-6C)alkylcarbonyl.

3. A quinazoline derivative according to claim 1 or claim 2, wherein n is 1, 2 or 3.

4. A quinazoline derivative according to claim 3, wherein n is 2 or 3.

5. A quinazoline derivative according to claim 4, wherein n is 2.

6. A quinazoline derivative according to claim 4, wherein n is 3.

7. A quinazoline derivative according to any one of the preceding claims, wherein each group R⁵ is a halogeno group.

8. A quinazoline derivative according to any one of the preceding claims, wherein each group R⁵ is selected from chloro and fluoro.

9. A quinazoline derivative according to any one of the preceding claims, which includes a group R⁵ positioned at an ortho- (2-) position on the benzene ring to which it is attached.

10. A quinazoline derivative according to claim 9, wherein the group R⁵ positioned at the ortho- (2-) position is fluoro.

11. A quinazoline derivative according to any one of the preceding claims wherein in the Formula I, the group of sub-formula (ii): 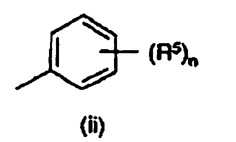 is a group of sub-formula (iii): 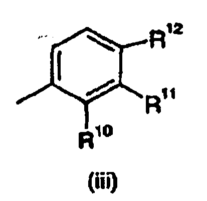 where (a) one of R¹⁰ or R¹² is hydrogen and the other is halogeno, and R¹¹ is halogeno, or (b) R¹⁰ is halogeno, R¹¹ is halogeno and R¹² is selected from hydrogen or halogeno, or (c) R¹⁰ is fluoro, R¹¹ is chloro, and R¹² is selected hydrogen or fluoro.

12. A quinazoline derivative according to claim 11, wherein one of R¹⁰ or R¹² is hydrogen and the other is fluoro, and R¹¹ is chloro.

13. A quinazoline derivative according to claim 11, wherein R¹⁰ is fluoro, R¹¹ is chloro, and R¹² is hydrogen.

14. A quinazoline derivative according to claim 11, wherein R¹⁰ is fluoro, R¹¹ is chloro, and R¹² is fluoro.

15. A quinazoline derivative according to any one of the preceding claims, wherein X¹ is oxygen.

16. A quinazoline derivative according to any one of the preceding claims, wherein R¹ is selected from hydrogen, (1-6C)alkyl and (1-6C)alkoxy(1-6C)alkyl, wherein any (1-6C)alkyl group in R¹ optionally bears one or more hydroxy or halogeno substituents.

17. A quinazoline derivative according to claim 16, wherein R¹ is selected from (1-6C)alkyl, which optionally bears one or more hydroxy or halogeno substituents.

18. A quinazoline derivative according to any one of the preceding claims, wherein R¹-X-is selected from hydrogen, methoxy, ethoxy and 2-methoxyethoxy.

19. A quinazoline derivative according to claim 18, wherein R¹-X¹- is methoxy.

20. A quinazoline derivative according to claim 1 of Formula IA: 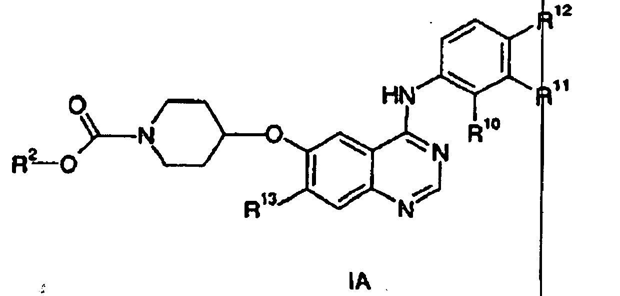 wherein R² is as defined in claim 1, R¹⁰, R¹¹ and R¹² are as defined in any one of claims 11 to 14, and R¹³ is selected from hydrogen, methoxy, ethoxy and 2-methoxyethoxy.

21. A quinazoline derivative according to claim 1 of Formula IB: 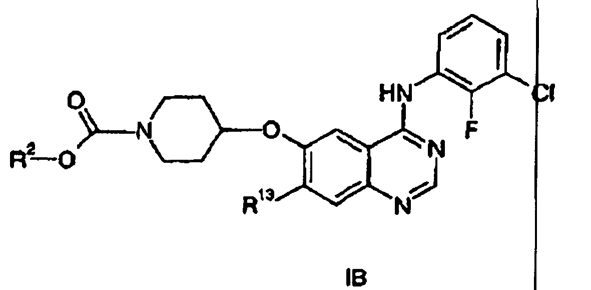 wherein R² is as defined in claim 1 and R¹³ is selected from hydrogen, methoxy, ethoxy and 2-methoxyethoxy

22. A quinazoline derivative according to claim 1 of Formula IC: 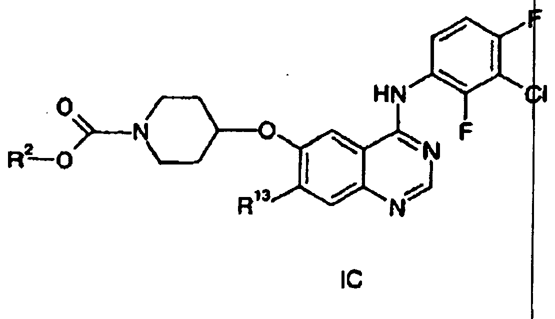 wherein R² is as defined in claim 1 and R¹³ is selected from hydrogen, methoxy, ethoxy and 2-methoxyethoxy

23. A quinazoline derivative according to any one of claims 20 to 22, wherein R¹³ is methoxy.

24. A quinazoline derivative according to any one of the preceding claims, wherein R² is a (1-6C)alkyl group, which is optionally substituted by a fluoro, (1-6C)alkoxy, (1-6C)alkylthio, (1-6)alkylsulfinyl, (1-6C)alkylsulfonyl, or a group of sub-formula (i) as defined in claim 1 or claim 2.

25. A quinazoline derivative according to any one of the preceding claims, wherein R² is a (1-3C)alkyl group, which is optionally substituted by a fluoro, (1-6C)alkoxy, (1-6C)alkylthio, (1-6)alkylsulfinyl, (1-6C)alkylsulfonyl, or a group of sub-formula (i) as defined in claim 1 or claim 2.

26. A quinazoline derivative according to any one of the preceding claims, wherein R² is a (1-6C)alkyl group, which is optionally substituted by a group of sub-formula (i) as defined in claim 1 or claim 2.

27. A quinazoline derivative according to any one of the preceding claims, wherein R² is a (1-3C)alkyl group, which is optionally substituted by a group of sub-formula (i) as defined in claim 1 or claim 2.

28. A quinazoline derivative according to any one of the preceding claims, wherein R² is methyl.

29. A quinazoline derivative according to any one of the preceding claims, wherein R² contains a substituent of sub-formula (i) as defined in claim 1 or claim 2.

30. A quinazoline derivative according to claim 29, wherein m is 1 or 2.

31. A quinazoline derivative according to claim 30, wherein m is 2.

32. A quinazoline derivative according to claim 30, wherein m is 1.

33. A quinazoline derivative according to any one of claims 29 to 32, wherein R³ and R⁴ together with the nitrogen atom to which they are attached form a pyrrolidine ring, a morpholine ring, a piperidine ring, or a piperazine ring which is optionally substituted on the available nitrogen atom by (1-3C) alkyl.

34. A quinazoline derivative according to claim 33, wherein R³ and R⁴ together with the nitrogen atom to which they are attached form a pyrrolidine ring, a morpholine ring, a piperidine ring, or a piperazine ring which is optionally substituted on the available nitrogen atom by methyl.

35. A quinazoline derivative according to claim 33 or claim 34, wherein R³ and R⁴ together with the nitrogen atom to which they are attached form a pyrrolidine ring.

36. A quinazoline derivative according to anyone of claims 29 to 32, wherein R³ and R⁴ are independently selected from (1-3C)alkyl.

37. A quinazoline derivative according to any one of the preceding claims, wherein R² is selected from methyl, 2-(pyrrolidin-1-yl)ethyl, 2-(dimethylamino)ethyl, 2-(diethylamino)ethyl, 2-(piperidinyl)ethyl, 2-(morpholin-4-yl)ethyl and 2-(4-methylpiperazin-1-yl)ethyl.

38. A quinazoline derivative according to claim 37, wherein R² is 2-(pyrrolidin-1-yl) ethyl.

39. A quinazoline derivative according to claim 1, which is selected, from one or more of the following:
4-(3-Chloro-2-fluoroanilino)-7-methoxy-6-{[1-(methoxycarbonyl)piperidin-4-yl]oxy} quinazoline;
4-(3-Chloro-2-fluoroanilino)-7-methoxy-6-{[1-{2-(pyrrolidin-1-yl)ethoxycarbonyl} piperidin-4-yl]oxy}quinazoline;
4-(3-Chloro-2-fluoroanilino)-7-methoxy-6-{[1-2-(N,N-dimethylamino)ethoxycarbonyl) piperidin-4-yl]oxy}quinazoline;
4-(3-Chloro-4-fluoroanilino)-7-methoxy-6-{[1-{2-(pyrrolidin-1-yl)ethoxycarbonyl} piperidin-4-yl]oxy}quinazoline;
4-(3-Chloroanilino)-7-methoxy-6-{[1-{2-(pyrrolidin-1-yl)ethoxycarbonyl}piperidin-4-yl] oxy}quinazoline;
4-(3-Chloro-2,4-difluoroanilino)-7-methoxy-6-{[1-(methoxycarbonyl)piperidin-4-yl] oxy}quinazoline;
4-(3-Chloro-2,4-difluoroanilino)-7-methoxy-6-{[1-{2-(pyrrolidin-1-yl)ethoxycarbonyl} piperidin-4-yl]oxy}quinazoline;
4-(3-Chloro-2,4-difluoroanilino)-7-methoxy-6-{[1-{2-(piperidin-1-yl)ethoxycarbonyl} piperidin-4-yl]oxy}quinazoline;
4-(3-Chloro-2-fluoroanilino)-7-methoxy-6-{[1-{2-(piperidin-1-yl)ethoxycarbonyl] piperidin-4-yl]oxy}quinazoline;
4-(3-Chloro-2-fluoroanilino)-6-{[1-{2-(diethylamino)ethoxycarbonyl}piperidin-4-yl]oxy}-7-methoxyquinazoline;
4-(3-Chloro-2-fluoroanilino)-7-methoxy-6-{[1-{2-(morpholin-4-yl)ethoxycarbonyl} piperidin-4-yl]oxy}quinazoline; and
4-(3-Chloro-2-fluoroanilino)-7-methoxy-6-{[1-{2-(4-methylpiperidin-1-yl) ethoxycarbonyl}piperidin-4-yl]oxy}quinazoline;
or a pharmaceutically acceptable salt thereof.

40. A process for preparing a quinazoline derivative according to any one of the preceding claims, which comprises either
**Process (a)** reacting a compound of the Formula II: 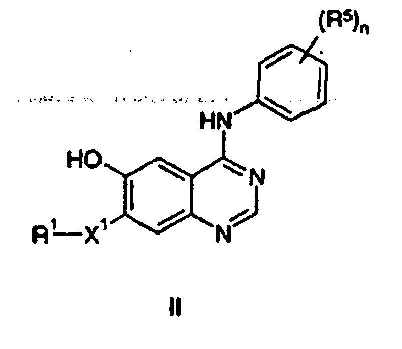 wherein R¹, X¹, R⁵ and n have any of the meanings defined in claim 1 except that any functional group is protected if necessary,
with a compound of the Formula III: 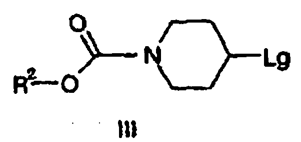 wherein R² have any of the meanings defined hereinbefore except that any functional group is protected if necessary and Lg is a displaceable group,
**Process (b)** modifying a substituent in or introducing a substituent into another quinazoline derivative of Formula I or a pharmaceutically acceptable salt thereof, as hereinbefore defined except that any functional group is protected if necessary,
**Process (c)** reacting a compound of Formula IV: 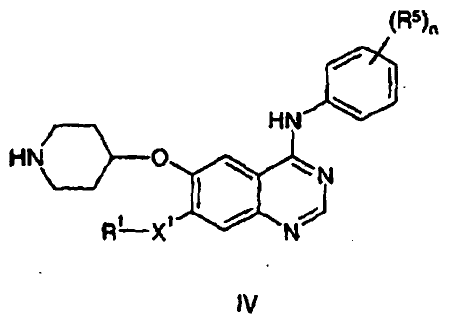 where R¹, X¹, R⁵ and n are as defined in relation to Formula I, with a compound of Formula V. 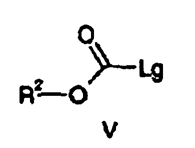 wherein R² is as defined above, and Lg is a displaceable group (for example halogeno such as chloro or bromo);
**Process (d)** removal of a protecting group from a quinazoline derivative of Formula I, or a pharmaceutically acceptable salt thereof.
**Process (e)** reacting a compound of the Formula II as hereinbefore defined with a compound of the Formula III as defined hereinbefore except Lg is OH under Mitsunobu conditions;
**Process (f)** for the preparation of those compounds of the Formula I wherein R¹-X¹ is a hydroxy group by the cleavage of a quinazoline derivative of the Formula I wherein R¹-X¹ is a (1-6C)alkoxy group;
**Process (g)** for the preparation of those compounds of the Formula I wherein X¹ is O, the reaction of a compound of the Formula VI: 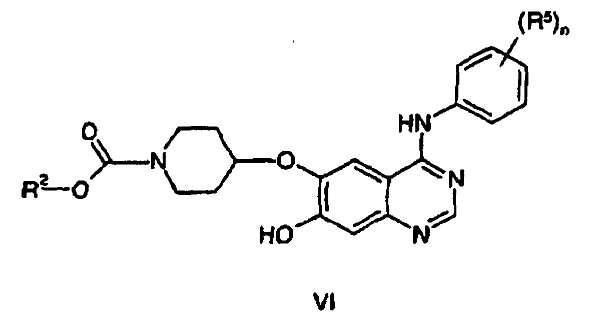 wherein R², R⁵ and n have any of the meanings defined in claim 1 except that any functional group is protected if necessary, with a compound of the formula R¹-Lg, wherein R¹ has any of the meanings defined hereinbefore, except that any functional group is protected if necessary and Lg is a displaceable group;
**Process (h)** for the preparation of those compounds of the Formula I wherein R¹ contains a (1-6C)alkoxy or substituted (1-6C)alkoxy group or a (1-6C)alkylamino or substituted (1-6C)alkylamino group, the alkylation of a quinazoline derivative of the Formula I wherein or R¹ contains a hydroxy group or a primary or secondary amino group as appropriate; **Process (i)** for the preparation of those compounds of the Formula I wherein R¹ is substituted by a group T, wherein T is selected from (1-6C)alkylamino, di-[(1-6C)alkyl]amino, (2-6C)alkanoylamino, (1-6C)alkylthio, (1-6C)alkylsulfinyl and (1-6C)alkylsulfonyl, the reaction of a compound of the Formula VII: 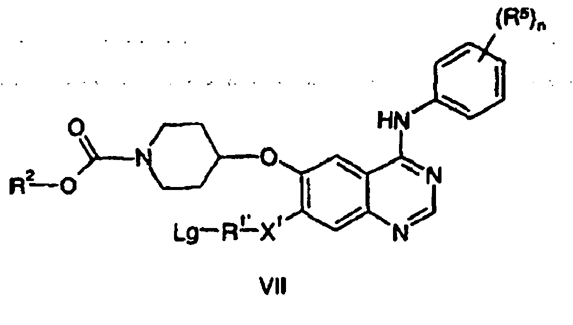 wherein R², R⁵, X¹, n and m have any of the meanings defined in claim 1 except that any functional group is protected if necessary, R^{1'} is a group R¹ as defined herein except that any T groups are replaced with Lg, and Lg is a displaceable group (for example chloro or bromo) with a compound of the formula TH, wherein T is as defmed above except that any functional group is protected if necessary;
**Process (j)** reacting a compound of the Formula VIII: 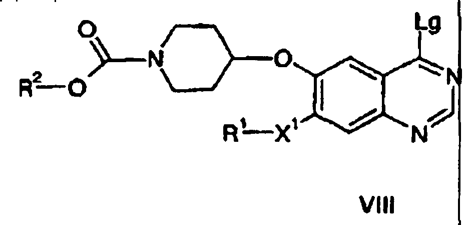 wherein R¹, R², X¹, and m have any of the meanings defined in claim 1 except that any functional group is protected if necessary and Lg is a displaceable group as hereinbefore defined, with an aniline of the Formula IX: 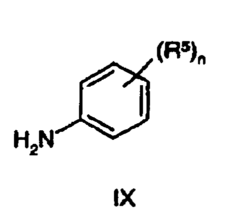 wherein R⁵ and n have any of the meanings defined hereinbefore except that any functional group is protected if necessary; those mentioned above in relation to this process (j); **Process (k)** reacting a compound of Formula X: 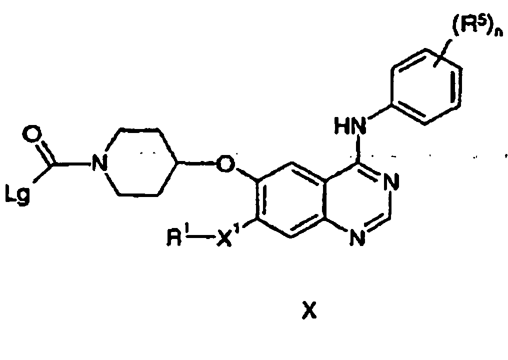 where R⁵, X¹, R¹ and n are as defined in claim 1 except any functional group is protected if necessary, and where Lg is a leaving group, with an alcohol of formula R2-OH, where R² is as defined in claim 1; or
**Process (l)** for compounds where R² includes a group of sub-formula (i), reacting a compound of Formula XI: 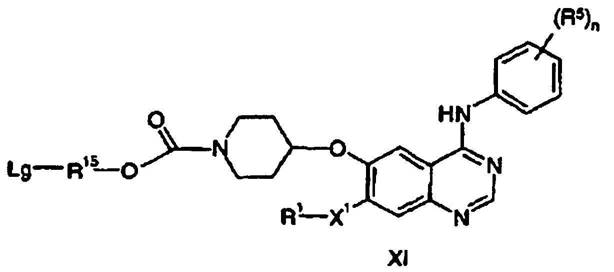 where R¹, X¹, R⁵, and n are as defined hereinbefore, R¹⁵ is a (1-6C)alkylene group, and Lg is a leaving group, with a compound of formula R³R⁴NH where R³ and R⁴ are as defined in relation to sub-formula (i) above;
and whereafter any of said processes, any protecting group that is present is removed.

41. A pharmaceutical composition which comprises a quinazoline derivative of the Formula I, or a pharmaceutically-acceptable salt thereof, as defined in any one of claims 1 to 39 in association with a pharmaceutically-acceptable diluent or carrier.

42. A quinazoline derivative of the Formula I as defined in any one of claims 1 to 39, or a pharmaceutically acceptable salt thereof, for use as a medicament.

43. The use of a quinazoline derivative of the Formula I, or a pharmaceutically-acceptable salt thereof, as defined in any one of claims 1 to 39 in the manufacture of a medicament for use in the production of an anti-proliferative effect in a warm-blooded animal.

44. A compound of the Formula VI, VII, X or XI as defined in claim 40 or a salt thereof.

45. The use of a quinazoline derivative of the Formula I, or a pharmaceutically-acceptable salt thereof, as defined in any one of claims 1 to 39 in the manufacture of a medicament for use in the treatment of a cancer.

46. The use of a quinazoline derivative of the Formula I, or a pharmaceutically-acceptable salt thereof, as defined in any one of claims 1 to 39 in the manufacture of a medicament for use in the treatment of a tumour.

## Patentansprüche

1. Chinazolinderivat der Formel I: 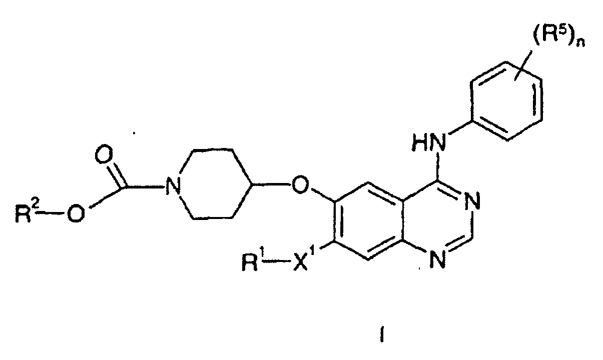 worin **n** für 0, 1, 2 oder 3 steht;
**R⁵** jeweils unabhängig voneinander unter Halogen, Cyano, Nitro, Hydroxy, Amino, Carboxy, Sulfamoyl, Trifluormethyl, C₁₋₆-Alkyl, C₂₋₈-Alkenyl, C₂₋₈-Alkinyl, C₁₋₆-Alkoxy, C₂₋₆-Alkenyloxy, C₂₋₆-Alkinyloxy, C₁₋₆-Alkylthio, C₁₋₆-Alkylsulfinyl, C₁₋₆-Alkylsulfonyl, C₁₋₆-Alkylamino, Di(C₁₋₆-alkyl)amino, C₁₋₆-Alkoxycarbonyl, N-C₁₋₆-Alkylsulfamoyl und N,N-Di(C₁₋₆-alkyl)sulfamoyl, C(O)NR⁶R⁷, worin R⁶ und R⁷ unabhängig voneinander unter Wasserstoff, gegebenenfalls substituiertem C₁₋₆-Alkyl, gegebenenfalls substituiertem C₃₋₈-Cycloalkyl oder gegebenenfalls substituiertem Aryl ausgewählt sind oder R⁶ und R⁷ gemeinsam mit dem Stickstoff, an den sie gebunden sind, einen gegebenenfalls substituierten heterocyclischen Ring bilden, der zusätzliche Heteroatome enthalten kann, ausgewählt ist;
**X¹** für eine direkte Bindung oder O steht;
**R¹** unter Wasserstoff und C₁₋₆-Alkyl ausgewählt ist, wobei die C₁₋₆-Alkylgruppe gegebenenfalls durch einen oder mehrere Substituenten, die gleich oder verschieden sein können und unter Hydroxy und Halogen ausgewählt sind, und/oder einen unter Amino, Nitro, Carboxy, Cyano, Halogen, C₁₋₆-Alkoxy, Hydroxy-C₁₋₆-alkoxy, C₂₋₈-Alkenyl, C₂₋₈-Alkinyl, C₁₋₆-Alkylthio, C₁₋₆-Alkylsulfinyl, C₁₋₆-Alkylsulfonyl, C₁₋₆-Alkylamino, Di(C₁₋₆-alkyl)amino, Carbamoyl, N-C₁₋₆-Alkylcarbamoyl, N,N-Di(C₁₋₆-alkyl)carbamoyl, C₂₋₆-Alkanoyl, C₂₋₆-Alkanoyloxy, C₂₋₆-Alkanoylamino, N-C₁₋₆-Alkyl-C₂₋₆-alkanoylamino, C₁₋₆-Alkoxycarbonyl, Sulfamoyl, N-C₁₋₆-Alkylsulfamoyl, N,N-Di (C₁₋₆-alkyl)sulfamoyl, C₁₋₆-Alkansulfonylamino und N-C₁₋₆-Alkyl-C₁₋₆-alkansulfonylamino ausgewählten Substituenten substituiert ist;
**R²** für C₁₋₆-Alkyl, C₂₋₆-Alkenyl oder C₂₋₆-Alkinyl steht, wobei alle diese Gruppen gegebenenfalls an einem Kohlenstoffatom durch Fluor, C₁₋₆-Alkoxy, C₁₋₆-Alkylthio, C₁₋₆-Alkylsulfinyl, C₁₋₆-Alkylsulfonyl oder eine Gruppe der Unterformel (i) 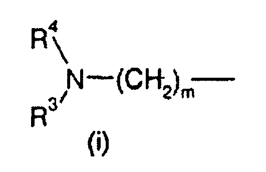
worin m für 0, 1, 2 oder 3 steht;
**R³** und **R⁴** unabhängig voneinander unter Wasserstoff oder C₁₋₆-Alkyl ausgewählt sind,
oder R³ und R⁴ gemeinsam mit dem Stickstoffatom, an das sie gebunden sind, einen gesättigten 5- oder 6-gliedrigen heterocyclischen Ring, der gegebenenfalls zusätzliche unter Sauerstoff, S, SO, SO₂ oder NR⁸, worin R⁸ für Wasserstoff, C₁₋₆-Alkyl, C₂₋₆-Alkenyl, C₂₋₆-Alkinyl, C₁₋₆-Alkylsulfonyl oder C₁₋₆-Alkylcarbonyl steht, ausgewählte Heteroatome enthält, bilden,
substituiert sein können;
mit der Maßgabe, daß es sich bei dem Chinazolinderivat nicht um:
4-[(3-Chlor-4-fluorphenyl)amino]-6-[1-(tert-butyloxycarbonyl)piperidin-4-yloxy]-7-methoxychinazolin;
4-[(3-Chlor-4-fluorphenyl)amino]-6-[1-(isopropyloxycarbonyl)piperidin-4-yloxy]-7-methoxychinazolin;
4-[(3-Ethinylphenyl)amino]-6-[1-(tert-butyloxycarbonyl)piperidin-4-yloxy]-7-methoxychinazolin oder
6-{[(1-tert-Butoxycarbonyl)piperidin-4-yl]oxy}-4-(3-chlor-2-fluoranilino)-7-methoxychinazolin handelt;
oder ein pharmazeutisch annehmbares Salz davon.

2. Chinazolinderivat nach Anspruch 1, worin R² für C₁₋₆-Alkyl, C₂₋₆-Alkenyl oder C₂₋₆-Alkinyl steht, wobei alle diese Gruppen gegebenenfalls durch Fluor, C₁₋₆-Alkoxy, C₁₋₆-Alkylthio, C₁₋₆-Alkylsulfinyl, C₁₋₆-Alkylsulfonyl oder eine Gruppe der Unterformel (i) 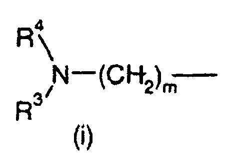 worin m für 1, 2 oder 3 steht und
R³ und R⁴ unabhängig voneinander unter Wasserstoff oder C₁₋₆-Alkyl ausgewählt sind,
oder R³ und R⁴ gemeinsam mit dem Stickstoffatom, an das sie gebunden sind, einen gesättigten 5- oder 6-gliedrigen heterocyclischen Ring, der gegebenenfalls zusätzliche unter Sauerstoff, S, SO, SO₂ oder NR⁸, worin R⁸ für Wasserstoff, C₁₋₆-Alkyl, C₂₋₆-Alkenyl, C₂₋₆-Alkinyl, C₁₋₆-Alkylsulfonyl oder C₁₋₆-Alkylcarbonyl steht, ausgewählte Heteroatome enthält, bilden,
substituiert sein können.

3. Chinazolinderivat nach Anspruch 1 oder 2, worin n für 1, 2 oder 3 steht.

4. Chinazolinderivat nach Anspruch 3, worin n für 2 oder 3 steht.

5. Chinazolinderivat nach Anspruch 4, worin n für 2 steht.

6. Chinazolinderivat nach Anspruch 4, worin n für 3 steht.

7. Chinazolinderivat nach einem der vorhergehenden Ansprüche, worin jede Gruppe R⁵ für eine Halogengruppe steht.

8. Chinazolinderivat nach einem der vorhergehenden Ansprüche, worin jede Gruppe R⁵ unter Chlor und Fluor ausgewählt ist.

9. Chinazolinderivat nach einem der vorhergehenden Ansprüche mit einer Gruppe R⁵ in ortho-Stellung (2-Stellung) an dem Benzolring, an den sie gebunden ist.

10. Chinazolinderivat nach Anspruch 9, worin es sich bei der Gruppe R⁵ in ortho-Stellung (2-Stellung) um Fluor handelt.

11. Chinazolinderivat nach einem der vorhergehenden Ansprüche, worin in Formel I die Gruppe der Unterformel (ii): 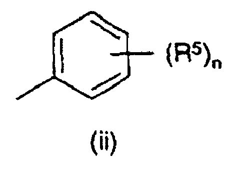 für eine Gruppe der Unterformel (iii): 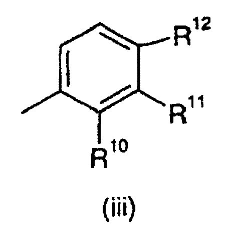 worin (a) eine der Gruppen R¹⁰ oder R¹² für Wasserstoff und die andere für Halogen steht und R¹¹ für Halogen steht oder (b) R¹⁰ für Halogen steht, R¹¹ für Halogen steht und R¹² unter Wasserstoff oder Halogen ausgewählt ist oder (c) R¹⁰ für Fluor steht, R¹¹ für Chlor steht und R¹² unter Wasserstoff oder Fluor ausgewählt ist, steht.

12. Chinazolinderivat nach Anspruch 11, worin eine der Gruppen R¹⁰ oder R¹² für Wasserstoff und die andere für Fluor steht und R¹¹ für Chlor steht.

13. Chinazolinderivat nach Anspruch 11, worin R¹⁰ für Fluor steht, R¹¹ für Chlor steht und R¹² für Wasserstoff steht.

14. Chinazolinderivat nach Anspruch 11, worin R¹⁰ für Fluor steht, R¹¹ für Chlor steht und R¹² für Fluor steht.

15. Chinazolinderivat nach einem der vorhergehenden Ansprüche, worin X¹ für Sauerstoff steht.

16. Chinazolinderivat nach einem der vorhergehenden Ansprüche, worin R¹ unter Wasserstoff, C₁₋₆-Alkyl und C₁₋₆-Alkoxy-C₁₋₆-alkyl ausgewählt ist, wobei jede C₁₋₆-Alkylgruppe in R¹ gegebenenfalls einen oder mehrere Hydroxy- oder Halogensubstituenten trägt.

17. Chinazolinderivat nach Anspruch 16, worin R¹ unter C₁₋₆-Alkyl ausgewählt ist, welches gegebenenfalls einen oder mehrere Hydroxy- oder Halogensubstituenten trägt.

18. Chinazolinderivat nach einem der vorhergehenden Ansprüche, worin R¹-X¹- unter Wasserstoff, Methoxy, Ethoxy und 2-Methoxyethoxy ausgewählt ist.

19. Chinazolinderivat nach Anspruch 18, worin R¹-X¹-für Methoxy steht.

20. Chinazolinderivat nach Anspruch 1 der Formel IA: 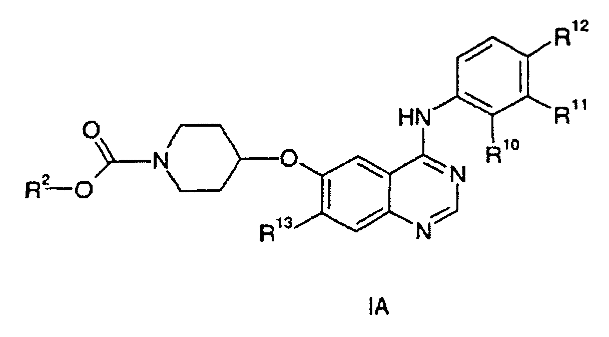 worin R² die in Anspruch 1 angegebene Bedeutung besitzt, R¹⁰, R¹¹ und R¹² die in einem der Ansprüche 11 bis 14 angegebene Bedeutung besitzen und R¹³ unter Wasserstoff, Methoxy, Ethoxy und 2-Methoxyethoxy ausgewählt ist.

21. Chinazolinderivat nach Anspruch 1 der Formel IB: 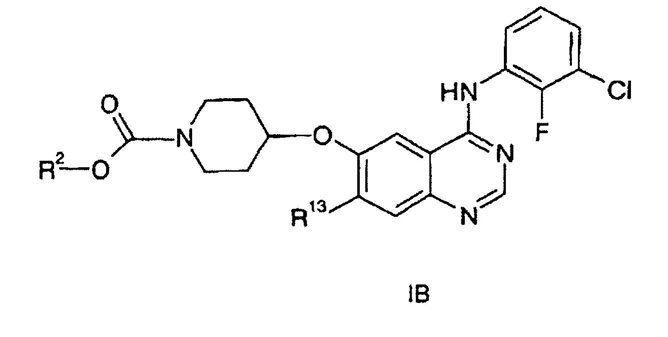 worin R² die in Anspruch 1 angegebene Bedeutung besitzt und R¹³ unter Wasserstoff, Methoxy, Ethoxy und 2-Methoxyethoxy ausgewählt ist.

22. Chinazolinderivat nach Anspruch 1 der Formel IC: 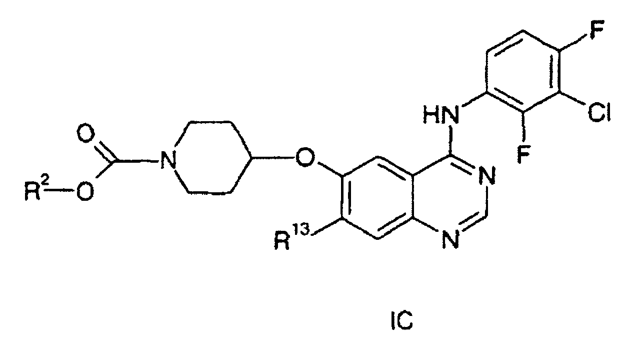 worin R² die in Anspruch 1 angegebene Bedeutung besitzt und R¹³ unter Wasserstoff, Methoxy, Ethoxy und 2-Methoxyethoxy ausgewählt ist.

23. Chinazolinderivat nach einem der Ansprüche 20 bis 22, worin R¹³ für Methoxy steht.

24. Chinazolinderivat nach einem der vorhergehenden Ansprüche, worin R² für eine C₁₋₆-Alkylgruppe steht, die gegebenenfalls durch Fluor, C₁₋₆-Alkoxy, C₁₋₆-Alkylthio, C₁₋₆-Alkylsulfinyl, C₁₋₆-Alkylsulfonyl oder eine Gruppe der Unterformel (i) gemäß Anspruch 1 oder Anspruch 2 substituiert ist.

25. Chinazolinderivat nach einem der vorhergehenden Ansprüche, worin R² für eine C₁₋₃-Alkylgruppe steht, die gegebenenfalls durch Fluor, C₁₋₆-Alkoxy, C₁₋₆-Alkylthio, C₁₋₆-Alkylsulfinyl, C₁₋₆-Alkylsulfonyl oder eine Gruppe der Unterformel (i) gemäß Anspruch 1 oder Anspruch 2 substituiert ist.

26. Chinazolinderivat nach einem der vorhergehenden Ansprüche, worin R² für eine C₁₋₆-Alkylgruppe steht, die gegebenenfalls durch eine Gruppe der Unterformel (i) gemäß Anspruch 1 oder Anspruch 2 substituiert ist.

27. Chinazolinderivat nach einem der vorhergehenden Ansprüche, worin R² für eine C₁₋₃-Alkylgruppe steht, die gegebenenfalls durch eine Gruppe der Unterformel (i) gemäß Anspruch 1 oder Anspruch 2 substituiert ist.

28. Chinazolinderivat nach einem der vorhergehenden Ansprüche, worin R² für Methyl steht.

29. Chinazolinderivat nach einem der vorhergehenden Ansprüche, worin R² einen Substituenten der Unterformel (i) gemäß Anspruch 1 oder Anspruch 2 enthält.

30. Chinazolinderivat nach Anspruch 29, worin m für 1 oder 2 steht.

31. Chinazolinderivat nach Anspruch 30, worin m für 2 steht.

32. Chinazolinderivat nach Anspruch 30, worin m für 1 steht.

33. Chinazolinderivat nach einem der Ansprüche 29 bis 32, worin R³ und R⁴ gemeinsam mit dem Stickstoffatom, an das sie gebunden sind, einen Pyrrolidinring, einen Morpholinring, einen Piperidinring oder einen Piperazinring, der gegebenenfalls an dem verfügbaren Stickstoffatom durch C₁₋₃-Alkyl substituiert ist, bilden.

34. Chinazolinderivat nach Anspruch 33, worin R³ und R⁴ gemeinsam mit dem Stickstoffatom, an das sie gebunden sind, einen Pyrrolidinring, einen Morpholinring, einen Piperidinring oder einen Piperazinring, der gegebenenfalls an dem verfügbaren Stickstoffatom durch Methyl substituiert ist, bilden.

35. Chinazolinderivat nach Anspruch 33 oder 34, worin R³ und R⁴ gemeinsam mit dem Stickstoffatom, an das sie gebunden sind, einen Pyrrolidinring bilden.

36. Chinazolinderivat nach einem der Ansprüche 29 bis 32, worin R³ und R⁴ unabhängig voneinander unter C₁₋₃-Alkyl ausgewählt sind.

37. Chinazolinderivat nach einem der vorhergehenden Ansprüche, worin R² unter Methyl, 2-(Pyrrolidin-1-yl)ethyl, 2-(Dimethylamino)ethyl, 2-(Diethylamino)ethyl, 2-(Piperidinyl)ethyl, (2-Morpholin-4-yl)ethyl und 2-(4-Methylpiperazin-1-yl)ethyl ausgewählt ist.

38. Chinazolinderivat nach Anspruch 37, worin R² für 2-(Pyrrolidin-1-yl)ethyl steht.

39. Chinazolinderivat nach Anspruch 1, ausgewählt aus einer oder mehreren der folgenden Verbindungen:
4-(3-Chlor-2-fluoranilino)-7-methoxy-6-{[1-(methoxycarbonyl)piperidin-4-yl]oxy}chinazolin;
4-(3-Chlor-2-fluoranilino)-7-methoxy-6-{[1-{2-(pyrrolidin-1-yl)ethoxycarbonyl}piperidin-4-yl]-oxy}chinazolin;
4-(3-Chlor-2-fluoranilino)-7-methoxy-6-{[1-2-(N,N-dimethylamino)ethoxycarbonyl)piperidin-4-yl]oxy}-chinazolin;
4-(3-Chlor-4-fluoranilino)-7-methoxy-6-{[1-{2-(pyrrolidin-1-yl)ethoxycarbonyl}piperidin-4-yl]-oxy}chinazolin;
4-(3-Chloranilino)-7-methoxy-6-{[1-{2-(pyrrolidin-1-yl)ethoxycarbonyl}piperidin-4-yl]oxy}chinazolin;
4-(3-Chlor-2,4-difluoranilino)-7-methoxy-6-{[1-(methoxycarbonyl)piperidin-4-yl]oxy}chinazolin;
4-(3-Chlor-2,4-difluoranilino)-7-methoxy-6-{[1-{2-(pyrrolidin-1-yl)ethoxycarbonyl}piperidin-4-yl]-oxy}chinazolin;
4-(3-Chlor-2,4-difluoranilino)-7-methoxy-6-{[1-{2-(piperidin-1-yl)ethoxycarbonyl}piperidin-4-yl]-oxy}chinazolin;
4-(3-Chlor-2-fluoranilino)-7-methoxy-6-{[1-{2-(piperidin-1-yl)ethoxycarbonyl}piperidin-4-yl]-oxy}chinazolin;
4-(3-Chlor-2-fluoranilino)-6-{[1-{2-(diethylamino)ethoxycarbonyl}piperidin-4-yl]oxy}-7-methoxychinazolin;
4-(3-Chlor-2-fluoranilino)-7-methoxy-6-{[1-{2-(morpholin-4-yl)ethoxycarbonyl}piperidin-4-yl]-oxy}chinazolin und
4-(3-Chlor-2-fluoranilino)-7-methoxy-6-{[1-{2-(4-methylpiperidin-1-yl)ethoxycarbonyl}piperidin-4-yl]oxy}chinazolin;
oder ein pharmazeutisch annehmbares Salz davon.

40. Verfahren zur Herstellung eines Chinazolinderivats nach einem der vorhergehenden Ansprüche, bei dem man
**Verfahren (a)** eine Verbindung der Formel II: 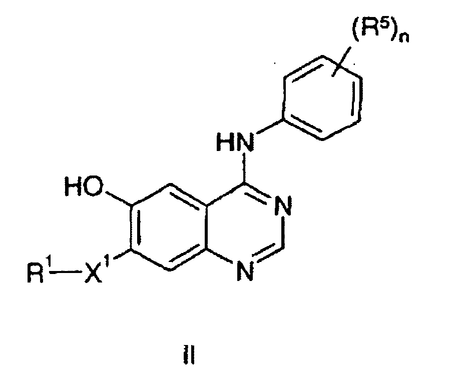 worin R¹, X¹, R⁵ und n eine der in Anspruch 1 angegebenen Bedeutungen besitzen, wobei jedoch jede funktionelle Gruppe gegebenenfalls geschützt ist,
mit einer Verbindung der Formel III: 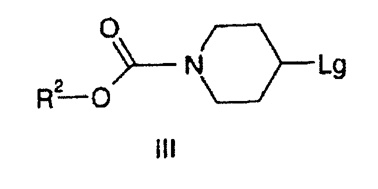 worin R², eine der oben angegebenen Bedeutungen besitzt, wobei jedoch jede funktionelle Gruppe gegebenenfalls geschützt ist, und Lg für eine austauschbare Gruppe steht, umsetzt;
**Verfahren (b)** einen Substituenten in einem anderen Chinazolinderivat der Formel I oder einem pharmazeutisch annehmbaren Salz davon gemäß obiger Definition modifiziert oder einen Substituenten in ein anderes Chinazolinderivat der Formel I oder ein pharmazeutisch annehmbares Salz davon gemäß obiger Definition einführt, wobei jedoch jede funktionelle Gruppe gegebenenfalls geschützt ist;
**Verfahren (c)** eine Verbindung der Formel IV: 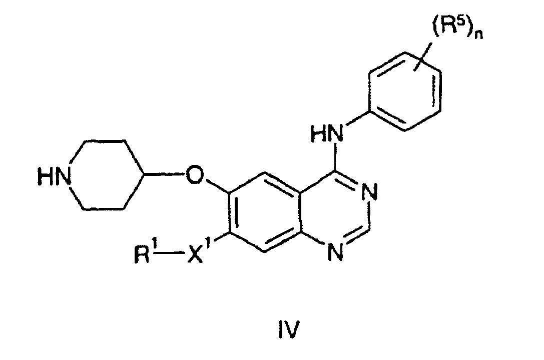 worin R¹, X¹, R⁵ und n die in Verbindung mit Formel I angegebene Bedeutung besitzen, wobei jedoch jede funktionelle Gruppe gegebenenfalls geschützt ist, mit einer Verbindung der Formel V: 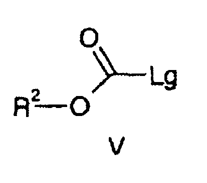 worin R² die oben angegebene Bedeutung besitzt und Lg für eine austauschbare Gruppe (beispielsweise Halogen wie Chlor oder Brom) steht, umsetzt;
**Verfahren (d)** aus einem Chinazolinderivat der Formel I oder einem pharmazeutisch annehmbaren Salz davon eine Schutzgruppe abspaltet;
**Verfahren (e)** eine Verbindung der Formel II gemäß obiger Definition mit einer Verbindung der Formel III gemäß obiger Definition, wobei jedoch Lg für OH steht, unter Mitsunobu-Bedingungen umsetzt;
Verfahren (f) zur Herstellung derjenigen Verbindungen der Formel I, worin R¹-X¹ für eine Hydroxygruppe steht, ein Chinazolinderivat der Formel I, worin R¹-X¹ für eine C₁₋₆-Alkoxygruppe steht, spaltet;
**Verfahren (g)** zur Herstellung derjenigen Verbindungen der Formel I, worin X¹ für O steht, eine Verbindung der Formel VI: 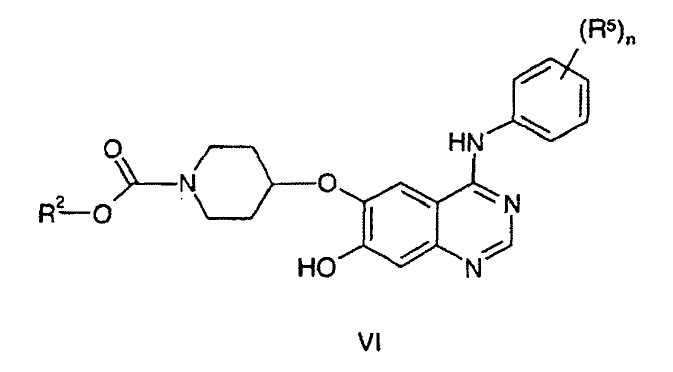 worin R², R⁵ und n eine der in Anspruch 1 angegebenen Bedeutungen besitzen, wobei jedoch jede funktionelle Gruppe gegebenenfalls geschützt ist, mit einer Verbindung der Formel R¹-Lg, worin R¹ eine der oben angegebenen Bedeutungen besitzt, wobei jedoch jede funktionelle Gruppe gegebenenfalls geschützt ist, und Lg für eine austauschbare Gruppe steht, umsetzt;
**Verfahren (h)** zur Herstellung derjenigen Verbindungen der Formel I, worin R¹ eine C₁₋₆-Alkoxygruppe oder substituierte C₁₋₆-Alkoxygruppe oder eine C₁₋₆-Alkylaminogruppe oder substituierte C₁₋₆-Alkylaminogruppe enthält, ein Chinazolinderivat der Formel I, worin R¹ eine Hydroxygruppe bzw. eine primäre oder sekundäre Aminogruppe enthält, alkyliert;
**Verfahren (i)** zur Herstellung derjenigen Verbindungen der Formel I, worin R¹ durch eine Gruppe T substituiert ist, wobei T unter C₁₋₆-Alkylamino, Di(C₁₋₆-alkyl)amino, C₂₋₆-Alkanoylamino, C₁₋₆-Alkylthio, C₁₋₆-Alkylsulfinyl und C₁₋₆-Alkylsulfonyl ausgewählt ist, eine Verbindung der Formel VII: 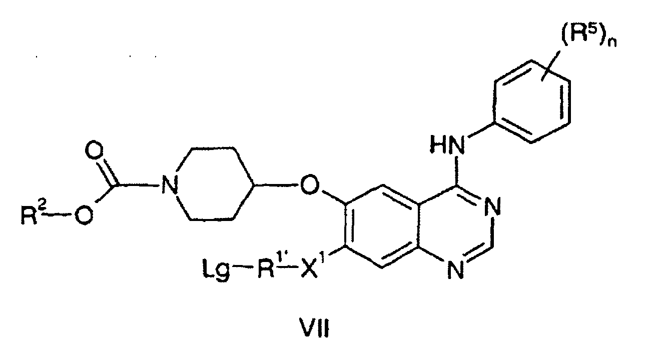 worin R², R⁵, X¹, n und m eine der in Anspruch 1 angegebenen Bedeutungen besitzen, wobei jedoch jede funktionelle Gruppe gegebenenfalls geschützt ist, R^{1'} für eine Gruppe R¹ gemäß der hier angegebenen Definition steht, wobei jedoch alle T-Gruppen durch Lg ersetzt sind, und Lg für eine austauschbare Gruppe (beispielsweise Chlor oder Brom) steht, mit einer Verbindung der Formel TH, worin T die oben angegebene Bedeutung besitzt, wobei jedoch jede funktionelle Gruppe gegebenenfalls geschützt ist, umsetzt;
**Verfahren (j)** eine Verbindung der Formel VIII: 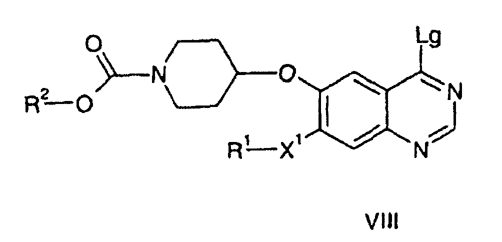 worin R¹, R², X¹ und m eine der in Anspruch 1 angegebenen Bedeutungen besitzen, wobei jedoch jede funktionelle Gruppe gegebenenfalls geschützt ist, und Lg für eine austauschbare Gruppe gemäß obiger Definition steht,
mit einem Anilin der Formel IX: 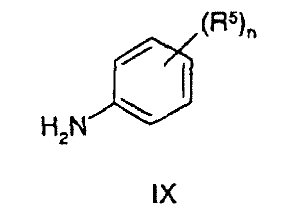 worin R⁵ und n eine der oben angegebenen Bedeutungen besitzen, wobei jedoch jede funktionelle Gruppe gegebenenfalls geschützt ist, umsetzt;
**Verfahren (k)** eine Verbindung der Formel X: 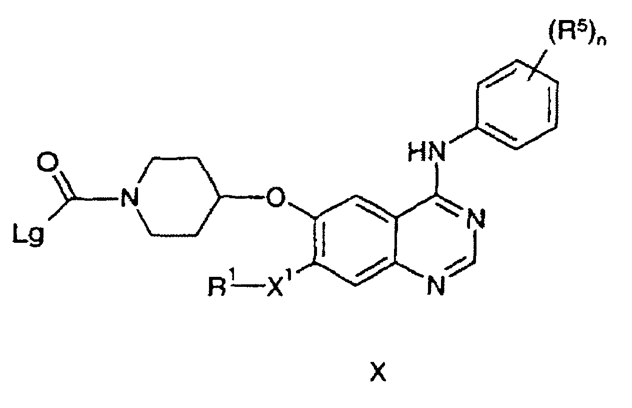 worin R⁵, X¹, R¹ und n die in Anspruch 1 angegebene Bedeutung besitzen, wobei jedoch jede funktionelle Gruppe gegebenenfalls geschützt ist, und Lg für eine Abgangsgruppe steht, mit einem Alkohol der Formel R²-OH, worin R² die in Anspruch 1 angegebene Bedeutung besitzt, umsetzt;
**Verfahren (l)** für Verbindungen, in denen R² eine Gruppe der Unterformel (i) enthält, eine Verbindung der Formel XI: 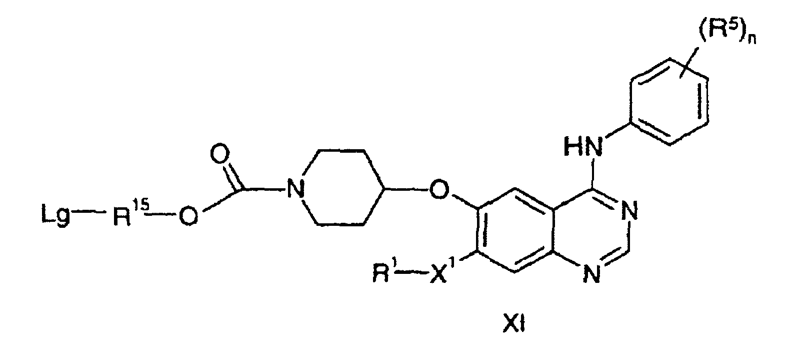 worin R¹, X¹, R⁵ und n die oben angegebene Bedeutung besitzen, R¹⁵ für eine C₁₋₆-Alkylengruppe steht und Lg für eine Abgangsgruppe steht, mit einer Verbindung der Formel R³R⁴NH, worin R³ und R⁴ die in Verbindung mit obiger Unterformel (i) angegebene Bedeutung besitzen, umsetzt;
und nach jedem der Verfahren jede vorhandene Schutzgruppe abspaltet.

41. Pharmazeutische Zusammensetzung, die ein Chinazolinderivat der Formel I oder ein pharmazeutisch annehmbares Salz davon nach einem der Ansprüche 1 bis 39 zusammen mit einem pharmazeutisch annehmbaren Verdünnungsmittel oder Träger enthält.

42. Chinazolinderivat der Formel I nach einem der Ansprüche 1 bis 39 oder ein pharmazeutisch annehmbares Salz davon zur Verwendung als Arzneimittel.

43. Verwendung eines Chinazolinderivats der Formel I oder eines pharmazeutisch annehmbaren Salzes davon nach einem der Ansprüche 1 bis 39 bei der Herstellung eines Arzneimittels zur Verwendung bei der Hervorrufung einer antiproliferativen Wirkung bei einem Warmblüter.

44. Verbindung der Formel VI, VII, X oder XI gemäß Anspruch 40 oder ein Salz davon.

45. Verwendung eines Chinazolinderivats der Formel I oder eines pharmazeutisch annehmbaren Salzes davon nach einem der Ansprüche 1 bis 39 bei der Herstellung eines Arzneimittels zur Verwendung bei der Behandlung von Krebs.

46. Verwendung eines Chinazolinderivats der Formel I oder eines pharmazeutisch annehmbaren Salzes davon nach einem der Ansprüche 1 bis 39 bei der Herstellung eines Arzneimittels zur Verwendung bei der Behandlung eines Tumors.

## Revendications

1. Dérivé de la quinazoline de formule I : 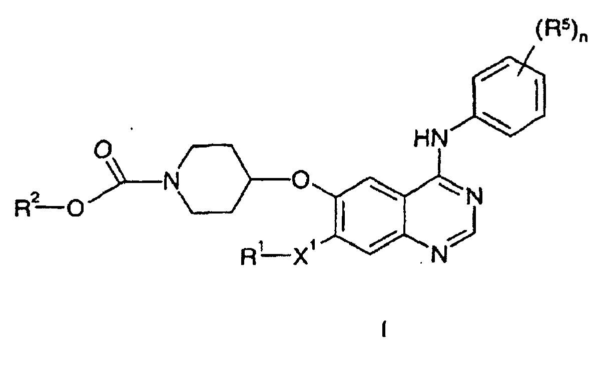 dans laquelle **n** est 0, 1, 2 ou 3,
chaque **R⁵** est choisi indépendamment parmi halogéno, cyano, nitro, hydroxy, amino, carboxy, sulfamoyle, trifluorométhyle, (1-6C)-alkyle, (2-8C)-alcényle, (2-8C)-alkynyle, (1-6C)-alcoxy, (2-6C)-alcényloxy, (2-6C)-alkynyloxy, (1-6C)-alkylthio, (1-6C)-alkylsulfinyle, (1-6C)-alkylsulfonyle, (1-6C)-alkylamino, di-[((1-6C)-alkyl)]amino, (1-6C)-alcoxycarbonyle, N-(1-6C)-alkylsulfamoyle et N,N-di-[((1-6C)-alkyl)]sulfamoyle, C(O)NR⁶R⁷, où R⁶ et R⁷ sont choisis indépendamment parmi hydrogène, ((1-6C))alkyle éventuellement substitué, (3-8C)cycloalkyle éventuellement substitué ou aryle éventuellement substitué, ou R⁶ et R⁷ ensemble avec l'azote auquel ils sont liés forment un cycle hétérocyclique éventuellement substitué qui peut contenir d'autres hétéroatomes ;
**X¹** est une liaison directe ou O ;
**R¹** est choisi parmi hydrogène et ((1-6C))alkyle, où le groupement ((1-6C))alkyle est éventuellement substitué par un ou plusieurs substituants, qui peuvent être identiques ou différents, choisis parmi hydroxy et halogéno, et/ou un substituant choisi parmi amino, nitro, carboxy, cyano, halogéno, ((1-6C))alcoxy, hydroxy((1-6C))alcoxy, ((2-8C))alcényle, ((2-8C))alkynyle, ((1-6C))alkylthio, ((1-6C))alkylsulfinyle, ((1-6C))alkylsulfonyle, ((1-6C))alkylamino, di-[((1-6C))alkyl]amino, carbamoyle, N-((1-6C))alkylcarbamoyle, N,N-di-[((1-6C))alkyl]carbamoyle, ((2-6C))alcanoyle, ((2-6C))alcanoyloxy, ((2-6C))alcanoylamino, N-((1-6C))alkyl-((2-6C))alcanoylamino, ((1-6C))alcoxycarbonyle, sulfamoyle, N-((1-6C))alkylsulfamoyle, N,N-di-[((1-6C))alkyl]sulfamoyle, ((1-6C))alcanesulfonylamino et N-((1-6C))alkyl-((1-6C))alcanesulfonylamino ;
**R²** est ((1-6C))alkyle, ((2-6C))alcényle ou ((2-6C))alkynyle, dont l'un quelconque peut éventuellement être substitué par fluoro, ((1-6C))alcoxy, ((1-6C))alkylthio, ((1-6C))alkylsulfinyle, ((1-6C))alkylsulfonyle, ou un groupement de sous-formule (i) 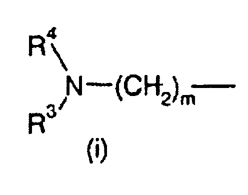
dans laquelle m est 0, 1, 2 ou 3 ;
**R³** et **R⁴** sont choisis indépendamment parmi hydrogène ou ((1-6C))alkyle,
ou R³ et R⁴ ensemble avec l'atome d'azote auquel ils sont liés forment un cycle hétérocyclique saturé à 5 ou 6 chaînons qui contient éventuellement d'autres hétéroatomes choisis parmi oxygène, S, SO, SO₂ ou NR⁸ où R⁸ est hydrogène, ((1-6C))alkyle, ((2-6C))alcényle ((2-6C))alkynyle, ((1-6C))alkylsulfonyle ou ((1-6C))alkylcarbonyle ;
à condition que le dérivé de la quinazoline ne soit pas :
la 4-[(3-chloro-4-fluorophényl)amino]-6-[1-(tert-butyloxycarbonyl)pipéridin-4-yloxy]-7-méthoxyquinazoline ;
la 4-[(3-chloro-4-fluorophényl)amino]-6-[1-(isopropyloxycarbonyl)pipéridin-4-yloxy]-7-méthoxyquinazoline ;
la 4-[(3-éthynylphényl)amino]-6-[1-(tert-butyloxycarbonyl)pipéridin-4-yloxy]-7-méthoxyquinazoline ; ou
la 6-{[(1-tert-butoxycarbonyl)pipéridin-4-yl]oxy}-4-(3-chloro-2-fluoroanilino)-7-méthoxyquinazoline ;
ou un sel pharmaceutiquement acceptable de celui-ci.

2. Dérivé de la quinazoline selon la revendication 1, **caractérisé en ce que** R² est ((1-6C))alkyle, ((2-6C))alcényle ou ((2-6C))alkynyle, dont l'un quelconque peut éventuellement être substitué par fluoro, ((1-6C))alcoxy, ((1-6C))alkylthio, ((1-6C))alkylsulfinyle, ((1-6C))alkylsulfonyle, ou un groupement de sous-formule (i) 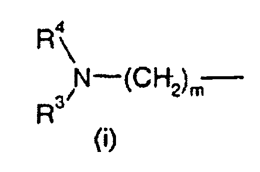 dans laquelle m est 1, 2 ou 3 ; et
R³ et R⁴ sont choisis indépendamment parmi hydrogène ou ((1-6C))alkyle,
ou R³ et R⁴ ensemble avec l'atome d'azote auquel ils sont liés forment un cycle hétérocyclique saturé à 5 ou 6 chaînons qui contient éventuellement d'autres hétéroatomes choisis parmi oxygène, S, SO, SO₂ ou NR⁸ où R⁸ est hydrogène, ((1-6C))alkyle, ((2-6C))alcényle, ((2-6C))alkynyle, ((1-6C))alkylsulfonyle ou ((1-6C))alkylcarbonyle.

3. Dérivé de la quinazoline selon la revendication 1 ou la revendication 2, **caractérisé en ce que** n est 1, 2 ou 3.

4. Dérivé de la quinazoline selon la revendication 3, **caractérisé en ce que** n est 2 ou 3.

5. Dérivé de la quinazoline selon la revendication 4, **caractérisé en ce que** n est 2.

6. Dérivé de la quinazoline selon la revendication 4, **caractérisé en ce que** n est 3.

7. Dérivé de la quinazoline selon l'une quelconque des revendications précédentes, **caractérisé en ce que** chaque groupement R⁵ est un groupement halogéno.

8. Dérivé de la quinazoline selon l'une quelconque des revendications précédentes, **caractérisé en ce que** chaque groupement R⁵ est choisi parmi chloro et fluoro.

9. Dérivé de la quinazoline selon l'une quelconque des revendications précédentes, **caractérisé en ce qu'**il inclut un groupement R⁵ positionné en position ortho- (2-) sur le cycle benzène auquel il est lié.

10. Dérivé de la quinazoline selon la revendication 9, **caractérisé en ce que** le groupement R⁵ positionné en position ortho- (2-) est fluoro.

11. Dérivé de la quinazoline selon l'une quelconque des revendications précédentes, **caractérisé en ce que** dans la formule I, le groupement de sous-formule (ii) : 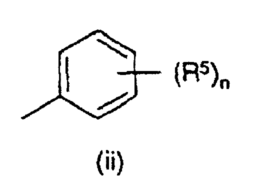 est un groupement de sous-formule (iii) : 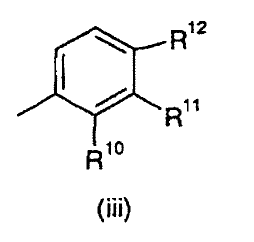 dans laquelle (a) l'un de R¹⁰ ou R¹² est hydrogène et l'autre est halogéno, et R¹¹ est halogéno, ou (b) R¹⁰ est halogéno, R¹¹ est halogéno et R¹² est choisi parmi hydrogène ou halogéno, ou (c) R¹⁰ est fluoro, R¹¹ est chloro, et R¹² est choisi parmi hydrogène ou fluoro.

12. Dérivé de la quinazoline selon la revendication 11, **caractérisé en ce que** l'un de R¹⁰ ou R¹² est hydrogène et l'autre est fluoro, et R¹¹ est chloro.

13. Dérivé de la quinazoline selon la revendication 11, **caractérisé en ce que** R¹⁰ est fluoro, R¹¹ est chloro, et R¹² est hydrogène.

14. Dérivé de la quinazoline selon la revendication 11, dans laquelle R¹⁰ est fluoro, R¹¹ est chloro, et R¹² est fluoro.

15. Dérivé de la quinazoline selon l'une quelconque des revendications précédentes, **caractérisé en ce que** X¹ est oxygène.

16. Dérivé de la quinazoline selon l'une quelconque des revendications précédentes, **caractérisé en ce que** R¹ est choisi parmi hydrogène, ((1-6C))alkyle et ((1-6C))alcoxy((1-6C))alkyle, où tout groupement ((1-6C))alkyle dans R¹ porte éventuellement un ou plusieurs substituants hydroxy ou halogéno.

17. Dérivé de la quinazoline selon la revendication 16, **caractérisé en ce que** R¹ est choisi parmi ((1-6C))alkyle, qui porte éventuellement un ou plusieurs substituants hydroxy ou halogéno.

18. Dérivé de la quinazoline selon l'une quelconque des revendications précédentes, **caractérisé en ce que** R¹-X- est choisi parmi hydrogène, méthoxy, éthoxy et 2-méthoxyéthoxy.

19. Dérivé de quinazoline selon la revendication 18, **caractérisé en ce que** R¹-X¹- est méthoxy.

20. Dérivé de quinazoline selon la revendication 1 de formule IA : 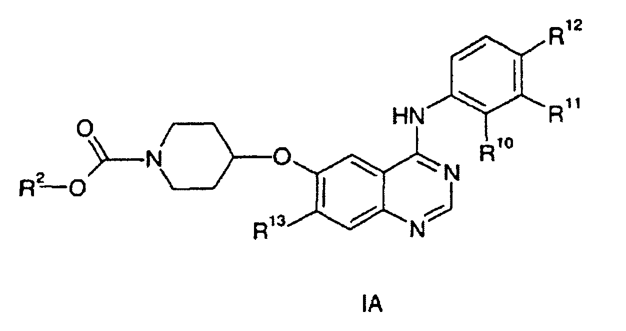 dans laquelle R² est tel que défini dans la revendication 1, R¹⁰, R¹¹ et R¹² sont tels que définis dans l'une quelconque des revendications 11 à 14, et R¹³ est choisi parmi hydrogène, méthoxy, éthoxy et 2-méthoxyéthoxy.

21. Dérivé de la quinazoline selon la revendication 1 de formule IB : 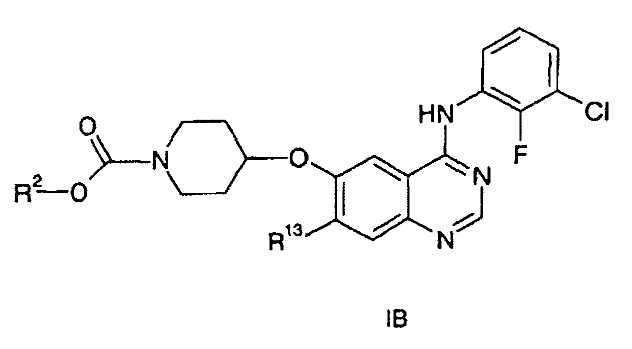 dans laquelle R² est tel que défini dans la revendication 1 et R¹³ est choisi parmi hydrogène, méthoxy, éthoxy et 2-méthoxyéthoxy.

22. Dérivé de la quinazoline selon la revendication 1 de formule IC : 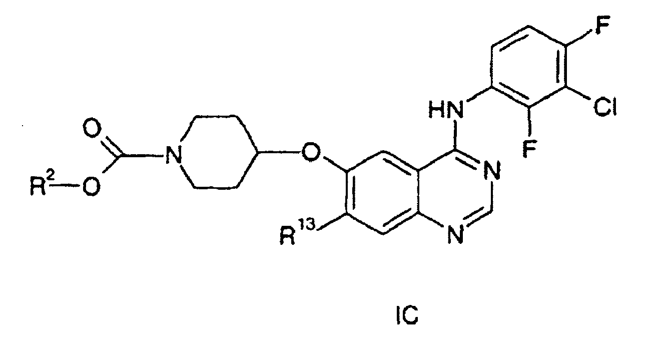 dans laquelle R² est tel que défini dans la revendication 1 et R¹³ est choisi parmi hydrogène, méthoxy, éthoxy et 2-méthoxyéthoxy.

23. Dérivé de la quinazoline selon l'une quelconque des revendications 20 à 22, **caractérisé en ce que** R¹³ est méthoxy.

24. Dérivé de la quinazoline selon l'une quelconque des revendications précédentes, **caractérisé en ce que** R² est un groupement ((1-6C))alkyle, qui est éventuellement substitué par un fluoro, ((1-6C))alcoxy, ((1-6C))alkylthio, ((1-6C))alkylsulfinyle, ((1-6C))alkylsulfonyle, ou un groupement de sous-formule (i) telle que définie dans la revendication 1 ou la revendication 2.

25. Dérivé de la quinazoline selon l'une quelconque des revendications précédentes, **caractérisé en ce que** R² est un groupement ((1-3C))alkyle, qui est éventuellement substitué par un fluoro, ((1-6C))alcoxy, ((1-6C))alkylthio, ((1-6C))alkylsulfinyle, ((1-6C))alkylsulfonyle, ou un groupement de sous-formule (i) telle que définie dans la revendication 1 ou la revendication 2.

26. Dérivé de la quinazoline selon l'une quelconque des revendications précédentes, **caractérisé en ce que** R² est un groupement ((1-6C))alkyle, qui est éventuellement substitué par un groupement de sous-formule (i) telle que définie dans la revendication 1 ou la revendication 2.

27. Dérivé de la quinazoline selon l'une quelconque des revendications précédentes, **caractérisé en ce que** R² est un groupement ((1-3C))alkyle, qui est éventuellement substitué par un groupement de sous-formule (i) telle que définie dans la revendication 1 ou la revendication 2.

28. Dérivé de la quinazoline selon l'une quelconque des revendications précédentes, **caractérisé en ce que** R² est méthyle.

29. Dérivé de la quinazoline selon l'une quelconque des revendications précédentes, **caractérisé en ce que** R² contient un substituant de sous-formule (i) telle que définie dans la revendication 1 ou la revendication 2.

30. Dérivé de la quinazoline selon la revendication 29, **caractérisé en ce que** m est 1 ou 2.

31. Dérivé de la quinazoline selon la revendication 30, **caractérisé en ce que** m est 2.

32. Dérivé de la quinazoline selon la revendication 30, **caractérisé en ce que** m est 1.

33. Dérivé de la quinazoline selon l'une quelconque des revendications 29 à 32, **caractérisé en ce que** R³ et R⁴ ensemble avec l'atome d'azote auquel ils sont liés forment un cycle pyrrolidine, un cycle morpholine, un cycle pipéridine, ou un cycle pipérazine qui est éventuellement substitué sur l'atome d'azote disponible par ((1-3C))alkyle.

34. Dérivé de la quinazoline selon la revendication 33, **caractérisé en ce que** R³ et R⁴ ensemble avec l'atome d'azote auquel ils sont liés forment un cycle pyrrolidine, un cycle morpholine, un cycle pipéridine, ou un cycle pipérazine qui est éventuellement substitué sur l'atome d'azote disponible par méthyle.

35. Dérivé de la quinazoline selon la revendication 33 ou la revendication 34, **caractérisé en ce que** R³ et R⁴ ensemble avec l'atome d'azote auquel ils sont liés forment un cycle pyrrolidine.

36. Dérivé de la quinazoline selon l'une quelconque des revendications 29 à 32, **caractérisé en ce que** R³ et R⁴ sont choisis indépendamment parmi ((1-3C))alkyle.

37. Dérivé de la quinazoline selon l'une quelconque des revendications précédentes, **caractérisé en ce que** R² est choisi parmi méthyle, 2-(pyrrolidin-1-yl)éthyle, 2-(diméthylamino)éthyle, 2-(diéthylamino)éthyle, 2-(pipéridinyl)éthyle, (2-morpholin-4-yl)éthyle et 2-(4-méthylpipérazin-1-yl)éthyle.

38. Dérivé de la quinazoline selon la revendication 37, **caractérisé en ce que** R² est 2-(pyrrolidin-1-yl)éthyle.

39. Dérivé de la quinazoline selon la revendication 1, **caractérisé en ce qu'**il est choisi parmi un ou plusieurs de ce qui suit :
la 4-(3-chloro-2-fluoroanilino)-7-méthoxy-6-{[1-(méthoxycarbonyl)pipéridin-4-yl]oxy}quinazoline ;
la 4-(3-chloro-2-fluoroanilino)-7-méthoxy-6-{[1-{2-(pyrrolidin-1-yl)éthoxycarbonyl}pipéridin-4-yl]oxy}quinazoline ;
la 4-(3-chloro-2-fluoroanilino)-7-méthoxy-6-{[1-2-(N,N-diméthylamino)éthoxycarbonyl)pipéridin-4-yl]oxy}quinazoline ;
la 4-(3-chloro-4-fluoroanilino)-7-méthoxy-6-{[1-{2-(pyrrolidin-1-yl)éthoxycarbonyl}pipéridin-4-yl]oxy}quinazoline ;
la 4-(3-chloroanilino)-7-méthoxy-6-{[1-{2-(pyrrolidin-1-yl)éthoxycarbonyl}pipéridin-4-yl]oxy}quinazoline ;
la 4-(3-chloro-2,4-difluoroanilino)-7-méthoxy-6-{[1-(méthoxycarbonyl)pipéridin-4-yl]oxy}quinazoline ;
la 4-(3-chloro-2,4-difluoroanilino)-7-méthoxy-6-{[1-{2-(pyrrolidin-1-yl)éthoxycarbonyl}pipéridin-4-yl]oxy}quinazoline ;
la 4-(3-chloro-2,4-difluoroanilino)-7-méthoxy-6-{[1-{2-(pipéridin-1-yl)éthoxycarbonyl}pipéridin-4-yl]oxy}quinazoline ;
la 4-(3-chloro-2-fluoroanilino)-7-méthoxy-6-{[1-{2-(pipéridin-1-yl)éthoxycarbonyl}pipéridin-4-yl]oxy}quinazoline ;
la 4-(3-chloro-2-fluoroanilino)-6-{[1-{2-(diéthylamino)éthoxycarbonyl}pipéridin-4-yl]oxy}-7-méthoxyquinazoline ;
la 4-(3-chloro-2-fluoroanilino)-7-méthoxy-6-{[1-{2-(morpholin-4-yl)éthoxycarbonyl}pipéridin-4-yl]oxy}quinazoline ; et
la 4-(3-chloro-2-fluoroanilino)-7-méthoxy-6-{[1-{2-(4-méthylpipéridin-1-yl)éthoxycarbonyl}pipéridin-4-yl]oxy}quinazoline ;
ou un sel pharmaceutiquement acceptable de celui-ci.

40. Procédé de préparation d'un dérivé de la quinazoline selon l'une quelconque des revendications précédentes, **caractérisé en ce qu'**il comprend soit
**Procédé (a)** la réaction d'un composé de formule II : 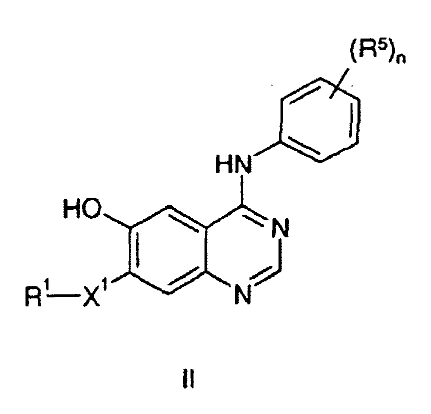 dans laquelle R¹, X¹, R⁵ et n ont l'une quelconque des significations définies dans la revendication 1 sauf qu'une fonction quelconque est protégée si nécessaire,
avec un composé de formule III : 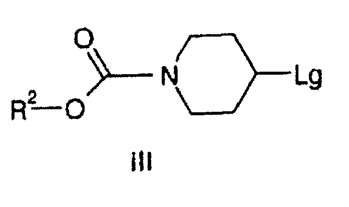 dans laquelle R² a l'une quelconque des significations définies précédemment sauf qu'une fonction quelconque est protégée si nécessaire, et Lg est un groupement déplaçable,
**Procédé (b)** la modification d'un substituant dans ou l'introduction d'un substituant dans un autre dérivé de la quinazoline de formule I ou un sel pharmaceutiquement acceptable de celui-ci, comme défini précédemment sauf qu'une fonction quelconque est protégée si nécessaire,
**Procédé (c)** la réaction d'un composé de formule IV : 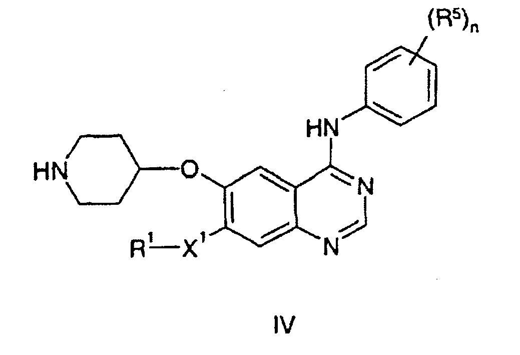 dans laquelle R¹, X¹, R⁵ et n sont tels que définis en relation avec la formule I, avec un composé de formule V : 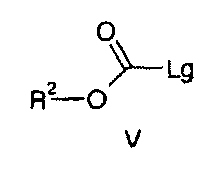 dans laquelle R² est tel que défini ci-dessus, et Lg est un groupement déplaçable (par exemple halogéno tel que chloro ou bromo) ;
**Procédé (d)** l'élimination d'un groupement protecteur d'un dérivé de la quinazoline de formule I, ou d'un sel pharmaceutiquement acceptable de celui-ci ;
**Procédé (e)** la réaction d'un composé de formule II telle que définie précédemment avec un composé de formule III telle que définie précédemment sauf que Lg est OH dans des conditions de Mitsunobu ;
**Procédé (f)** pour la préparation des composés de formule I dans laquelle R¹-X¹ est un groupement hydroxy par le clivage d'un dérivé de la quinazoline de formule I dans laquelle R¹-X¹ est un groupement ((1-6C))alcoxy ;
**Procédé (g)** pour la préparation des composés de formule I dans laquelle X¹ est O, la réaction d'un composé de formule VI : 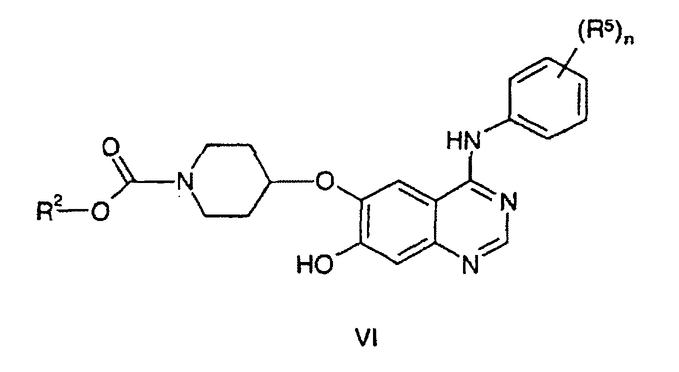 dans laquelle R², R⁵ et n ont l'une quelconque des significations définies dans la revendication 1 sauf qu'une fonction quelconque est protégée si nécessaire, avec un composé de formule R¹-Lg, dans laquelle R¹ a l'une quelconque des significations définies précédemment, sauf qu'une fonction quelconque est protégée si nécessaire et Lg est un groupement déplaçable ;
**Procédé (h)** pour la préparation des composés de formule I dans laquelle R¹ contient un groupement ((1-6C))alcoxy ou ((1-6C))alcoxy substitué ou un groupement ((1-6C))alkylamino ou ((1-6C))alkylamino substitué, l'alkylation d'un dérivé de la quinazoline de formule I dans laquelle R¹ contient un groupement hydroxy ou un groupement amino primaire ou secondaire comme approprié ;
**Procédé (i)** pour la préparation des composés de formule I dans laquelle R¹ est substitué par un groupement T, où T est choisi parmi ((1-6C))alkylamino, di-[((1-6C))alkyl]amino, ((2-6C))alcanoylamino, ((1-6C))alkylthio, ((1-6C))alkylsulfinyle et ((1-6C))alkylsulfonyle, la réaction d'un composé de formule VII : 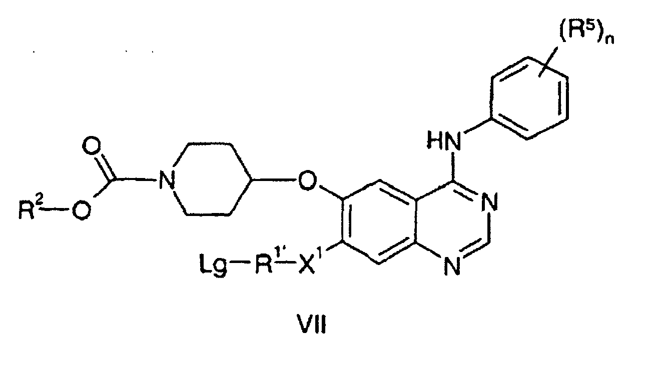 dans laquelle R², R⁵, X¹, n et m ont l'une quelconque des significations définies dans la revendication 1 sauf qu'une fonction quelconque est protégée si nécessaire, R^{1'} est un groupement R¹ tel que défini dans la présente sauf que tous les groupements T sont remplacés par Lg, et Lg est un groupement déplaçable (par exemple chloro ou bromo) avec un composé de formule TH, dans laquelle T est tel que défini ci-dessus sauf qu'une fonction quelconque est protégée si nécessaire ;
**Procédé (j)** la réaction d'un composé de formule VIII : 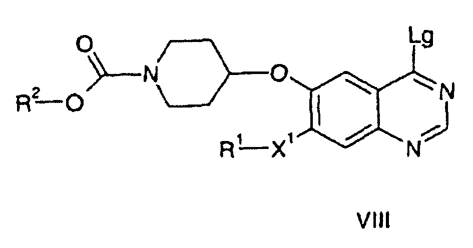 dans laquelle R¹, R², X¹ et m ont l'une quelconque des significations définies dans la revendication 1 sauf qu'une fonction quelconque est protégée si nécessaire et Lg est un groupement déplaçable tel que défini précédemment, avec une aniline de formule IX : 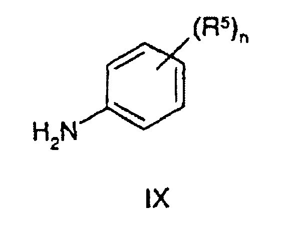 dans laquelle R⁵ et n ont l'une quelconque des significations définies précédemment sauf qu'une fonction quelconque est protégée si nécessaire ;
**Procédé (k)** la réaction d'un composé de formule X : 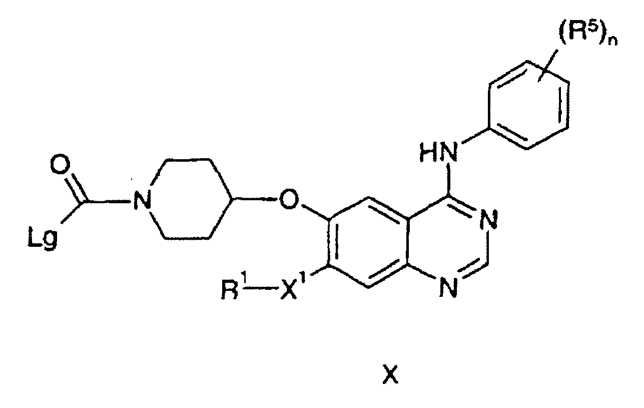 dans laquelle R⁵, X¹, R¹ et n sont tels que définis dans la revendication 1 sauf qu'une fonction quelconque est protégée si nécessaire, et où Lg est un groupement partant, avec un alcool de formule R²-OH, dans laquelle R² est tel que défini dans la revendication 1 ; ou
**Procédé (l)** pour les composés où R² inclut un groupement de sous-formule (i), la réaction d'un composé de formule XI : 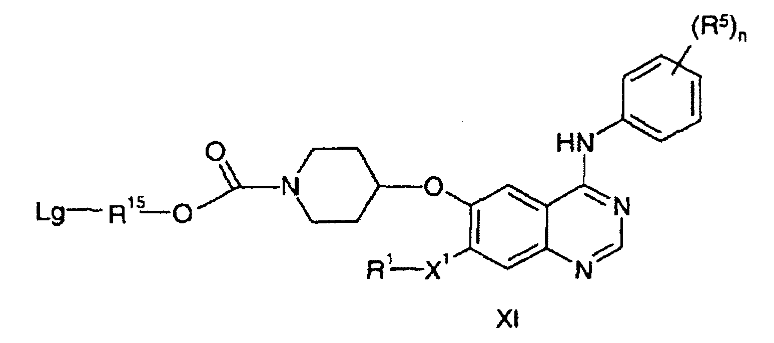 dans laquelle R¹, X¹, R⁵, et n sont tels que définis précédemment, R¹⁵ est un groupement ((1-6C))alkylène, et Lg est un groupement partant, avec un composé de formule R³R⁴NH dans laquelle R³ et R⁴ sont tels que définis en relation avec la sous-formule (i) ci-dessus ;
et à la suite de l'un quelconque desdits procédés, un groupement protecteur quelconque qui est présent est éliminé.

41. Composition pharmaceutique, **caractérisée en ce qu'**elle comprend un dérivé de la quinazoline de formule I, ou un sel pharmaceutiquement acceptable de celui-ci, tel que défini dans l'une quelconque des revendications 1 à 39 en association avec un diluant ou un support pharmaceutiquement acceptable.

42. Dérivé de la quinazoline de formule I tel que défini dans l'une quelconque des revendications 1 à 39, ou un sel pharmaceutiquement acceptable de celui-ci, destiné à être utilisé comme médicament.

43. Utilisation d'un dérivé de la quinazoline de formule I, ou d'un sel pharmaceutiquement acceptable de celui-ci, tel que défini dans l'une quelconque des revendications 1 à 39 dans la fabrication d'un médicament destiné à être utilisé dans la production d'un effet anti-prolifératif chez un animal à sang chaud.

44. Composé de formule VI, VII, X ou XI tel que défini dans la revendication 40 ou un sel de celui-ci.

45. Utilisation d'un dérivé de la quinazoline de formule I, ou d'un sel pharmaceutiquement acceptable de celui-ci, tel que défini dans l'une quelconque des revendications 1 à 39 dans la fabrication d'un médicament destiné à être utilisé dans le traitement d'un cancer.

46. Utilisation d'un dérivé de la quinazoline de formule I, ou d'un sel pharmaceutiquement acceptable de celui-ci, tel que défini dans l'une quelconque des revendications 1 à 39 dans la fabrication d'un médicament destiné à être utilisé dans le traitement d'une tumeur.
